(19) Europäisches Patentamt
European Patent Office
Office européen des brevets

(11) EP 4 190 359 A1

(12) # EUROPEAN PATENT APPLICATION

(43) Date of publication:
**07.06.2023 Bulletin 2023/23**

(21) Application number: **21383098.7**

(22) Date of filing: **03.12.2021**

(51) International Patent Classification (IPC):
***A61K 47/55*** (2017.01) ***A61K 47/64*** (2017.01)
***A61K 39/39*** (2006.01) ***A61K 39/385*** (2006.01)
***A61K 39/00*** (2006.01) ***C07H 15/256*** (2006.01)
***C07J 51/00*** (2006.01) ***A61P 25/28*** (2006.01)
***A61P 31/00*** (2006.01) ***A61P 35/00*** (2006.01)

(52) Cooperative Patent Classification (CPC):
**A61K 47/55; A61K 39/00117; A61K 39/385; A61K 39/39; A61K 47/6425; A61K 47/646; A61P 25/28; A61P 31/00; A61P 35/00; C07H 15/256; C07J 63/008; C07K 7/08; C07K 14/005; C07K 14/22; C07K 14/33;** (Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Asociación Centro de Investigación Cooperativa en Biociencias - CIC bioGUNE**
**48160 Derio (ES)**

(72) Inventors:
- **FERNÁNDEZ-TEJADA, Alberto**
  **48160 Derio, Vizcaya (ES)**
- **PIFFERI, Carlo**
  **48160 Derio, Vizcaya (ES)**
- **FUENTES GARCÍA, Roberto**
  **48160 Derio, Vizcaya (ES)**
- **ANGUITA CASTILLO, Juan**
  **48160 Derio, Vizcaya (ES)**

(74) Representative: **ABG Intellectual Property Law, S.L.**
**Avenida de Burgos, 16D**
**Edificio Euromor**
**28036 Madrid (ES)**

(54) **SAPONIN-BASED ADJUVANTS AND VACCINES**

(57) The present application relates to adjuvants and adjuvant-antigen conjugate vaccines based on a triterpene glycoside saponin scaffold incorporating an oxime group at position C4 of the triterpene domain and pharmaceutical compositions thereof as well as the use of said compounds and compositions in the treatment of and immunization for diseases such as neurodegenerative and infectious diseases and cancers.

EP 4 190 359 A1

(52) Cooperative Patent Classification (CPC): (Cont.)
**C07K 14/445; C07K 14/4748; C07K 14/705;**
A61K 2039/55577; A61K 2039/6075

## Description

### TECHNICAL FIELD OF THE INVENTION

The present application is encompassed within the field of chemical immunology. More specifically, it relates to synthetic adjuvants and vaccines based on a triterpene glycoside saponin scaffold and pharmaceutical compositions thereof as well as the use of said compounds and compositions in the treatment of and immunization for diseases such as neurodegenerative and infectious diseases and cancers.

### BACKGROUND OF THE INVENTION

Vaccines have been approved and have improved health care over the last several decades. Attenuated or inactivated pathogens and their toxins have been historically used as vaccines. Modern subunit vaccines based on homogeneous antigens offer more precise targeting and improved safety compared with traditional whole-pathogen vaccines. However, they are also less immunogenic and require an adjuvant to increase the immunogenicity of the antigen and potentiate the immune response (Pifferi, C. et al. Nat. Rev. Chem. 2021, 25 (4), 3-7). Adjuvants enhance antigen-specific immune responses by modulating and enhancing the innate and adaptive (acquired) immunity when delivered together with an antigen (Bergmann-Leitner, E. et al. Vaccines 2014, 2 (2), 252-296). Additionally, they allow the dose of expensive antigens to be decreased, reduce booster immunizations, generate more rapid and durable immune responses, and increase the effectiveness of vaccines in poor responders (Reed, S. G. et al. Nat. Med. 2013, 19 (12), 1597-1608).

However, few adjuvants are of sufficient potency and acceptable toxicity for clinical use. Aluminum-containing adjuvants were the first human vaccine adjuvants approved in clinical use. Aluminum salts, either alone (alum) or in proprietary mixtures (AS04), and oil-in-water emulsions containing squalene (MF59, AS03) have been used as adjuvants in a number of vaccines, but have relatively low potency and significant side effects, respectively. These limitations highlight the urgent need for new, potent and safe adjuvants.

QS-21 is one of the most promising adjuvants currently under investigation. Isolated from *Quillaja saponaria* tree bark, it is composed of four structural domains: a branched trisaccharide, a quillaic acid triterpene, a bridging linear tetrasaccharide, and a pseudodimeric acyl chain. QS-21 is not a single molecule but a ≈ 2:1 mixture of two isomers that differ at the terminal sugar of the linear tetrasaccharide domain, the first isomer having a terminal apiose (QS-21-Api) and the second one having a xylose (QS-21-Xyl).

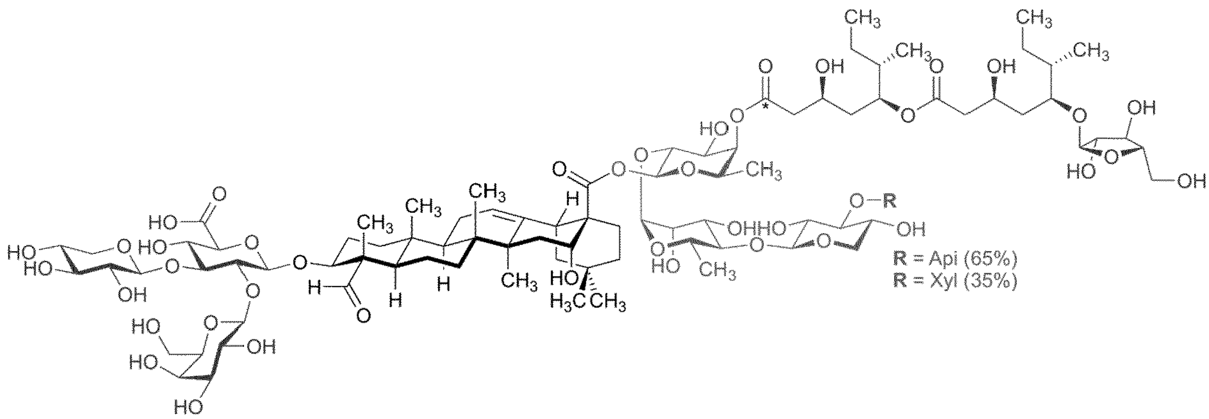

*QS-21*

Vaccines that contain QS-21 have been investigated or are under development for several cancers, and for infectious and neurodegenerative diseases (malaria, acquired immunodeficiency syndrome, hepatitis, tuberculosis and Alzheimer's disease). Despite its promise, QS-21 suffers from several liabilities, including limited access from its natural source, toxic side effects and chemical instability through spontaneous hydrolysis of the acyl chain. With the exception of its recent approval as part of the malaria and shingles vaccines, the scarcity, heterogeneity and dose-limiting toxicity of QS-21 have hampered its further use in human vaccines.

Triterpene glycoside saponin-derived adjuvants are disclosed in WO2009/126737, WO2015/184451, WO2017/079582, WO2017/106836, WO2018/191598, WO2018/200645 WO2018/200656 and WO2019/079160.

However, the difficulties in obtaining pure species from Quillaja saponin extract, the difficulties in correctly

identifying their structures as well as the poor understanding of the molecular mechanism of action of these saponin-based adjuvants impedes the rational development of analogues with improved efficacy and decreased toxicity. Thus, there is still a need for new derivatives that present potent adjuvant activity and/or low toxicity.

Moreover, it would be also highly desirable that the new adjuvants could serve as self-adjuvanting built-in moieties in the development of novel unimolecular vaccine constructs. Compared to traditional approaches involving co-formulation of mixtures of adjuvants and antigens, a self-adjuvanting vaccine strategy consists in the conjugation of the antigen to a well-defined adjuvant. In this way, immune cells can simultaneously up-take both covalently linked components, resulting in enhanced immune responses against the conjugated antigen. Therefore, self-adjuvanting vaccines do not require co-administration of additional adjuvants or conjugation to carrier proteins, avoiding the use of toxic adjuvants and the induction of undesired responses.

## BRIEF DESCRIPTION OF THE INVENTION

The present invention solves one or more of the aforementioned needs by the provision of new triterpene glycoside saponin adjuvants and conjugates thereof. The active compounds of the present invention, which are characterized *inter alia* by bearing an oxime group (-C=NO-) at position C4 of the triterpene domain, have been found to enhance the antibody response against antigens much more than analogous saponins of the state of the art with a C4-aldehyde. Further, some of the saponins of the present invention are conjugated or functionalized with substances capable of inducing an immune response (i.e. having at least one immunogenic region) both through the chain of the carbohydrate domain and via an oxime linkage at the C4-position of the triterpene domain, being thus useful as efficient self-adjuvanting vaccines.

One aspect of the present invention relates to a compound selected from:

- a compound of general formula (I) or a pharmaceutically acceptable salt thereof

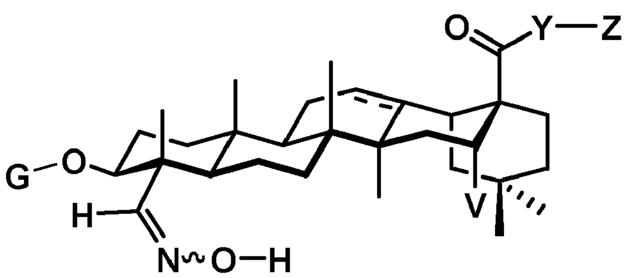

(I)

wherein G is hydrogen, a branched trisacchride of formula (VI) or a stereoisomer of formula (VI)

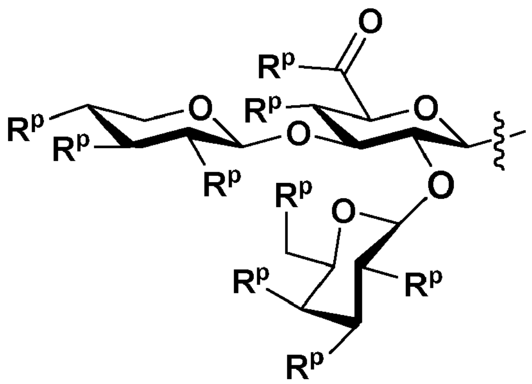

(VI)

wherein each occurrence of $R^p$ is independently hydrogen or $OR^q$;

wherein each occurrence of $R^q$ is independently hydrogen or an optionally substituted group selected from 6-10-

membered aryl, benzyl, $C_{1-6}$ aliphatic, or $C_{1-6}$ heteroaliphatic having 1-2 heteroatoms independently selected from the group consisting of nitrogen, oxygen, and sulfur; or two $R^q$ are taken together to form a 5-7-membered heterocyclic ring having 1-2 heteroatoms independently selected from the group consisting of nitrogen, oxygen, and sulfur;

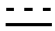 is a single or double bond;

V is H or $OR^x$;

Y is $CH_2$, -O-, -S-, -NR, or-NH-;

Z is a carbohydrate domain having the structure:

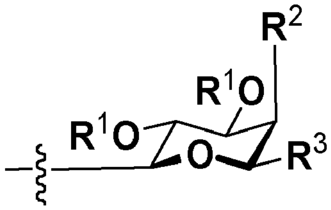

or

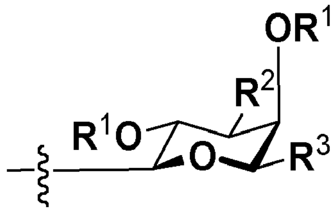

wherein each occurrence of $R^1$ is $R^X$ or a carbohydrate domain having the structure:

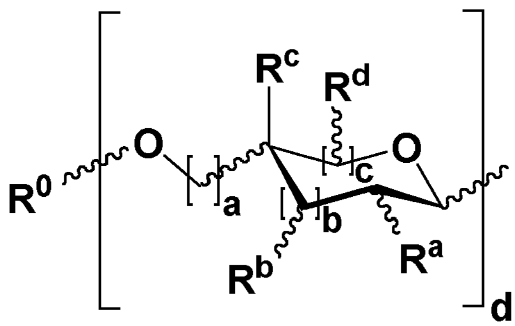

wherein:

each occurrence of a, b, and c is independently 0, 1, or 2;

d is an integer from 1-5, wherein each d bracketed structure may be the same or different; with the proviso that the d bracketed structure represents a furanose or a pyranose moiety, and the sum of b and c is 1 or 2;

$R^0$ is hydrogen; an oxygen protecting group selected from the group consisting of alkyl ethers, benzyl ethers, silyl ethers, acetals, ketals, esters, carbamates, and carbonates; or an optionally substituted moiety selected from the group consisting of acyl, $C_{1-10}$ aliphatic, $C_{1-6}$ heteroaliphatic, 6-10-membered aryl, arylalkyl, 5-10 membered heteroaryl having 1-4 heteroatoms independently selected from nitrogen, oxygen, or sulfur, 4-7 membered heterocyclyl having 1-2 heteroatoms independently selected from the group consisting of nitrogen, oxygen, and sulfur;

each occurrence of $R^a$, $R^b$, $R^c$, and $R^d$ is independently hydrogen, halogen, OH, OR, $OR^x$, $NR_2$, NHCOR, or an optionally substituted group selected from acyl, $C_{1-10}$ aliphatic, $C_{1-6}$ heteroaliphatic, 6-10-membered aryl, arylalkyl, 5-10-membered heteroaryl having 1-4 heteroatoms independently selected from nitrogen, oxygen, and sulfur; 4-7-membered heterocyclyl having 1-2 heteroatoms independently selected from the group consisting of nitrogen, oxygen, and sulfur;

$R^2$ is hydrogen, halogen, OH, OR, $OC(O)R^4$, $OC(O)OR^4$, $OC(O)NHR^4$, $OC(O)NRR^4$, $OC(O)SR^4$, $NHC(O)R^4$, $NRC(O)R^4$, $NHC(O)OR^4$, $NHC(O)NHR^4$, $NHC(O)NRR^4$, $NHR^4$, $N(R^4)_2$, $NHR^4$, $NRR^4$, $N_3$, or an optionally substituted group selected from $C_{1-10}$ aliphatic, $C_{1-6}$ heteroaliphatic, 6-10-membered aryl, arylalkyl, 5-10-membered heteroaryl having 1-4 heteroatoms independently selected from the group consisting of nitrogen, oxygen, and

sulfur, 4-7-membered heterocyclyl having 1-2 heteroatoms independently selected from the group consisting of nitrogen, oxygen, and sulfur;

$R^3$ is hydrogen, halogen, $CH_2OR^1$, or an optionally substituted group selected from the group consisting of acyl, $C_{1-10}$ aliphatic, $C_{1-6}$ heteroaliphatic, 6-10-membered aryl, arylalkyl, 5-10-membered heteroaryl having 1-4 heteroatoms independently selected from the group consisting of nitrogen, oxygen, and sulfur, 4-7-membered heterocyclyl having 1-2 heteroatoms independently selected from the group consisting of nitrogen, oxygen, and sulfur;

$R^4$ is $-T-R^z$, $-C(O)-T-R^z$, $-NH-T-R^z$, $-O-T-R^z$, $-S-T-R^z$, $-C(O)NH-T-R^z$, $C(O)O-T-R^z$, $C(O)S-T-R^z$, $C(O)NH-T-O-T-R^z$, $- O-T-R^z$, $-T-O-T-R^z$, $-T-S-T-R^z$, or

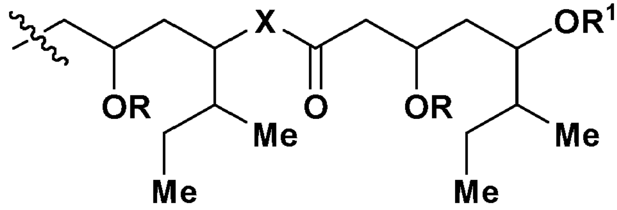

,

wherein

X is -O-, -NR-, or $T-R^z$;

T is a covalent bond or a bivalent $C_{1-26}$ saturated or unsaturated, straight or branched, aliphatic or heteroaliphatic chain; and

$R^z$ is hydrogen, halogen, -OR, $-OR^x$, $-OR^1$, -SR, $NR_2$, -C(O)OR, -C(O)R, -NHC(O)R, - NHC(O)OR, -NC(O)OR, or an optionally substituted group selected from acyl, arylalkyl, heteroarylalkyl, $C_{1-6}$ aliphatic, 6-10-membered aryl, 5-10-membered heteroaryl having 1-4 heteroatoms independently selected from nitrogen, oxygen, or sulfur, 4-7-membered heterocyclyl having 1-2 heteroatoms independently selected from the group consisting of nitrogen, oxygen, and sulfur;

each occurrence of $R^x$ is independently hydrogen or an oxygen protecting group selected from the group consisting of alkyl ethers, benzyl ethers, silyl ethers, acetals, ketals, esters, carbamates, and carbonates;

each occurrence of R is independently hydrogen, an optionally substituted group selected from acyl, arylalkyl, 6-10-membered aryl, $C_{1-6}$ aliphatic, or $C_{1-6}$ heteroaliphatic having 1-2 heteroatoms independently selected from the group consisting of nitrogen, oxygen, and sulfur, or,

two R on the same nitrogen atom are taken with the nitrogen atom to form a 4-7-membered heterocyclic ring having 1-2 heteroatoms independently selected from the group consisting of nitrogen, oxygen, and sulfur; and

- a compound of general formula (II) or a pharmaceutically acceptable salt thereof

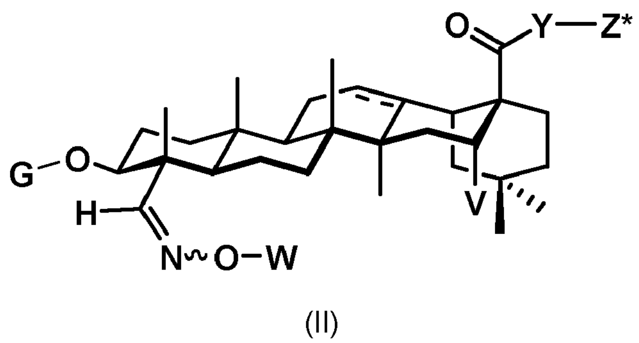

(II)

wherein

- - -.

,G, V and Y take the meanings as in formula (I);

W is H or a moiety comprising either i) at least one T cell epitope, ii) at least one B cell epitope or iii) at least one T cell epitope and at least one B cell epitope;

Z* represents that Z, as defined in formula (I), is optionally conjugated with a moiety comprising either i) at least one T cell epitope, ii) at least one B cell epitope or iii) at least one T cell epitope and at least one B cell epitope;

with the proviso that at least one T cell epitope and at least one B cell epitope are present in the compound of formula (II);

wherein the at least one T cell epitope is independently selected in every instance from a helper T cell epitope or a CD8 epitope.

Another aspect of the present invention relates to a compound selected from:

- a compound of general formula (III) or a pharmaceutically acceptable salt thereof

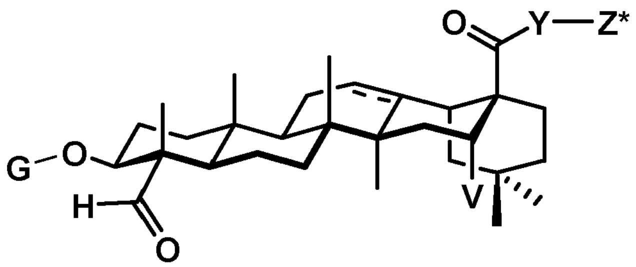

(III)

wherein

- - -,

, G, V and Y take the meanings as previously defined in formula (I) and Z* represents that Z, as defined in formula (I), is conjugated with a moiety comprising either i) at least one T cell epitope selected from a helper T cell epitope or a CD8 epitope, ii) at least one B cell epitope or iii) at least one T cell epitope selected from a helper T cell epitope or a CD8 epitope and at least one B cell epitope;
and

- a compound of general formula (IV) or a pharmaceutically acceptable salt thereof

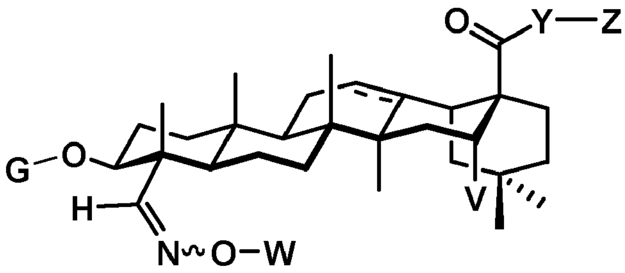

(IV)

wherein

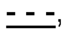,

, G, V, Y and Z take the meanings as previously defined in formula (I) and W is a moiety comprising either i) at least one T cell epitope selected from a helper T cell epitope or a CD8 epitope, ii) at least one B cell epitope or iii) at least one T cell epitope selected from a helper T cell epitope or a CD8 epitope and at least one B cell epitope.

Another aspect of the present invention relates to a method of synthesizing the compound of formula (I) or formula (II).

A method of synthesizing the compound of formula (I) comprises:

a) providing a compound of formula (V) or a salt thereof

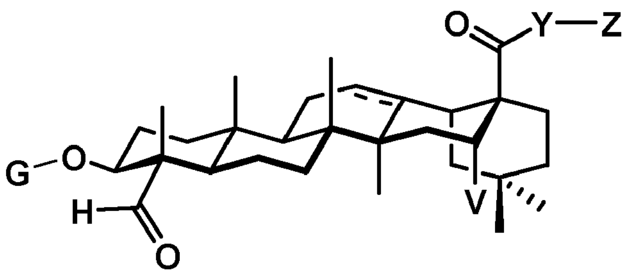

(V)

wherein

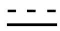

, G, V, Y and Z take the meanings as previously defined in formula (I); and

b) reacting the compound of formula (V) with $NH_2OH$ or a salt thereof, to form a compound of formula (I) or a salt thereof

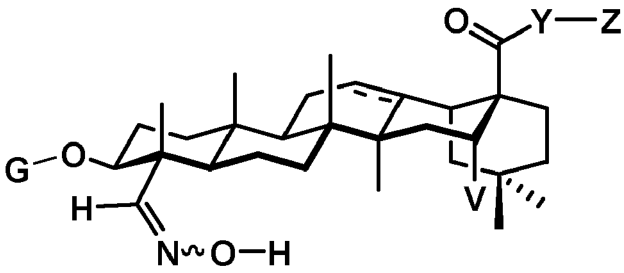

(I).

A method of synthesizing the compound of formula (II) is selected from:

- a method comprising:

a) providing a compound of formula (V) or a salt thereof

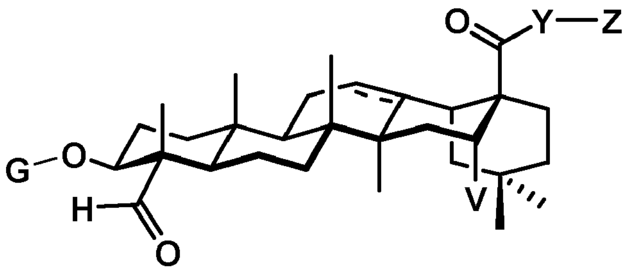

(V)

wherein

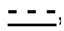

, G, V, Y and Z take the meanings as previously defined in formula (I);

b) conjugating the compound of formula (V) or a salt thereof with a moiety comprising either i) at least one T cell epitope selected from a helper T cell epitope or a CD8 epitope, ii) at least one B cell epitope or iii) at least one T cell epitope selected from a helper T cell epitope or a CD8 epitope and at least one B cell epitope, to form a compound of formula (III) or a salt thereof

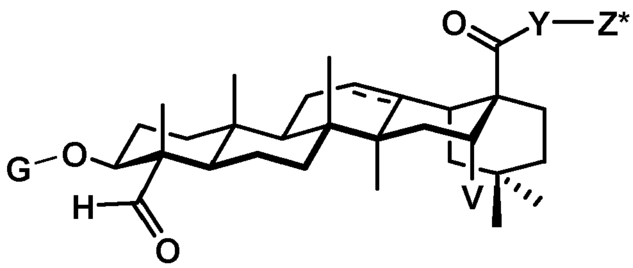

(III);

c) reacting the compound of formula (III) or a salt thereof with a compound of formula

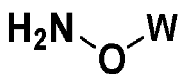

or a salt thereof, wherein W is H or a moiety comprising either i) at least one T cell epitope selected from a helper T cell epitope or a CD8 epitope, ii) at least one B cell epitope or iii) at least one T cell epitope selected from a helper T cell epitope or a CD8 epitope and at least one B cell epitope, to form a compound of formula (II) or a salt thereof

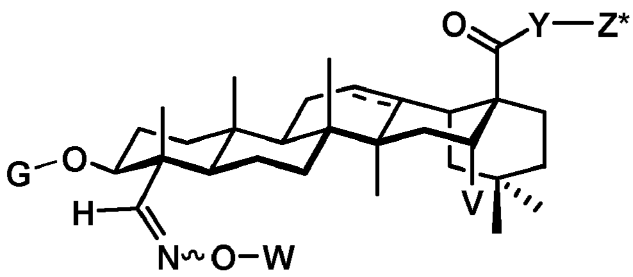

(II);

- a method comprising:

a) providing a compound of formula (V) or a salt thereof

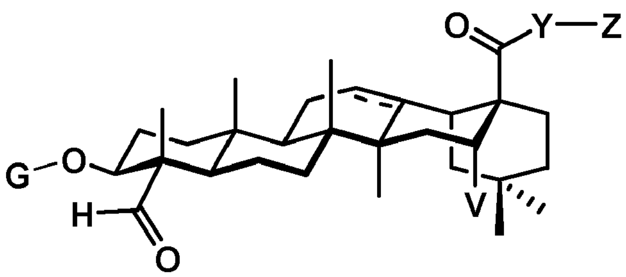

(V)

wherein

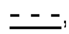

, G, V, Y and Z take the meanings as previously defined in formula (I);
b) reacting the compound of formula (V) or a salt thereof with a compound of formula

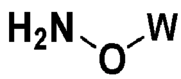

or a salt thereof, wherein W is H or a moiety comprising either i) at least one T cell epitope selected from a helper T cell epitope or a CD8 epitope, ii) at least one B cell epitope or iii) at least one T cell epitope selected from a helper T cell epitope or a CD8 epitope and at least one B cell epitope, to form a compound of formula (IV) or a salt thereof

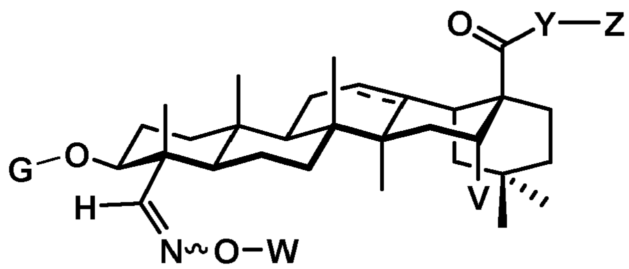

(IV)

wherein the compound of formula (IV) when W is a moiety comprising at least one T cell epitope selected from a helper T cell epitope or a CD8 epitope and at least one B cell epitope falls under formula (II);

c) optionally, conjugating the compound of formula (IV) or a salt thereof with a moiety comprising either i) at least one T cell epitope selected from a helper T cell epitope or a CD8 epitope, ii) at least one B cell epitope or iii) at least one T cell epitope selected from a helper T cell epitope or a CD8 epitope and at least one B cell epitope, to form a compound of formula (II) or a salt thereof

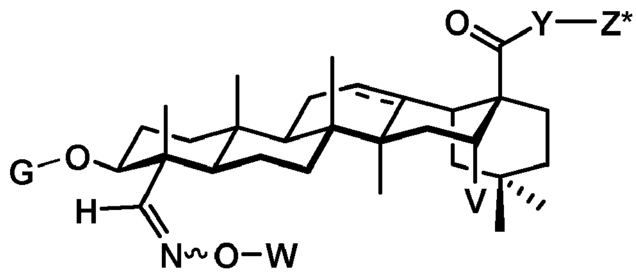

(II).

Another aspect of the present invention relates to a pharmaceutical composition comprising:

- a compound of formula (I) or a pharmaceutically acceptable salt thereof as previously defined, a pharmaceutically acceptable carrier and an antigen, or
- a compound of formula (II) or a pharmaceutically acceptable salt thereof as previously defined and a pharmaceutically acceptable carrier.

Another aspect of the present invention relates to a compound of formula (I), formula (II) or a pharmaceutically acceptable salt thereof as previously defined for use in medicine.

Another aspect of the present invention relates to a compound of formula (I), formula (II) or a pharmaceutically acceptable salt thereof or a pharmaceutical composition thereof for use in the treatment and/or prevention of cancer, an infectious disease or a neurodegenerative disease.

These aspects and preferred embodiments thereof are additionally also defined hereinafter in the detailed description and in the claims.

## BRIEF DESCRIPTION OF THE FIGURES

To better understand the invention, its objects and advantages, the following figures are attached to the specification in which the following is depicted:

**Figure 1** shows the complete structures vaccine structures **1-4.** Compounds **1** and **2** are di-component synthetic vaccines including a saponin adjuvant core scaffold based on quillaic acid connected to the MUC1 **(1)** or TnMUC1 **(2)** B-cell epitopes through a polyethylene glycol (PEG) spacer. Compounds **3** and **4** are tri-component vaccines including the KLFAVWKITYKDT peptide sequence from poliovirus [PV(103-115)] as helper T cell epitope. Such peptide sequence has been covalently attached to saponin's quillaic acid triterpene via an oxime linker through aldehyde functionalization. Compounds' abbreviated name: **1** →**QA-MUC1** ; **2** →**QA-TnMUC1** ; **3** → **QA(PV)-MUC1** ; **4** → **QA(PV)-TnMUC1.**

**Figure 2** shows the complete structures of the synthetic vaccine constructs 26-29. Compounds **26** and **28** are di-component synthetic conjugates including a saponin adjuvant core scaffold based on quillaic acid connected to the Tn-Thr **(26)** or Gb3 **(28)** B-cell epitopes through a polyethylene glycol (PEG) spacer. Compounds **27** and **29** are tri-component conjugates including the KLFAVWKITYKDT peptide sequence from poliovirus [PV(103-115)] as helper T cell epitope. Such peptide sequence has been covalently attached to the saponin quillaic acid triterpene at its C4 aldehyde substituent via an oxime linkage. Compounds' abbreviated name: **26** → **QA-Tn-Thr** ; **27** → **QA(PV)-Tn-Thr** ; **28** → **QA-Gb3; 29** → **QA(PV)-Gb3.**

**Figure 3** shows the preparation of MUC1- and TnMUC1-functionalized BSA (**23** and **24**).

**Figure 4** shows the production of total IgG of anti-OVA antibodies in mouse sera (endpoint) resulting from *in vivo* experiment 1 *(infra)* in order to compare the adjuvant activities of the prior art compound **13** and the compound of the invention **25.** Total IgG values were calculated using chicken ovalbumin (OVA) as a model antigen for four groups of mice treated with the following subcutaneous injections: i) toxin-free OVA antigen (20 μg) in combination with the known QA-based adjuvant **13** (50 μg) (identified as QA + OVA), ii) toxin-free OVA antigen (20 μg) in combination with the new adjuvant **25** (50 μg) (identified as QA(oxime) + OVA), iii) toxin-free OVA (20 μg) alone as no-adjuvant control group (identified as OVA) and iv) PBS only as negative control (identified as PBS).

**Figure 5** is a representation of the immunization schedule followed in *in vivo* experiment 2 *(infra)* including sera bleeding (day 21), endpoint sera collection (day 42), and immunization groups (A-D) for MUC1-containing synthetic antigens. Mice were immunized in the experiment with the following synthetic molecules: (A) the MUC1 peptide epitope alone (compound **5**) [Group A], (B) a mixture of the MUC1 peptide **(5)** and the PV Th epitope (compound **22**) [Group B], (C) a mixture of the di-component saponin-MUC1 conjugate **1** and the Th epitope **(22)** [Group C], and (D) the tri-component saponin construct QA(PV)-MUC1 **(3)** incorporating both the MUC1 and the PV peptides [Group D].

**Figure** 6 shows anti-MUC1 total IgG antibody titers from a) middle-point (day 21), and b) end-point (day 42) sera resulting from *in vivo* experiment 2 *(infra).* ELISA plates were coated with MUC1-functionalized BSA conjugates (conjugate **23**).

**Figure 7** shows IgG subtyping of anti-MUC1 antibodies in endpoint (day 42) sera resulting from *in vivo* experiment 2 *(infra).*

**Figure 8** is a representation of the immunization schedule followed in *in vivo* experiment 3 *(infra)* including sera bleeding (day 10), endpoint sera collection (day 24), and immunization groups (A-G) for TnMUC1-containing synthetic antigens. Three immunization groups received the free TnMUC1 glycopeptide **(6),** which was co-administered with either: (i) the known QA-based saponin adjuvant **13** [Group B], (ii) a mixture of QA saponin **13** and PV epitope **22** [Group C], or (iii) a newly synthesized QA saponin-(PV) conjugate, that is molecule **14** [Group F]. Two immunization groups were administered with the di-component QA saponin-TnMUC1 construct **2:** either alone [Group D] or mixed with the PV epitope **22** [Group E], and another group was injected with the tri-component QA(PV)-TnMUC1 construct **4** incorporating both PV and TnMUC1 peptides (**22** and **6,** respectively) [Group G]. Finally, a negative control group was included receiving PBS only [Group A].

**Figure 9** shows anti-TnMUC1 total IgG antibody titers for endpoint (day 24) sera resulting from *in vivo* experiment 3 *(infra).*

**Figure 10** shows IgG subtyping of anti-TnMUC1 antibodies in endpoint (day 24) sera resulting from *in vivo* experiment 3 *(infra).*

## DETAILED DESCRIPTION OF THE INVENTION

The clinical success of anticancer, antiviral, and antimicrobial vaccines critically depends on the identification of, and access to, novel potent adluvants with attenuated toxicity. In this context, specific fractions from extracts of the bark of *Quillaja saponaria* (QS) have proven to be exceedingly powerful adjuvants in immunotherapy. The QS-21 fraction is one of the most potent adjuvants known. QS-21 stimulates both antibody-based humoral immune responses (Th2) and cellular immunity (Th1), including production of antigen-specific cytotoxic T-lymphocytes. Vaccines containing QS-21, either alone in purified form or as a major component of adjuvant mixtures (e.g., Quil A, ISCOMs, ISCOMATRIX, AS01, AS02), have been investigated in clinical trials for cancers (melanoma, sarcoma, breast, prostate, ovarian, lung),

infectious diseases (hepatitis, HIV, malaria, tuberculosis) and Alzheimer's disease.

Despite its remarkable potency and extensive clinical investigation, which has resulted in its approval as part of recently licensed vaccines against malaria and shingles disease, QS-21 suffers from several limitations. First, access to homogeneous QS-21 is limited due to an exceedingly low-yielding isolation and heterogeneity of crude extracts from *Quillaja saponaria.* Second, QS-21 is associated with clinical toxicity including swelling and erythema at the injection site, and systemic flu-like symptoms. Third, QS-21 undergoes spontaneous hydrolysis of the acyl chain domain ester linkages, producing adjuvant-inactive and hemolytic byproducts, complicating formulation and storage. Finally, the mechanisms of action of QS-21 are poorly understood, hindering rational design of improved variants and optimal matching of adjuvants with vaccine antigens based on desired immunological end points. The inherent liabilities of QS-21 highlight the need for improved analogues.

After extensive research, the present inventors have unexpectedly found new saponin compounds in which the native C4-aldehyde substituent of QS-21 and other prior art variants is replaced with an oxime group. The compounds of the invention act as potent adjuvants when co-administered with antigens, inducing an antibody response significantly higher than analogous saponins that have a C4-aldehyde substituent. Besides, it has been found that thanks to an oxime linkage at the C4-position as well as conjugation through the chain of the carbohydrate domain the saponin scaffold may be functionalized with substances capable of stimulating an immune response, thus resulting in self-adjuvanting vaccines.

Unless defined otherwise, all technical and scientific terms and expressions used herein have the same meaning as commonly understood to one of ordinary skill in the art to which this disclosure belongs.

The term "aliphatic" or "aliphatic group" as used herein, means a straight-chain (i.e., unbranched) or branched, substituted or unsubstituted hydrocarbon chain that is completely saturated or that contains one or more units of unsaturation, or a monocyclic hydrocarbon or bicyclic hydrocarbon that is completely saturated or that contains one or more units of unsaturation, but which is not aromatic (also referred to herein as "carbocycle," "cycloaliphatic" or "cycloalkyl"), that has a single point of attachment to the rest of the molecule. Unless otherwise specified, aliphatic groups contain 1-12 aliphatic carbon atoms. In some embodiments, aliphatic groups contain 1-6 aliphatic carbon atoms. In some embodiments, aliphatic groups contain 1-5 aliphatic carbon atoms. In other embodiments, aliphatic groups contain 1-4 aliphatic carbon atoms. In still other embodiments, aliphatic groups contain 1-3 aliphatic carbon atoms, and in yet other embodiments, aliphatic groups contain 1-2 aliphatic carbon atoms. In some embodiments, "cycloaliphatic" (or "carbocycle" or "cycloalkyl") refers to a monocyclic $C_3$-$C_6$ hydrocarbon that is completely saturated or that contains one or more units of unsaturation, but which is not aromatic, that has a single point of attachment to the rest of the molecule. Suitable aliphatic groups include, but are not limited to, linear or branched, substituted or unsubstituted alkyl, alkenyl, alkynyl groups and hybrids thereof such as (cycloalkyl)alkyl, (cycloalkenyl)alkyl or (cycloalkyl)alkenyl.

The term "lower alkyl" refers to a $C_{1-4}$ straight or branched alkyl group. Exemplary lower alkyl groups are methyl, ethyl, propyl, isopropyl, butyl, isobutyl, and tert-butyl.

The term "lower haloalkyl" refers to a $C_{1-4}$ straight or branched alkyl group that is substituted with one or more halogen atoms.

The term "heteroatom" means one or more of oxygen, sulfur, nitrogen, phosphorus, or silicon (including, any oxidized form of nitrogen, sulfur, phosphorus, or silicon; the quaternized form of any basic nitrogen or; a substitutable nitrogen of a heterocyclic ring, for example N (as in 3,4-dihydro-2*H*-pyrrolyl), NH (as in pyrrolidinyl) or $NR^+$ (as in N-substituted pyrrolidinyl)).

The term "unsaturated," as used herein, means that a moiety has one or more units of unsaturation.

As used herein, the term "bivalent $C_{1-12}$ (or $C_{1-26}$, $C_{1-16}$, $C_{1-8}$) or saturated or unsaturated, straight or branched, hydrocarbon chain," refers to bivalent alkylene, alkenylene, and alkynylene chains that are straight or branched as defined herein.

The term "alkylene" refers to a bivalent alkyl group. An "alkylene chain" is a polymethylene group, i.e., $-(CH_2)_n-$, wherein n is a positive integer, preferably from 1 to 30, from 1 to 28, from 1 to 26, from 1 to 24, from 1 to 22, from 1 to 20, from 1 to 18, from 1 to 16, from 1 to 14, from 1 to 12, from 1 to 10, from 1 to 8, from 1 to 6, from 1 to 4, from 1 to 3, from 1 to 2, or from 2 to 3. A substituted alkylene chain is a polymethylene group in which one or more methylene hydrogen atoms are replaced with a substituent. Suitable substituents include those described below for a substituted aliphatic group.

The term "alkenylene" refers to a bivalent alkenyl group. A substituted alkenylene chain is a polymethylene group containing at least one double bond in which one or more hydrogen atoms are replaced with a substituent. Suitable substituents include those described below for a substituted aliphatic group.

The term "alkynylene" refers to a bivalent alkynyl group. A substituted alkynylene chain is a polymethylene group containing at least one double bond in which one or more hydrogen atoms are replaced with a substituent. Suitable substituents include those described below for a substituted aliphatic group.

The term "acyl," used alone or a part of a larger moiety, refers to groups formed by removing a hydroxy group from a carboxylic acid.

The term "halogen" means F, Cl, Br, or I.

The terms "aralkyl" and "arylalkyl" are used interchangeably and refer to alkyl groups (e.g. $C_{1-6}$ alkyl) in which a hydrogen atom has been replaced with an aryl group (e.g. 6-10-membered aryl). Such groups include, without limitation, benzyl, cinnamyl, and dihyrocinnamyl.

The term "aryl" used alone or as part of a larger moiety as in "aralkyl", "aralkoxy", or "aryloxyalkyl", refers to monocyclic or bicyclic ring systems having a total of five to fourteen ring members, wherein at least one ring in the system is aromatic and wherein each ring in the system contains 3 to 7 ring members. The term "aryl" may be used interchangeably with the term "aryl ring".

In certain embodiments of the present invention, "aryl" refers to an aromatic ring system which includes, but not limited to, phenyl, biphenyl, naphthyl, anthracyl and the like, which may bear one or more substituents. Also, included within the scope of the term "aryl," as it is used herein, is a group in which an aromatic ring is fused to one or more non-aromatic rings, such as indanyl, phthalimidyl, naphthimidyl, phenanthridinyl, or tetrahydronaphthyl, and the like.

The terms "heteroaryl" and "heteroar-" used alone or as part of a larger moiety, e.g., "heteroaralkyl" or "heteroaralkoxy" refer to groups having 5 to 10 ring atoms, preferably 5, 6, or 9 ring atoms; having 6, 10, or 14 $\pi$ electrons shared in a cyclic array; and having, in addition to carbon atoms, from one to five heteroatoms. The term "heteroatom" refers to nitrogen, oxygen, or sulfur, and includes any oxidized form of nitrogen or sulfur, and any quaternized form of a basic nitrogen. Heteroaryl groups include, without limitation, thienyl, furanyl, pyrrolyl, imidazolyl, pyrazolyl, triazolyl, tetrazolyl, oxazolyl, isoxazolyl, oxadiazolyl, thiazolyl, isothiazolyl, thiadiazolyl, pyridyl, pyridazinyl, pyrimidinyl, pyrazinyl, indolizinyl, purinyl, naphthyridinyl, and pteridinyl. The terms "heteroaryl" and "heteroar-", as used herein, also include groups in which a heteroaromatic ring is fused to one or more aryl, cycloaliphatic, or heterocyclyl rings, where the radical or point of attachment is on the heteroaromatic ring. Nonlimiting examples include indolyl, isoindolyl, benzothienyl, benzofuranyl, dibenzofuranyl, indazolyl, benzimidazolyl, benzthiazolyl, quinolyl, isoquinolyl, cinnolinyl, phthalazinyl, quinazolinyl, quinoxalinyl, 4*H*-quinolizinyl, carbazolyl, acridinyl, phenazinyl, phenothiazinyl, phenoxazinyl, tetrahydroquinolinyl, tetrahydroisoquinolinyl, and pyrido[2,3-b]-1,4-oxazin-3(4*H*)-one. A heteroaryl group may be mono- or bicyclic. The term "heteroaryl" may be used interchangeably with the terms "heteroaryl ring", "heteroaryl group" or "heteroaromatic" any of which terms include rings that are optionally substituted. The terms "heteroaralkyl" and "heteroarylalkyl" refer to an alkyl group substituted by a heteroaryl moiety, wherein the alkyl and heteroaryl portions independently are optionally substituted.

The term "heteroaliphatic" as used herein, means aliphatic groups wherein one or two carbon atoms are independently replaced by one or more of oxygen, sulfur, nitrogen, or phosphorus. Heteroaliphatic groups may be substituted or unsubstituted, branched or unbranched, cyclic or acyclic, and include "heterocycle", "heterocyclyl", "heterocycloaliphatic" or "heterocyclic" groups.

As used herein, the terms "heterocycle", "heterocyclyl", "heterocyclic radical" and "heterocyclic ring" are used interchangeably and refer to a stable 5- to 7-membered monocyclic or 7-10-membered bicyclic heterocyclic moiety that is either saturated or partially unsaturated, and having, in addition to carbon atoms, one or more, preferably one to four, heteroatoms, as defined above. When used in reference to a ring atom of a heterocycle, the term "nitrogen" includes a substituted nitrogen. As an example, in a saturated or partially unsaturated ring having 0-3 heteroatoms selected from oxygen, sulfur or nitrogen, the nitrogen may be N (as in 3,4-dihydro-2*H*-pyrrolyl), NH (as in pyrrolidinyl), or $^{+}NR$ (as in N-substituted pyrrolidinyl).

A heterocyclic ring can be attached to its pendant group at any heteroatom or carbon atom that results in a stable structure and any of the ring atoms can be optionally substituted. Examples of such saturated or partially unsaturated heterocyclic radicals include, without limitation, tetrahydrofuranyl, tetrahydrothiophenyl pyrrolidinyl, piperidinyl, pyrrolinyl, tetrahydroquinolinyl, tetrahydroisoquinolinyl, decahydroquinolinyl, oxazolidinyl, piperazinyl, dioxanyl, dioxolanyl, diazepinyl, oxazepinyl, thiazepinyl, morpholinyl, and quinuclidinyl. The terms "heterocycle", "heterocyclyl", "heterocyclyl ring", "heterocyclic group", "heterocyclic moiety" and "heterocyclic radical" are used interchangeably herein, and also include groups in which a heterocyclyl ring is fused to one or more aryl, heteroaryl, or cycloaliphatic rings, such as indolinyl, 3*H*-indolyl, chromanyl, phenanthridinyl, or tetrahydroquinolinyl, where the radical or point of attachment is on the heterocyclyl ring. A heterocyclyl group may be mono- or bicyclic. The term "heterocyclylalkyl" refers to an alkyl group substituted by a heterocyclyl, wherein the alkyl and heterocyclyl portions independently are optionally substituted.

As used herein, the term "partially unsaturated" refers to a ring moiety that includes at least one double or triple bond. The term "partially unsaturated" is intended to encompass rings having multiple sites of unsaturation but is not intended to include aryl or heteroaryl moieties, as herein defined.

In another aspect, the present invention provides "pharmaceutically acceptable" compositions, which comprise a therapeutically effective amount of one or more of the compounds described herein, formulated together with one or more pharmaceutically acceptable carriers (additives) and/or diluents. As described in detail, the pharmaceutical compositions of the present invention may be specially formulated for administration by injection.

The phrase "pharmaceutically acceptable" is employed herein to refer to those compounds, materials, compositions, and/or dosage forms which are, within the scope of sound medical judgment, suitable for use in contact with the

tissues of human beings and animals without excessive toxicity, irritation, allergic response, or other problem or complication, commensurate with a reasonable benefit/risk ratio.

The phrase "pharmaceutically acceptable carrier" as used herein means a pharmaceutically acceptable material, composition or vehicle, such as a liquid or solid filler, diluent, excipient, or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; pH buffered solutions; polyesters, polycarbonates and/or polyanhydrides; and other non-toxic compatible substances employed in pharmaceutical formulations.

As used herein, the term "pharmaceutically acceptable salt" refers to those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge et al., describe pharmaceutically acceptable salts in detail in J. Pharmaceutical Sciences, 1977, 66, 1-19, incorporated herein by reference. Pharmaceutically acceptable salts of the compounds of this invention include those derived from suitable inorganic and organic acids and bases. Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, pivalate, propionate, stearate, succinate, sulfate, tartrate, thiocyanate, p-toluenesulfonate, undecanoate, valerate salts, and the like.

In other cases, the compounds of the present invention may contain one or more acidic functional groups and, thus, are capable of forming pharmaceutically acceptable salts with pharmaceutically acceptable bases. The term "pharmaceutically acceptable salts" in these instances refers to the relatively non-toxic, inorganic and organic base addition salts of compounds of the present invention. These salts can likewise be prepared in situ in the administration vehicle or the dosage form manufacturing process, or by separately reacting the purified compound in its free acid form with a suitable base, such as the hydroxide, carbonate or bicarbonate of a pharmaceutically acceptable metal cation, with ammonia, or with a pharmaceutically acceptable organic primary, secondary, tertiary, or quaternary amine. Salts derived from appropriate bases include alkali metal, alkaline earth metal, ammonium and $N^+(C_{1-4}alkyl)_4$ salts. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, lower alkyl sulfonate and aryl sulfonate. Representative organic amines useful for the formation of base addition salts include ethylamine, diethylamine, ethylenediamine, ethanolamine, diethanolamine, piperazine and the like. (See, for example, Berge et al., *supra*).

Unless otherwise stated, structures depicted herein are also meant to include all isomeric (e.g., enantiomeric, diastereomeric, and geometric (or conformational)) forms of the structure; for example, the R and S configurations for each stereocenter, Z and E double bond isomers, and Z and E conformational isomers. Therefore, single stereochemical isomers as well as enantiomeric, diastereomeric, and geometric (or conformational) mixtures of the present compounds are within the scope of the invention. Unless otherwise stated, all tautomeric forms of the compounds of the invention are within the scope of the invention.

Provided compounds may comprise one or more saccharide moieties. Unless otherwise specified, both D- and L-configurations, and mixtures thereof, are within the scope of the invention. Unless otherwise specified, both $\alpha$- and $\beta$-linked embodiments, and mixtures thereof, are contemplated by the present invention.

If, for instance, a particular enantiomer of a compound of the present invention is desired, it may be prepared by asymmetric synthesis, chiral chromatography, or by derivation with a chiral auxiliary, where the resulting diastereomeric mixture is separated and the auxiliary group cleaved to provide the pure desired enantiomers. Alternatively, where the molecule contains a basic functional group, such as amino, or an acidic functional group, such as carboxyl, diastereomeric salts are formed with an appropriate optically active acid or base, followed by resolution of the diastereomers thus formed

by fractional crystallization or chromatographic means well known in the art, and subsequent recovery of the pure enantiomers.

Additionally, unless otherwise stated, structures depicted herein are also meant to include compounds that differ only in the presence of one or more isotopically enriched atoms. For example, compounds having the present structures including the replacement of hydrogen by deuterium or tritium, or the replacement of a carbon by a $^{13}C$- or $^{14}C$-enriched carbon are within the scope of this invention. Such compounds are useful, for example, as analytical tools, as probes in biological assays, or as therapeutic agents in accordance with the present invention.

One of ordinary skill in the art will appreciate that the synthetic methods, as described herein, utilize a variety of protecting groups. By the term "protecting group," as used herein, it is meant that a particular functional moiety, e.g., O, S, or N, is masked or blocked, permitting, if desired, a reaction to be carried out selectively at another reactive site in a multifunctional compound. In preferred embodiments, a protecting group reacts selectively in good yield to give a protected substrate that is stable to the projected reactions; the protecting group is preferably selectively removable by readily available, preferably non-toxic reagents that do not attack the other functional groups; the protecting group forms a separable derivative (more preferably without the generation of new stereogenic centers); and the protecting group will preferably have a minimum of additional functionality to avoid further sites of reaction. As detailed herein, oxygen, sulfur, nitrogen, and carbon protecting groups may be utilized. Suitable carboxyl protecting groups are well known in the art and include those described in detail in Protecting Groups in Organic Synthesis, T. W. Greene and P. G. M. Wuts, 3rd edition, John Wiley & Sons, 1999, the entirety of which is incorporated herein by reference. By way of non-limiting example, hydroxyl protecting groups include methyl, methoxylmethyl (MOM), methylthiomethyl (MTM), *t*-butylthiomethyl, (phenyldimethylsilyl)methoxymethyl (SMOM), benzyloxymethyl (BOM), p-methoxybenzyloxymethyl (PMBM), (4-methoxyphenoxy)methyl (p-AOM), guaiacolmethyl (GUM), *t*-butoxymethyl, 4-pentenyloxymethyl (POM), siloxymethyl, 2-methoxyethoxymethyl (MEM), 2,2,2-trichloroethoxymethyl, bis(2-chloroethoxy) methyl, 2-(trimethylsilyl)ethoxymethyl (SEMOR), tetrahydropyranyl (THP), 3-bromotetrahydropyranyl, tetrahydrothiopyranyl, 1-methoxycyclohexyl, 4-methoxytetrahydropyranyl (MTHP), 4-methoxytetrahydrothiopyranyl, 4-methoxytetrahydrothiopyranyl S,S-dioxide, 1-[(2-chloro-4-methyl)phenyl]-4-methoxypiperidin-4-yl (CTMP), 1,4-dioxan-2-yl, tetrahydrofuranyl, tetrahydrothiofuranyl, 2,3,3a,4,5,6,7,7a-octahydro-7,8,8-trimethyl-4,7-methanobenzofuran-2-yl, 1-ethoxyethyl, 1-(2-chloroethoxy)ethyl, 1-methyl-1-methoxyethyl, 1-methyl-1-benzyloxyethyl, 1-methyl-1-benzyloxy-2-fluoroethyl, 2,2,2-trichloroethyl, 2-trimethylsilylethyl, 2-(phenylselenyl)ethyl, *t*-butyl, allyl, *p*-chlorophenyl, *p*-methoxyphenyl, 2,4-dinitrophenyl, benzyl, *p*-methoxybenzyl, 3,4-dimethoxybenzyl, o-nitrobenzyl, *p*-nitrobenzyl, *p*-halobenzyl, 2,6-dichlorobenzyl, *p*-cyanobenzyl, *p*-phenylbenzyl, 2-picolyl, 4-picolyl, 3-methyl-2-picolyl N-oxido, diphenylmethyl, *p,p*'-dinitrobenzhydryl, 5-dibenzosuberyl, triphenylmethyl, α-naphthyldiphenylmethyl, *p*-methoxyphenyldiphenylmethyl, di(*p*-methoxyphenyl)phenylmethyl, tri(p- methoxyphenyl)methyl, 4-(4'-bromophenacyloxyphenyl)diphenylmethyl, 4,4',4"-tris(4,5-dichlorophthalimidophenyl)methyl, 4,4',4"-tris(levulinoyloxyphenyl)methyl, 4,4',4"-tris(benzoyloxyphenyl)methyl, 3-(imidazol-1-yl)bis(4',4"-dimethoxyphenyl)methyl, 1,1-bis(4-methoxyphenyl)-1'-pyrenylmethyl, 9-anthryl, 9-(9-phenyl)xanthenyl, 9-(9-phenyl-10-oxo)anthryl, 1,3-benzodithiolan-2-yl, benzisothiazolyl S,S-dioxido, trimethylsilyl (TMS), triethylsilyl (TES), triisopropylsilyl (TIPS), dimethylisopropylsilyl (IPDMS), diethylisopropylsilyl (DEIPS), dimethylthexylsilyl, *t*-butyldimethylsilyl (TBDMS), *t*-butyldiphenylsilyl (TBDPS), tribenzylsilyl, tri-p-xylylsilyl, triphenylsilyl, diphenylmethylsilyl (DPMS), t-butylmethoxyphenylsilyl (TBMPS), formate, benzoylformate, acetate, chloroacetate, dichloroacetate, trichloroacetate, trifluoroacetate, methoxyacetate, triphenylmethoxyacetate, phenoxyacetate, p-chlorophenoxyacetate, 3-phenylpropionate, 4-oxopentanoate (levulinate), 4,4-(ethylenedithio)pentanoate (levulinoyldithioacetal), pivaloate, adamantoate, crotonate, 4-methoxycrotonate, benzoate, p-phenylbenzoate, 2,4,6-trimethylbenzoate (mesitoate), alkyl methyl carbonate, 9-fluorenylmethyl carbonate (Fmoc), alkyl ethyl carbonate, alkyl 2,2,2-trichloroethyl carbonate (Troc), 2-(trimethylsilyl)ethyl carbonate (TMSEC), 2-(phenylsulfonyl)ethyl carbonate (Psec), 2-(triphenylphosphonio) ethyl carbonate (Peoc), alkyl isobutyl carbonate, alkyl vinyl carbonate alkyl allyl carbonate, alkyl p-nitrophenyl carbonate, alkyl benzyl carbonate, alkyl p-methoxybenzyl carbonate, alkyl 3,4-dimethoxybenzyl carbonate, alkyl o-nitrobenzyl carbonate, alkyl p-nitrobenzyl carbonate, alkyl S-benzyl thiocarbonate, 4-ethoxy-1-napththyl carbonate, methyl dithiocarbonate, 2-iodobenzoate, 4-azidobutyrate, 4-nitro-4-methylpentanoate, o-(dibromomethyl)benzoate, 2-formylbenzenesulfonate, 2-(methylthiomethoxy)ethyl, 4-(methylthiomethoxy)butyrate, 2-(methylthiomethoxymethyl)benzoate, 2,6-dichloro-4-methylphenoxyacetate, 2,6-dichloro-4-(1,1,3,3-tetramethylbutyl)phenoxyacetate, 2,4-bis(1,1-dimethylpropyl)phenoxyacetate, chlorodiphenylacetate, isobutyrate, monosuccinoate, (E)-2-methyl-2-butenoate, o-(methoxycarbonyl)benzoate, α-naphthoate, nitrate, alkyl *N,N,N',N'*-tetramethylphosphorodiamidate, alkyl *N*-phenylcarbamate, borate, dimethylphosphinothioyl, alkyl 2,4-dinitrophenylsulfenate, sulfate, methanesulfonate (mesylate), benzylsulfonate, and tosylate (Ts). For protecting 1,2- or 1,3-diols, the protecting groups include methylene acetal, ethylidene acetal, 1-t-butylethylidene ketal, 1-phenylethylidene ketal, (4-methoxyphenyl)ethylidene acetal, 2,2,2-trichloroethylidene acetal, acetonide, cyclopentylidene ketal, cyclohexylidene ketal, cycloheptylidene ketal, benzylidene acetal, p-methoxybenzylidene acetal, 2,4-dimethoxybenzylidene ketal, 3,4-dimethoxybenzylidene acetal, 2-nitrobenzylidene acetal, methoxymethylene acetal, ethoxymethylene acetal, dimethoxymethylene ortho ester, 1-methoxyethylidene ortho ester, 1-ethoxyethylidine ortho ester, 1,2-dimethoxyethylidene ortho ester, α-methoxybenzylidene ortho ester, 1-(*N*,*N*-dimethylamino)ethylidene derivative, α-(*N*,*N*'-dimethylamino)benzylidene derivative, 2-oxacyclopentylidene ortho ester, di-*t*-butylsilylene group (DTBS), 1,3-(1,1,3,3-tetraisopropyldisiloxanylidene) derivative (TIPDS), tetra-t-butoxydisiloxane-1,3-diylidene derivative (TBDS), cyclic carbonates, cyclic boronates, ethyl boronate, and phenyl boronate. Amino-protecting groups include methyl carbamate, ethyl carbamante, 9-fluorenylmethyl carbamate (Fmoc), 9-(2-sulfo)fluorenylmethyl carbamate, 9-(2,7-dibromo)fluoroenylmethyl carbamate, 2,7-di-*t*-butyl-[9-(10,10-dioxo-10,10,10,10-tetrahydrothioxanthyl)]methyl carbamate (DBD-Tmoc), 4-methoxyphenacyl carbamate (Phenoc), 2,2,2-trichloroethyl carbamate (Troc), 2-trimethylsilylethyl carbamate (Teoc), 2-phenylethyl carbamate (hZ), 1-(1-adamantyl)-1-methylethyl carbamate (Adpoc), 1,1-dimethyl-2-haloethyl carbamate, 1,1-dimethyl-2,2-dibromoethyl carbamate (DB-*t*-BOC), 1,1-dimethyl-2,2,2-trichloroethyl carbamate (TCBOC), 1-methyl-1-(4-biphenylyl)ethyl carbamate (Bpoc), 1-(3,5-di-*t*-butylphenyl)-1-methylethyl carbamate (t-Bumeoc), 2-(2'- and 4'-pyridyl)ethyl carbamate (Pyoc), 2-(*N,N*-dicyclohexylcarboxamido)ethyl carbamate, *t*-butyl carbamate (BOC), 1-adamantyl carbamate (Adoc), vinyl carbamate (Voc), allyl carbamate (Alloc), 1-isopropylallyl carbamate (Ipaoc), cinnamyl carbamate (Coc), 4-nitrocinnamyl carbamate (Noc), 8-quinolyl carbamate, *N*-hydroxypiperidinyl carbamate, alkyldithio carbamate, benzyl carbamate (Cbz), p-methoxybenzyl carbamate (Moz), p-nitrobenzyl carbamate, p-bromobenzyl carbamate, p-chlorobenzyl carbamate, 2,4-dichlorobenzyl carbamate, 4-methylsulfinylbenzyl carbamate (Msz), 9-anthrylmethyl carbamate, diphenylmethyl carbamate, 2-methylthioethyl carbamate, 2-methylsulfonylethyl carbamate, 2-(p-toluenesulfonyl)ethyl carbamate, [2-(1,3-dithianyl)]methyl carbamate (Dmoc), 4-methylthiophenyl carbamate (Mtpc), 2,4-dimethylthiophenyl carbamate (Bmpc), 2-phosphonioethyl carbamate (Peoc), 2-triphenylphosphonioisopropyl carbamate (Ppoc), 1,1-dimethyl-2-cyanoethyl carbamate, *m*-chloro-p-acyloxybenzyl carbamate, *p*-(dihydroxyboryl)benzyl carbamate, 5-benzisoxazolylmethyl carbamate, 2-(trifluoromethyl)-6-chromonylmethyl carbamate (Tcroc), m-nitrophenyl carbamate, 3,5-dimethoxybenzyl carbamate, *o*-nitrobenzyl carbamate, 3,4-dimethoxy-6-nitrobenzyl carbamate, phenyl(o-nitrophenyl)methyl carbamate, phenothiazinyl-(10)-carbonyl derivative, *N'*-p-toluenesulfonylaminocarbonyl derivative, *N'*-phenylaminothiocarbonyl derivative, *t*-amyl carbamate, S-benzyl thiocarbamate, p-cyanobenzyl carbamate, cyclobutyl carbamate, cyclohexyl carbamate, cyclopentyl carbamate, cyclopropylmethyl carbamate, *p*-decyloxybenzyl carbamate, 2,2-dimethoxycarbonyl vinyl carbamate, *o*-(*N,N*-dimethylcarboxamido)benzyl carbamate, 1,1-dimethyl-3-(*N,N*-dimethylcarboxamido)propyl carbamate, 1, 1-dimethylpropynyl carbamate, di(2-pyridyl)methyl carbamate, 2-furanylmethyl carbamate, 2-iodoethyl carbamate, isoborynl carbamate, isobutyl carbamate, isonicotinyl carbamate, *p*-(*p*'-methoxyphenylazo)benzyl carbamate, 1-methylcyclobutyl carbamate, 1-methylcyclohexyl carbamate, 1-methyl-1- cyclopropylmethyl carbamate, 1-methyl-1-(3,5-dimethoxyphenyl)ethyl carbamate, 1-methyl-1-(*p*-phenylazophenyl)ethyl carbamate, 1-methyl-1-phenylethyl carbamate, 1-methyl-1-(4-pyridyl)ethyl carbamate, phenyl carbamate, *p*-(phenylazo)benzyl carbamate, 2,4,6-tri-*t*-butylphenyl carbamate, 4-(trimethylammonium)benzyl carbamate, 2,4,6-trimethylbenzyl carbamate, formamide, acetamide, chloroacetamide, trichloroacetamide, trifluoroacetamide, phenylacetamide, 3-phenylpropanamide, picolinamide, 3-pyridylcarboxamide, *N*-benzoylphenylalanyl derivative, benzamide, p-phenylbenzamide, o-nitophenylacetamide, *o*-nitrophenoxyacetamide, acetoacetamide, (*N*'-dithiobenzyloxycarbonylamino)acetamide, 3-(*p*-hydroxyphenyl)propanamide, 3-(o-nitrophenyl)propanamide, 2-methyl-2-(*o*-nitrophenoxy)propanamide, 2-methyl-2-(*o*-phenylazophenoxy)propanamide, 4-chlorobutanamide, 3-methyl-3-nitrobutanamide, *o*-nitrocinnamide, *N*-acetylmethionine derivative, o-nitrobenzamide, *o*-(benzoyloxymethyl)benzamide, 4,5-diphenyl-3-oxazolin-2-one, *N*-phthalimide, *N*-dithiasuccinimide (Dts), N-2,3-diphenylmaleimide, *N*-2,5-dimethylpyrrole, *N*-1,1,4,4-tetramethyldisilylazacyclopentane adduct (STABASE), 5-substituted 1,3-dimethyl-1,3,5-triazacyclohexan-2-one, 5-substituted 1,3-dibenzyl-1,3,5-triazacyclohexan-2-one, 1 -substituted 3,5-dinitro-4-pyridone, *N*-methylamine, *N*-allylamine, *N*-[2-(trimethylsilyl)ethoxy]methylamine (SEM), *N*-3-acetoxypropylamine, *N*-(1-isopropyl-4-nitro-2-oxo-3-pyroolin-3-yl)-amine, quaternary ammonium salts, *N*-benzylamine, *N*-di(4-methoxyphenyl)methylamine, *N*-5-dibenzosuberylamine, *N*-triphenylmethylamine (Tr), *N*-[(4-methoxyphenyl)diphenylmethyl]amine (MMTr), *N*-9-phenylfluorenylamine (PhF), N-2,7-dichloro-9-fluorenylmethyleneamine, *N*-ferrocenylmethylamino (Fern), *N*-2-picolylamino *N*'-oxide, *N*-1,1-dimethylthiomethyleneamine, *N*-benzylideneamine, *N*-*p*-methoxybenzylideneamine, *N*-diphenylmethyleneamine, *N*-[(2-pyridyl)mesityl]methyleneamine, *N*-(*N*',*N*'-dimethylaminomethylene)amine, N',N'-isopropylidenediamine, *N*-p-nitrobenzylideneamine, N-salicylideneamine, N-5-chlorosalicylideneamine, *N*-(5-chloro-2-hydroxyphenyl)phenylmethyleneamine, N-cyclohexylideneamine, *N*-(5,5-dimethyl-3-oxo-1-cyclohexenyl)amine, N-borane derivative, N-diphenylborinic acid derivative, *N*-[phenyl(pentacarbonylchromium- or tungsten)carbonyl]amine, N-copper chelate, *N*-zinc chelate, *N*-nitroamine, *N*-nitrosoamine, amine *N*-oxide, diphenylphosphinamide (Dpp), dimethylthiophosphinamide (Mpt), diphenylthiophosphinamide (Ppt), dialkyl phosphoramidates, dibenzyl phosphoramidate, diphenyl phosphoramidate, benzenesulfenamide, o-nitrobenzenesulfenamide (Nps), 2,4-dinitrobenzenesulfenamide, pentachlorobenzenesulfenamide, 2-nitro-4-methoxybenzenesulfenamide, triphenylmethylsulfenamide, 3-nitropyridinesulfenamide (Npys), p-toluenesulfonamide (Ts), benzenesulfonamide, 2,3,6,-trimethyl-4-methoxybenzenesulfonamide (Mtr), 2,4,6-trimethoxybenzenesulfonamide (Mtb), 2,6-dimethyl-4-methoxybenzenesulfonamide (Pme), 2,3,5,6-tetramethyl-4-methoxybenzenesulfonamide (Mte), 4-methoxybenzenesulfonamide (Mbs), 2,4,6-trimethylbenzenesulfonamide (Mts), 2,6-dimethoxy-4-methylbenzenesulfonamide (iMds), 2,2,5,7,8-pentamethylchroman-6-sulfonamide (Pmc), methanesulfonamide (Ms), β-trimethylsilylethanesulfonamide (SES), 9-anthracenesulfonamide, 4-(4',8'-dimethoxynaphthylmethyl)benzenesulfonamide (DNMBS), benzylsulfonamide, trifluoromethylsulfonamide, and phenacylsulfonamide. Exemplary protecting groups are detailed herein, however,

it will be appreciated that the present invention is not intended to be limited to these protecting groups; rather, a variety of additional equivalent protecting groups can be readily identified using the above criteria and utilized in the method of the present invention. Additionally, a variety of protecting groups are described by Greene and Wuts (*supra*).

As described herein, compounds of the invention may contain "optionally substituted" moieties. In general, the term "substituted" whether preceded by the term "optionally" or not, means that one or more hydrogens of the designated moiety are replaced with a suitable substituent. Unless otherwise indicated, an "optionally substituted" group may have a suitable substituent at each substitutable position of the group, and when more than one position in any given structure may be substituted with more than one substituent selected from a specified group, the substituent may be either the same or different at every position. Combinations of substituents envisioned by this invention are preferably those that result in the formation of stable or chemically feasible compounds. The term "stable" as used herein, refers to compounds that are not substantially altered when subjected to conditions to allow for their production, detection, and, in certain embodiments, their recovery, purification, and use for one or more of the purposes disclosed herein.

Suitable monovalent substituents on a substitutable carbon atom of an "optionally substituted" group are independently halogen; $-(CH_2)_{0-4}R^{\circ}$; $-(CH_2)_{0-4}OR^{\circ}$; $-O(CH_2)_{0-4}R^{\circ}$, $-O-(CH_2)_{0-4}C(O)OR^{\circ}$; $-(CH_2)_{0-4}CH(OR^{\circ})_2$ ; $-(CH_2)_{0-4}SR^{\circ}$; $-(CH_2)_{0-4}Ph$, which may be substituted with R°; $-(CH_2)_{0-4}O(CH_2)_{0-1}Ph$, which may be substituted with R°; -CH=CHPh, which may be substituted with R°; $-(CH_2)_{0-4}O(CH_2)_{0-1}$-pyridyl which may be substituted with R°; $-NO_2$ ; -CN;-N(R°)C(S)NR°$_2$; $-(CH_2)_{0-4}N(R^{\circ})C(O)R^{\circ}$; -N(R°)N(R°)C(0)R°; -N(R°)N(R°)C(O)NR°$_2$;-N(R°)N(R°)C(O)OR°; $-(CH_2)_{0-4}C(O)R^{\circ}$; -C(S)R°; $-(CH_2)_{0-4}C(O)OR^{\circ}$; $-(CH_2)_{0-4}C(O)SR^{\circ}$; $-(CH_2)_{0-4}C(O)OSiR^{\circ}_3$; $-(CH_2)_{0-4}OC(O)R^{\circ}$; $-OC(O)(CH_2)_{0-4}SR$, -SC(S)SR°; $-(CH_2)_{0-4}SC(O)R^{\circ}$; $-(CH_2)_{0-4}C(O)NR^{\circ}_2$; -C(S)NR°$_2$; -C(S)SR°; -SC(S)SR°; $-(CH_2)_{0-4}OC(O)NR^{\circ}_2$; -C(O)N(OR°)R°; - C(O)C(O)R°; $-C(O)CH_2C(O)R^{\circ}$; -C(NOR°)R°; $-(CH_2)_{0-4}SSR^{\circ}$; $-(CH_2)_{0-4}S(O)_2R^{\circ}$; $-(CH_2)_{0-4}S(O)_2OR^{\circ}$; $-(CH_2)_{0-4}OS(O)_2R^{\circ}$; $-S(O)_2NR^{\circ}_2$; $-(CH_2)_{0-4}S(O)R^{\circ}$; $-N(R^{\circ})S(O)_2NR^{\circ}_2$; - $N(R^{\circ})S(O)_2R^{\circ}$; -N(OR°)R°; -C(NH)NR°$_2$; $-P(O)_2R^{\circ}$; -P(O)R°$_2$; -OP(O)R°$_2$; -OP(O)(OR°)$_2$;SiR°$_3$; -($C_{1-4}$ straight or branched)alkylene)O-N(R°)$_2$ ; or -($C_{1-4}$ straight or branched)alkylene)C(O)ON(R°)$_2$, wherein each R° may be substituted as defined below and is independently hydrogen, $C_{1-6}$ aliphatic, $-CH_2Ph$, $-O(CH_2)_{0-1}$ Ph, $-CH_2$-(5-6-membered heteroaryl ring), or a 5-6-membered saturated, partially unsaturated, or aryl ring having 0-4 heteroatoms independently selected from nitrogen, oxygen, or sulfur, or, notwithstanding the definition above, two independent occurrences of R° taken together with their intervening atom(s), form a 3-12-membered saturated, partially unsaturated, or aryl mono- or bicyclic ring having 0-4 heteroatoms independently selected from nitrogen, oxygen, or sulfur, which may be substituted as defined below.

Suitable monovalent substituents on R° (or the ring formed by taking two independent occurrences of R° together with their intervening atoms), are independently halogen, $-(CH_2)_{0-2}R^{\Delta}$, -(halo$R^{\Delta}$), $-(CH_2)_{0-2}OH$, $-(CH_2)_{0-2}OR^{\Delta}$, $-(CH_2)_{0-2}CH(OR^{\Delta})_2$; -O(halo$R^{\Delta}$), -CN, $-N_3$, $-(CH_2)_{0-2}C(O)R^{\Delta}$, $-(CH_2)_{0-2}C(O)OH$, $-(CH_2)_{0-2}C(O)OR^{\Delta}$, $-(CH_2)_{0-2}SR^{\Delta}$, $-(CH_2)_{0-2}SH$, $-(CH_2)_{0-2}NH_2$, $-(CH_2)_{0-2}NHR^{\Delta}$, $-(CH_2)_{0-2}NR^{\Delta}_2$, $-NO_2$, $-SiR^{\Delta}_3$, $-OSiR^{\Delta}_3$, $-C(O)SR^{\Delta}$, -($C_{1-4}$straight or branched alkylene)C(O)OR$^{\Delta}$, or -SSR. wherein each $R^{\Delta}$ is unsubstituted or where preceded by "halo" is substituted only with one or more halogens, and is independently selected from $C_{1-4}$ aliphatic, $-CH_2Ph$, $-O(CH_2)_{0-1}$ Ph, or a 5- 6-membered saturated, partially unsaturated, or aryl ring having 0-4 heteroatoms independently selected from nitrogen, oxygen, or sulfur. Suitable divalent substituents on a saturated carbon atom of R° include =O and =S.

Suitable divalent substituents on a saturated carbon atom of an "optionally substituted" group include the following: =O, =S, $=NNR^*_2$, =NNHC(O)R*, =NNHC(O)OR*, $=NNHS(O)_2R^*$, =NR*, =NOR*, $-O(C(R^*_2))_{2-3}O-$, or $-S(C(R^*_2))_{2-3}S-$, wherein each independent occurrence of R* is selected from hydrogen, $C_{1-6}$ aliphatic which may be substituted as defined below, or an unsubstituted 5-6-membered saturated, partially unsaturated, or aryl ring having 0-4 heteroatoms independently selected from nitrogen, oxygen, or sulfur. Suitable divalent substituents that are bound to vicinal substitutable carbons of an "optionally substituted" group include: $-O(CR^*_2)_{2-3}O-$, wherein each independent occurrence of R* is selected from hydrogen, $C_{1-6}$ aliphatic which may be substituted as defined below, or an unsubstituted 5-6-membered saturated, partially unsaturated, or aryl ring having 0-4 heteroatoms independently selected from nitrogen, oxygen, or sulfur.

Suitable substituents on the aliphatic group of R* include halogen, $-R^{\Delta}$, -(halo$R^{\Delta}$), -OH, $-OR^{\Delta}$, -O(halo$R^{\Delta}$), -CN, -C(O)OH, $-C(O)OR^{\Delta}$, $-NH_2$, $-NHR^{\Delta}$, $-NR^{\Delta}_2$, or $-NO_2$, wherein each $R^{\Delta}$ is unsubstituted or where preceded by "halo" is substituted only with one or more halogens, and is independently $C_{1-4}$ aliphatic, $-CH_2Ph$, $-O(CH_2)_{0-1}$ Ph, or a 5-6-membered saturated, partially unsaturated, or aryl ring having 0-4 heteroatoms independently selected from nitrogen, oxygen, or sulfur.

Suitable substituents on a substitutable nitrogen of an "optionally substituted" group include $-R^{\dagger}$, $-NR^{\dagger}_2$, $-C(O)R^{\dagger}$, $-C(O)OR^{\dagger}$, $-C(O)C(O)R^{\dagger}$, $-C(O)CH_2C(O)R^{\dagger}$, $-S(O)_2R^{\dagger}$, $-S(O)_2NR^{\dagger}_2$, $-C(S)NR^{\dagger}_2$, $-C(NH)NR^{\dagger}_2$, or $-N(R^{\dagger})S(O)_2R^{\dagger}$; wherein each $R^{\dagger}$ is independently hydrogen, $C_{1-6}$ aliphatic which may be substituted as defined below, unsubstituted -OPh, or an unsubstituted 5-6-membered saturated, partially unsaturated, or aryl ring having 0-4 heteroatoms independently selected from nitrogen, oxygen, or sulfur, or, notwithstanding the definition above, two independent occurrences of $R^{\dagger}$, taken together with their intervening atom(s) form an unsubstituted 3-12-membered saturated, partially unsaturated, or aryl mono- or bicyclic ring having 0-4 heteroatoms independently selected from nitrogen, oxygen, or sulfur. Suitable

substituents on the aliphatic group of $R^\dagger$ are independently halogen, $-R^\Delta$, $-(haloR^\Delta)$, -OH, $-OR^\Delta$, $-O(haloR^\Delta)$, -CN, -C(O)OH, $-C(O)OR^\Delta$, $-NH_2$, $-NHR^\Delta$, $-NR^\Delta_2$, or- $NO_2$, wherein each $R^\Delta$ is unsubstituted or where preceded by "halo" is substituted only with one or more halogens, and is independently $C_{1-4}$ aliphatic, $-CH_2Ph$, $-O(CH_2)_{0-1}$ Ph, or a 5-6-membered saturated, partially unsaturated, or aryl ring having 0-4 heteroatoms independently selected from nitrogen, oxygen, or sulfur.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticulare, subcapsular, subarachnoid, intraspinal and intrasternal injection and infusion.

The phrases "systemic administration", "administered systemically", "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

"Liposomes" as used herein refer to closed bilayer membranes containing an entrapped aqueous volume. Liposomes may also be uni-lamellar vesicles possessing a single membrane bilayer or multi-lamellar vesicles with multiple membrane bilayers, each separated from the next by an aqueous layer. The structure of the resulting membrane bilayer is such that the hydrophobic (non-polar) tails of the lipid are oriented toward the center of the bilayer while the hydrophilic (polar) heads orient towards the aqueous phase. Liposomes, as they are ordinarily used, consist of smectic mesophases, and can consist of either phospholipid or nonphospholipid smectic mesophases. Smectic mesophase is most accurately described by Small, HANDBOOK OF LIPID RESEARCH, Vol. 4, Plenum, NY, 1986, pp. 49-50. According to Small, "[w]hen a given molecule is heated, instead of melting directly into an isotropic liquid, it may instead pass through intermediate states called mesophases or liquid crystals, characterized by residual order in some directions but by lack of order in others... In general, the molecules of liquid crystals are somewhat longer than they are wide and have a polar or aromatic part somewhere along the length of the molecule. The molecular shape and the polar-polar, or aromatic, interaction permit the molecules to align in partially ordered arrays... These structures characteristically occur in molecules that possess a polar group at one end. Liquid crystals with long-range order in the direction of the long axis of the molecule are called smectic, layered, or lamellar liquid crystals... In the smectic states the molecules may be in single or double layers, normal or tilted to the plane of the layer, and with frozen or melted aliphatic chains."

The term "enriched" as used herein refers to a mixture having an increased proportion of one or more species. In some embodiments, the mixture is "enriched" following a process that increases the proportion of one or more desired species in the mixture. In some embodiments, the desired species comprise(s) greater than 10% of the mixture. In some embodiments, the desired species comprise(s) greater than 25% of the mixture. In some embodiments, the desired species comprise(s) greater than 40% of the mixture. In some embodiments, the desired species comprise(s) greater than 60% of the mixture. In some embodiments, the desired species comprise(s) greater than 75% of the mixture. In some embodiments, the desired species comprise(s) greater than 85% of the mixture. In some embodiments, the desired species comprise(s) greater than 90% of the mixture. In some embodiments, the desired species comprise(s) greater than 95% of the mixture. Such proportions can be measured any number of ways, for example, as a molar ratio, volume to volume, or weight to weight.

The term "pure" refers to compounds that are substantially free of compounds of related non-target structure or chemical precursors (when chemically synthesized). This quality may be measured or expressed as "purity." In some embodiments, a target compound has less than about 30%, 20%, 10%, 5%, 2%, 1%, 0.5%, and 0.1% of non-target structures or chemical precursors.

The term "carbohydrate" refers to a sugar or polymer of sugars. The terms "saccharide", "polysaccharide", "carbohydrate", and "oligosaccharide", may be used interchangeably. Most carbohydrates are aldehydes or ketones with many hydroxyl groups, usually one on each carbon atom of the molecule. Carbohydrates generally have the molecular formula $C_nH_{2n}O_n$. A carbohydrate may be a monosaccharide, a disaccharide, trisaccharide, oligosaccharide, or polysaccharide. The most basic carbohydrate is a monosaccharide, such as glucose, sucrose, galactose, mannose, ribose, arabinose, xylose, and fructose. Disaccharides are two joined monosaccharides. Exemplary disaccharides include sucrose, maltose, cellobiose, and lactose. Typically, an oligosaccharide includes between three and six monosaccharide units (e.g., raffinose, stachyose), and polysaccharides include six or more monosaccharide units. Exemplary polysaccharides include starch, glycogen, and cellulose. Carbohydrates may contain modified saccharide units such as 2'-deoxyribose wherein a hydroxyl group is removed, 2'-fluororibose wherein a hydroxyl group is replaced with a fluorine, or N-acetylglucosamine, a nitrogen-containing form of glucose, (e.g., 2'-fluororibose, deoxyribose, and hexose). Carbohydrates may exist in many different forms, for example, conformers, cyclic forms, acyclic forms, stereoisomers, tautomers, anomers, and isomers.

**Compounds of formula (I): Adjuvants**

In one aspect, the present application provides compounds of formula (I) or a pharmaceutically acceptable salt thereof useful as adjuvants

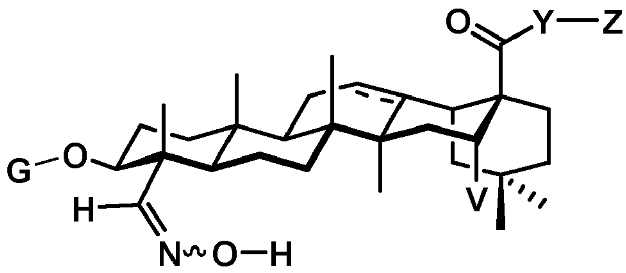

(I)

wherein the variables

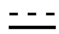

, G, V, Y and Z take the meanings as defined above in formula (I).

In some embodiments, the present application provides compounds of formula (I) or a pharmaceutically acceptable salt thereof

wherein G is hydrogen or

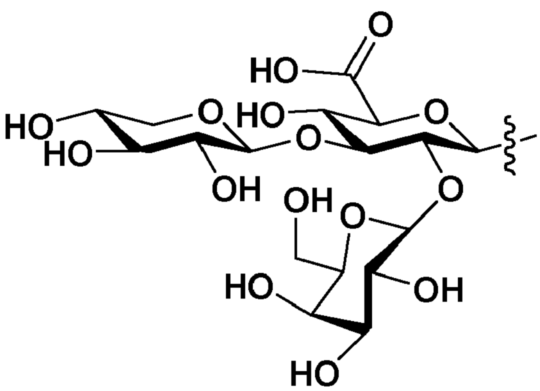

;

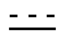

is a single or double bond;

V is H or OH;

Y is -O-;

Z is a carbohydrate domain having the structure:

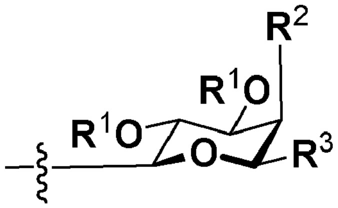

wherein

$R^1$ is independently H or

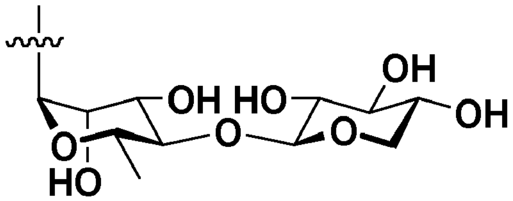

$R^2$ is $NHR^4$;

$R^3$ is $CH_2OH$; and

$R^4$ is -T-$R^z$, -C(O)-T-$R^z$, -NH-T-$R^z$, -O-T-$R^z$, -S-T-$R^z$, -C(O)NH-T-$R^z$, C(O)O-T-$R^z$, C(O)S-T-$R^z$, C(O)NH-T-O-T-$R^z$, -O-T-$R^z$, -T-O-T-$R^z$, -T-S-T-$R^z$, or

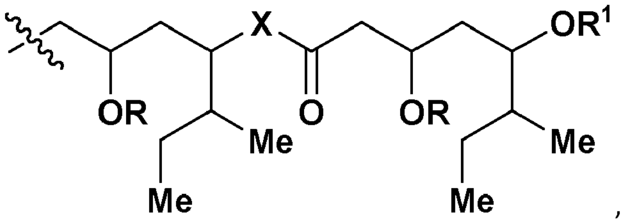

,

wherein:

X is -O-, -NR-, or T-$R^z$;
T is a covalent bond or a bivalent $C_{1-26}$ saturated or unsaturated, straight or branched, aliphatic or heteroaliphatic chain; and
$R^z$ is hydrogen, halogen, -OR, - $OR^x$, -$OR^1$, - SR, -$NR_2$, -C(O)OR, -C(O)R, -NHC(O)R, -NHC(O)OR, -NC(O)OR, or an optionally substituted group selected from acyl, arylalkyl, heteroarylalkyl, $C_{1-6}$ aliphatic, 6-10-membered aryl, 5-10-membered heteroaryl having 1-4 heteroatoms independently selected from nitrogen, oxygen, or sulfur, 4-7-membered heterocyclyl having 1-2 heteroatoms independently selected from the group consisting of nitrogen, oxygen, and sulfur;
$R^x$ is independently hydrogen or an oxygen protecting group selected from the group consisting of alkyl ethers, benzyl ethers, silyl ethers, acetals, ketals, esters, carbamates, and carbonates; and
R is independently hydrogen, an optionally substituted group selected from acyl, arylalkyl, 6-10-membered aryl, $C_{1-6}$ aliphatic, or $C_{1-6}$ heteroaliphatic having 1-2 heteroatoms independently selected from the group consisting of nitrogen, oxygen, and sulfur, or;
two R on the same nitrogen atom are taken with the nitrogen atom to form a 4-7-membered heterocyclic ring having 1-2 heteroatoms independently selected from the group consisting of nitrogen, oxygen, and sulfur.

In certain embodiments, G is hydrogen. In certain embodiments, G is a branched trisacchride of formula (VI) or a stereoisomer of formula (VI):

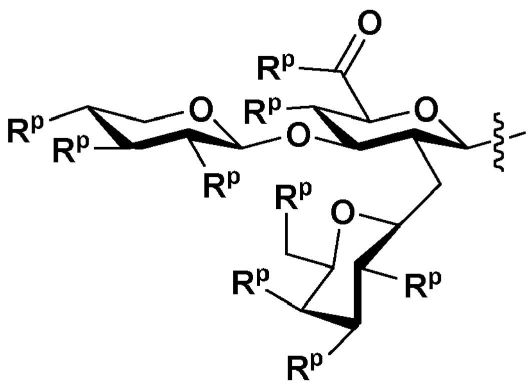

(VI)

wherein each occurrence of $R^p$ is independently hydrogen or $OR^q$;
wherein each occurrence of $R^q$ is independently hydrogen or an optionally substituted group selected from 6-10-membered aryl, benzyl, $C_{1-6}$ aliphatic, or $C_{1-6}$ heteroaliphatic having 1-2 heteroatoms independently selected from the group consisting of nitrogen, oxygen, and sulfur; or two $R^q$ are taken together to form a 5-7-membered heterocyclic ring having 1-2 heteroatoms independently selected from the group consisting of nitrogen, oxygen, and sulfur. In formula (VI), $R^q$ is preferably OH.

In certain embodiments, V is $OR^x$. In certain embodiments V is OH. In certain embodiments, V is H.

In certain embodiments, Y is -O-. In certain embodiments, Y is -NH-. In certain embodiments, Y is -NR-. In certain embodiments, Y is $CH_2$. In certain embodiments, Y is -S-.

In certain embodiments, Z is a carbohydrate domain having the structure:

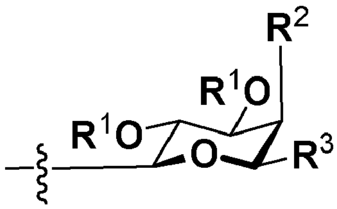

wherein

$R^1$ is independently H or

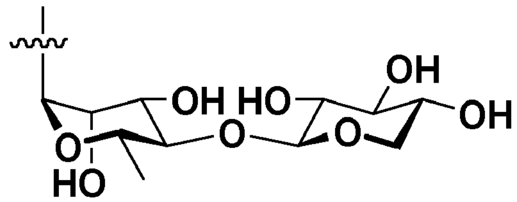

$R^2$ is $NHR^4$;

$R^3$ is $CH_2OH$; and

$R^4$ is -T-$R^z$, -C(O)-T-$R^z$, -NH-T-$R^z$, -O-T-$R^z$, -S-T-$R^z$, -C(O)NH-T-$R^z$, -C(O)O-T-$R^z$, C(O)S-T-$R^z$, C(O)NH-T-O-T-$R^z$, -O-T-$R^z$, -T-O-T-$R^z$, -T-S-T-$R^z$, or

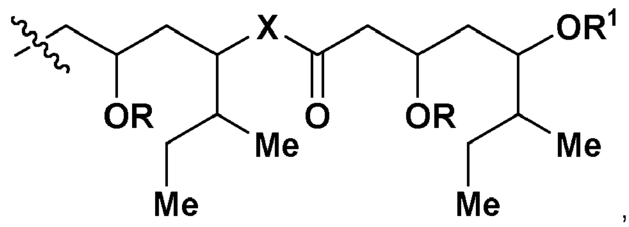

,

wherein:

X is -O-, -NR-, or T-$R^z$;

T is a covalent bond or a bivalent $C_{1-26}$ saturated or unsaturated, straight or branched, aliphatic or heteroaliphatic chain; and

$R^z$ is hydrogen, halogen, -OR, -$OR^x$, -$OR^1$, -SR, -$NR_2$, -C(O)OR, -C(O)R, -NHC(O)R, - NHC(O)OR, -NC(O)OR, or an optionally substituted group selected from acyl, arylalkyl, heteroarylalkyl, $C_{1-6}$ aliphatic, 6-10-membered aryl, 5-10-membered heteroaryl having 1-4 heteroatoms independently selected from nitrogen, oxygen, or sulfur, 4-7-membered heterocyclyl having 1-2 heteroatoms independently selected from the group consisting of nitrogen, oxygen, and sulfur;

$R^x$ is independently hydrogen or an oxygen protecting group selected from the group consisting of alkyl ethers, benzyl ethers, silyl ethers, acetals, ketals, esters, carbamates, and carbonates; and

R is independently hydrogen, an optionally substituted group selected from acyl, arylalkyl, 6-10-membered aryl, $C_{1-6}$ aliphatic, or $C_{1-6}$ heteroaliphatic having 1-2 heteroatoms independently selected from the group consisting of nitrogen, oxygen, and sulfur, or;

two R on the same nitrogen atom are taken with the nitrogen atom to form a 4-7-membered heterocyclic ring having 1-2 heteroatoms independently selected from the group consisting of nitrogen, oxygen, and sulfur.

In certain embodiments, Z is a carbohydrate domain having the structure:

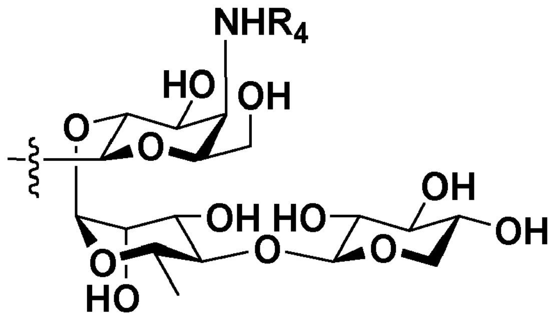

wherein $R^4$ is -C(O)-T-$R^z$;

T is a bivalent $C_{1-26}$ saturated or unsaturated, straight or branched, aliphatic or heteroaliphatic chain, preferably a bivalent $C_{1-15}$ saturated or unsaturated, straight or branched, aliphatic or heteroaliphatic chain, more preferably a bivalent $C_{1-15}$ (e.g. $C_1$, $C_2$, $C_3$, $C_4$, $C_5$, $C_6$, $C_7$, $C_8$, $C_9$, $C_{10}$, $C_{11}$, $C_{12}$, $C_{13}$, $C_{14}$, or $C_{15}$) saturated straight aliphatic chain;

$R^z$ is hydrogen, halogen, -OR, -$OR^x$, -SR, -$NR_2$, -C(O)OR, -C(O)R, -NHC(O)R, - NHC(O)OR, -NC(O)OR, or an optionally substituted group selected from acyl, arylalkyl, heteroarylalkyl, $C_{1-6}$ aliphatic, 6-10-membered aryl, 5-10-membered heteroaryl having 1-4 heteroatoms independently selected from nitrogen, oxygen, or sulfur, 4-7-membered heterocyclyl having 1-2 heteroatoms independently selected from the group consisting of nitrogen, oxygen, and sulfur;

$R^x$ is independently hydrogen or an oxygen protecting group selected from the group consisting of alkyl ethers, benzyl ethers, silyl ethers, acetals, ketals, esters, carbamates, and carbonates; and

R is independently hydrogen, an optionally substituted group selected from acyl, arylalkyl, 6-10-membered aryl, $C_{1-6}$ aliphatic, or $C_{1-6}$ heteroaliphatic having 1-2 heteroatoms independently selected from the group consisting of nitrogen, oxygen, and sulfur, or;

two R on the same nitrogen atom are taken with the nitrogen atom to form a 4-7-membered heterocyclic ring having 1-2 heteroatoms independently selected from the group consisting of nitrogen, oxygen, and sulfur.

In certain embodiments, $R^z$ is -OR, -OR$^x$, -SR, -NR$_2$, -C(O)OR, -C(O)R, -NHC(O)R, - NHC(O)OR, or -NC(O)OR; wherein

$R^x$ is independently hydrogen or an oxygen protecting group selected from the group consisting of alkyl ethers, benzyl ethers, silyl ethers, acetals, ketals, esters, carbamates, and carbonates; and

R is independently hydrogen, an optionally substituted group selected from acyl, arylalkyl, 6-10-membered aryl, $C_{1-6}$ aliphatic, or $C_{1-6}$ heteroaliphatic having 1-2 heteroatoms independently selected from the group consisting of nitrogen, oxygen, and sulfur, or;

two R on the same nitrogen atom are taken with the nitrogen atom to form a 4-7-membered heterocyclic ring having 1-2 heteroatoms independently selected from the group consisting of nitrogen, oxygen, and sulfur.

In certain embodiments, $R^z$ is -$NH_2$ or -C(O)OH.
In certain embodiments,

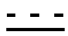

is a double bond, G is H, V is OH, Y is O and Z is a carbohydrate domain having the structure:

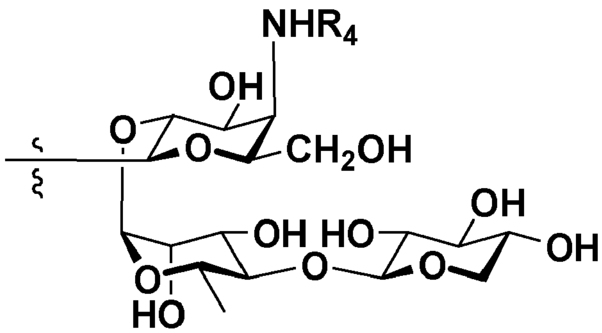

In certain embodiments, $R^4$ is selected from:

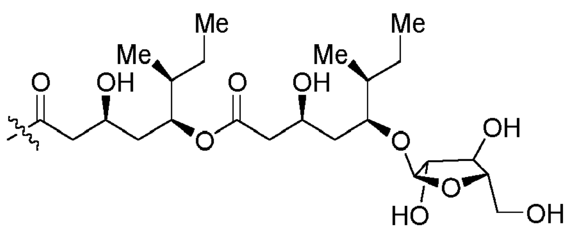

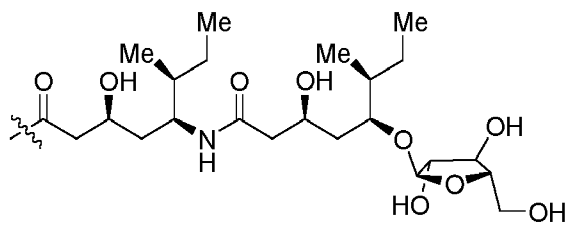

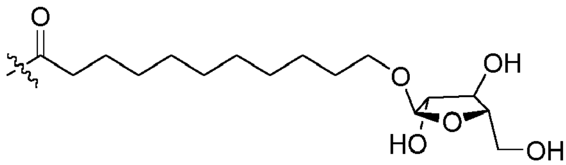

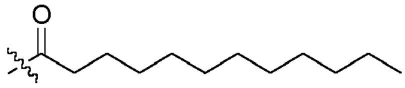

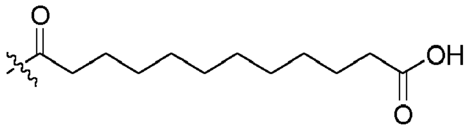

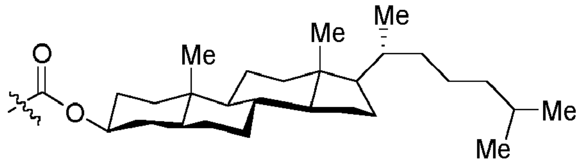

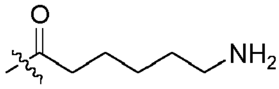

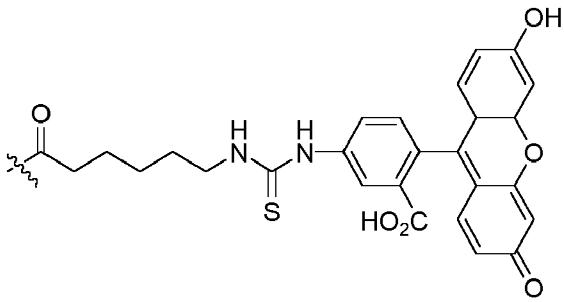

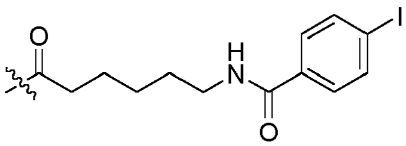

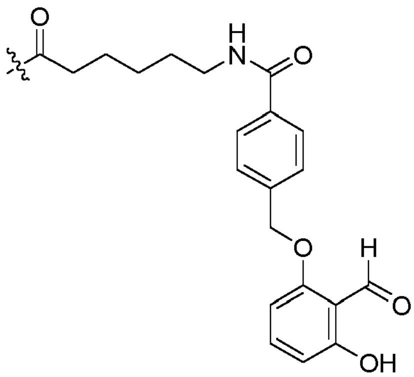

In some embodiments, $R^1$ is $R^X$. In other embodiments, $R^1$ a carbohydrate domain having the structure:

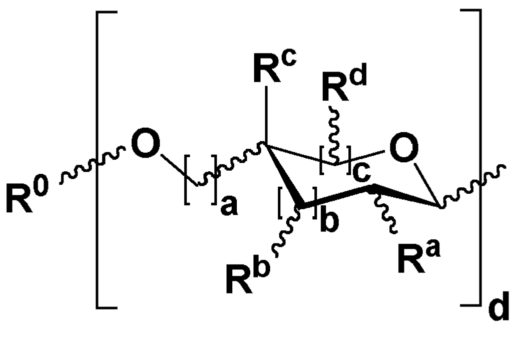

In some aspects, each occurrence of a, b, and c is independently 0, 1, or 2. In some embodiments, d is an integer from 1-5. In some embodiments, each d bracketed structure may be the same. In some embodiments, each d bracketed structure may be different. In some embodiments, the d bracketed structure represents a furanose or a pyranose moiety. In some embodiments, and the sum of b and c is 1 or 2.

In some embodiments, $R^0$ is hydrogen. In some embodiments, $R^0$ is an oxygen protecting group selected from the group. In some embodiments, $R^0$ is an alkyl ether. In some embodiments, $R^0$ is a benzyl ether. In some embodiments,

$R^0$ is a silyl ether. In some embodiments, $R^0$ is an acetal. In some embodiments, $R^0$ is ketal. In some embodiments, $R^0$ is an ester. In some embodiments, $R^0$ is a carbamate. In some embodiments, $R^0$ is a carbonate. In some embodiments, $R^0$ is an optionally substituted moiety. In some embodiments, $R^0$ is an acyl. In some embodiments, $R^0$ is a $C_{1-10}$ aliphatic. In some embodiments, $R^0$ is a $C_{1-6}$ heteroaliphatic. In some embodiments, $R^0$ is a 6-10-membered aryl. In some embodiments, $R^0$ is arylalkyl. In some embodiments, $R^0$ is a 5-10 membered heteroaryl having 1-4 heteroatoms independently selected from nitrogen, oxygen, or sulfur. In some embodiments, $R^0$ is a 4-7 membered heterocyclyl having 1-2 heteroatoms independently selected from the group consisting of nitrogen, oxygen, and sulfur.

In some embodiments, $R^a$ is hydrogen. In some embodiments, $R^a$ is a halogen. In some embodiments, $R^a$ is OH. In some embodiments, $R^a$ is OR. In some embodiments, $R^a$ is $OR^x$. In some embodiments, $R^a$ is $NR_2$. In some embodiments, $R^a$ is NHCOR. In some embodiments, $R^a$ an acyl. In some embodiments, $R^a$ is $C_{1-10}$ aliphatic. In some embodiments, $R^a$ is $C_{1-6}$ heteroaliphatic. In some embodiments, $R^a$ is 6-10-membered aryl. In some embodiments, $R^a$ is arylalkyl. In some embodiments, $R^a$ is 5-10-membered heteroaryl having 1-4 heteroatoms independently selected from nitrogen, oxygen, sulfur. In some embodiments, $R^a$ is 4-7-membered heterocyclyl having 1-2 heteroatoms independently selected from the group consisting of nitrogen, oxygen, and sulfur.

In some embodiments, $R^b$ is hydrogen. In some embodiments, $R^b$ is a halogen. In some embodiments, $R^b$ is OH. In some embodiments, $R^b$ is OR. In some embodiments, $R^b$ is $OR^x$. In some embodiments, $R^b$ is $NR_2$. In some embodiments, $R^b$ is NHCOR. In some embodiments, $R^b$ an acyl. In some embodiments, $R^b$ is $C_{1-10}$ aliphatic. In some embodiments, $R^b$ is $C_{1-6}$ heteroaliphatic. In some embodiments, $R^b$ is 6-10-membered aryl. In some embodiments, $R^b$ is arylalkyl. In some embodiments, $R^b$ is 5-10-membered heteroaryl having 1-4 heteroatoms independently selected from nitrogen, oxygen, sulfur. In some embodiments, $R^b$ is 4-7-membered heterocyclyl having 1-2 heteroatoms independently selected from the group consisting of nitrogen, oxygen, and sulfur.

In some embodiments, $R^c$ is hydrogen. In some embodiments, $R^c$ is a halogen. In some embodiments, $R^c$ is OH. In some embodiments, $R^c$ is OR. In some embodiments, $R^c$ is $OR^x$. In some embodiments, $R^c$ is $NR_2$. In some embodiments, $R^c$ is NHCOR. In some embodiments, $R^c$ an acyl. In some embodiments, $R^c$ is $C_{1-10}$ aliphatic. In some embodiments, $R^c$ is $C_{1-6}$ heteroaliphatic. In some embodiments, $R^c$ is 6-10-membered aryl. In some embodiments, $R^c$ is arylalkyl. In some embodiments, $R^c$ is 5-10-membered heteroaryl having 1-4 heteroatoms independently selected from nitrogen, oxygen, sulfur. In some embodiments, $R^c$ is 4-7-membered heterocyclyl having 1-2 heteroatoms independently selected from the group consisting of nitrogen, oxygen, and sulfur.

In some embodiments, $R^d$ is hydrogen. In some embodiments, $R^d$ is a halogen. In some embodiments, $R^d$ is OH. In some embodiments, $R^d$ is OR. In some embodiments, $R^d$ is $OR^x$. In some embodiments, $R^d$ is $NR_2$. In some embodiments, $R^d$ is NHCOR. In some embodiments, $R^d$an acyl. In some embodiments, $R^d$ is $C_{1-10}$ aliphatic. In some embodiments, $R^d$ is $C_{1-6}$ heteroaliphatic. In some embodiments, $R^d$ is 6-10-membered aryl. In some embodiments, $R^d$ is arylalkyl. In some embodiments, $R^d$ is 5-10-membered heteroaryl having 1-4 heteroatoms independently selected from nitrogen, oxygen, sulfur. In some embodiments, $R^d$ is 4-7-membered heterocyclyl having 1-2 heteroatoms independently selected from the group consisting of nitrogen, oxygen, and sulfur.

In some embodiments, $R^2$ is hydrogen. In some embodiments, $R^2$ is a halogen. In some embodiments, $R^2$ is OH. In some embodiments, $R^2$ is OR. In some embodiments, $R^2$ is $OC(O)R^4$. In some embodiments, $R^2$ is $OC(O)OR^4$. In some embodiments, $R^2$ is $OC(O)NHR^4$. In some embodiments, $R^2$ is $OC(O)NRR^4$. In some embodiments, $R^2$ is $OC(O)SR^4$. In some embodiments, $R^2$ is $NHC(O)R^4$. In some embodiments, $R^2$ is $NRC(O)R^4$. In some embodiments, $R^2$ is $NHC(O)OR^4$. In some embodiments, $R^2$ is $NHC(O)NHR^4$. In some embodiments, $R^2$ is $NHC(O)NRR^4$. In some embodiments, $R^2$ is $NHR^4$. In some embodiments, $R^2$ is $N(R^4)_2$. In some embodiments, $R^2$ is $NHR^4$. In some embodiments, $R^2$ is $NRR^4$. In some embodiments, $R^2$ is $N_3$. In some embodiments, $R^2$ is $C_{1-10}$ aliphatic. In some embodiments, $R^2$ is $C_{1-6}$ heteroaliphatic. In some embodiments, $R^2$ is 6-10-membered aryl. In some embodiments, $R^2$ is arylalkyl. In some embodiments, $R^2$ is 5-10-membered heteroaryl having 1-4 heteroatoms independently selected from the group consisting of nitrogen, oxygen, and sulfur. In some embodiments, $R^2$ is 4-7-membered heterocyclyl having 1-2 heteroatoms independently selected from the group consisting of nitrogen, oxygen, and sulfur.

In some embodiments, $R^3$ is hydrogen. In some embodiments, $R^3$ is a halogen. In some embodiments, $R^3$ is $CH_2OR^1$. In some embodiments, $R^3$ is an acyl. In some embodiments, $R^3$ is $C_{1-10}$ aliphatic. In some embodiments, $R^3$ is $C_{1-6}$ heteroaliphatic. In some embodiments, $R^3$ is 6-10-membered aryl. In some embodiments, $R^3$ is arylalkyl. In some embodiments, $R^3$ is 5-10-membered heteroaryl having 1-4 heteroatoms independently selected from the group consisting of nitrogen, oxygen, and sulfur. In some embodiments, $R^3$ is 4-7-membered heterocyclyl having 1-2 heteroatoms independently selected from the group consisting of nitrogen, oxygen, and sulfur.

In some embodiments, $R^4$ is $-T-R^z$. In some embodiments, $R^4$ is $-C(O)-T-R^z$. In some embodiments, $R^4$ is $-NH-T-R^z$. In some embodiments, $R^4$ is $-O-T-R^z$. In some embodiments, $R^4$ is $-S-T-R^z$. In some embodiments, $R^4$ is $-C(O)NH-T-R^z$. In some embodiments, $R^4$ is $- C(O)O-T-R^z$. In some embodiments, $R^4$ is $-C(O)S-T-R^z$. In some embodiments, $R^4$ is $-C(O)NH-T-O-T-R^z$. In some embodiments, $R^4$ is $-O-T-R^z$. In some embodiments, $R^4$ is $-T-O-T-R^z$. In some embodiments, $R^4$ is $-T-S-T-R^z$. In some embodiments, $R^4$ is

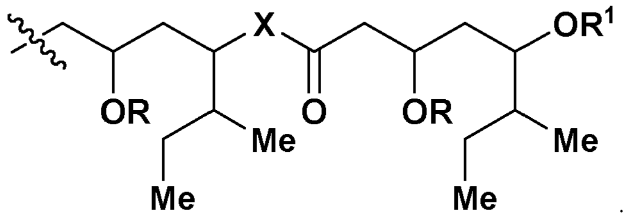

In some embodiments, X is -O-. In some embodiments, X is -NR-. In some embodiments, X is T-$R^z$.

In some embodiments, T is a covalent bond or a bivalent $C_{1-26}$ saturated or unsaturated, straight or branched, aliphatic or heteroaliphatic chain.

In some embodiments, $R^z$ is hydrogen. In some embodiments, $R^z$ is a halogen. In some embodiments, $R^z$ is -OR. In some embodiments, $R^z$ is -$OR^x$. In some embodiments, $R^z$ is -$OR^1$. In some embodiments, $R^z$ is -SR. In some embodiments, $R^z$ is -$NR_2$. In some embodiments, $R^z$ is -C(O)OR. In some embodiments, $R^z$ is -C(O)R. In some embodiments, $R^z$ is -NHC(O)R. In some embodiments, $R^z$ is -NHC(O)OR. In some embodiments, $R^z$ is -NC(O)OR. In some embodiments, $R^z$ is an acyl. In some embodiments, $R^z$ is arylalkyl. In some embodiments, $R^z$ is heteroarylalkyl. In some embodiments, $R^z$ is $C_{1-6}$ aliphatic. In some embodiments, $R^z$ is 6-10-membered aryl. In some embodiments, $R^z$ is 5-10-membered heteroaryl having 1-4 heteroatoms independently selected from nitrogen, oxygen, or sulfur. In some embodiments, $R^z$ is 4-7-membered heterocyclyl having 1-2 heteroatoms independently selected from the group consisting of nitrogen, oxygen, and sulfur.

In some embodiments, $R^x$ is hydrogen. In some embodiments, $R^x$ is an oxygen protecting group. In some embodiments, $R^x$ is an alkyl ether. In some embodiments, $R^x$ is a benzyl ether. In some embodiments, $R^x$ is silyl ether. In some embodiments, $R^x$ is an acetal. In some embodiments, $R^x$ is ketal. In some embodiments, $R^x$ is ester. In some embodiments, $R^x$ is carbamate. In some embodiments, $R^x$ is carbonate.

In some embodiments, R is hydrogen. In some embodiments, R is an acyl. In some embodiments, R is arylalkyl. In some embodiments, R is 6-10-membered aryl. In some embodiments, R is $C_{1-6}$ aliphatic. In some embodiments, R is $C_{1-6}$ heteroaliphatic having 1-2 heteroatoms independently selected from the group consisting of nitrogen, oxygen, and sulfur. In some embodiments, two R on the same nitrogen atom are taken with the nitrogen atom to form a 4-7-membered heterocyclic ring having 1-2 heteroatoms independently selected from the group consisting of nitrogen, oxygen, and sulfur.

Exemplary compounds of formula (I) are set forth below:

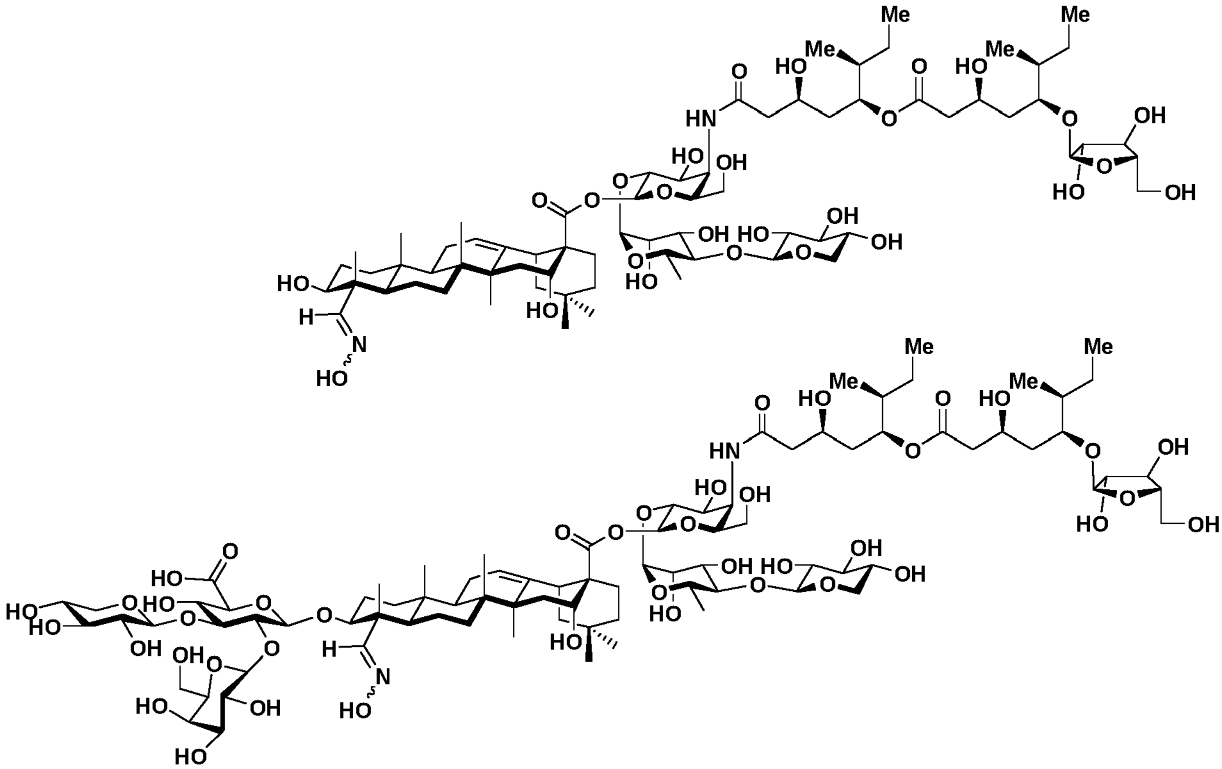

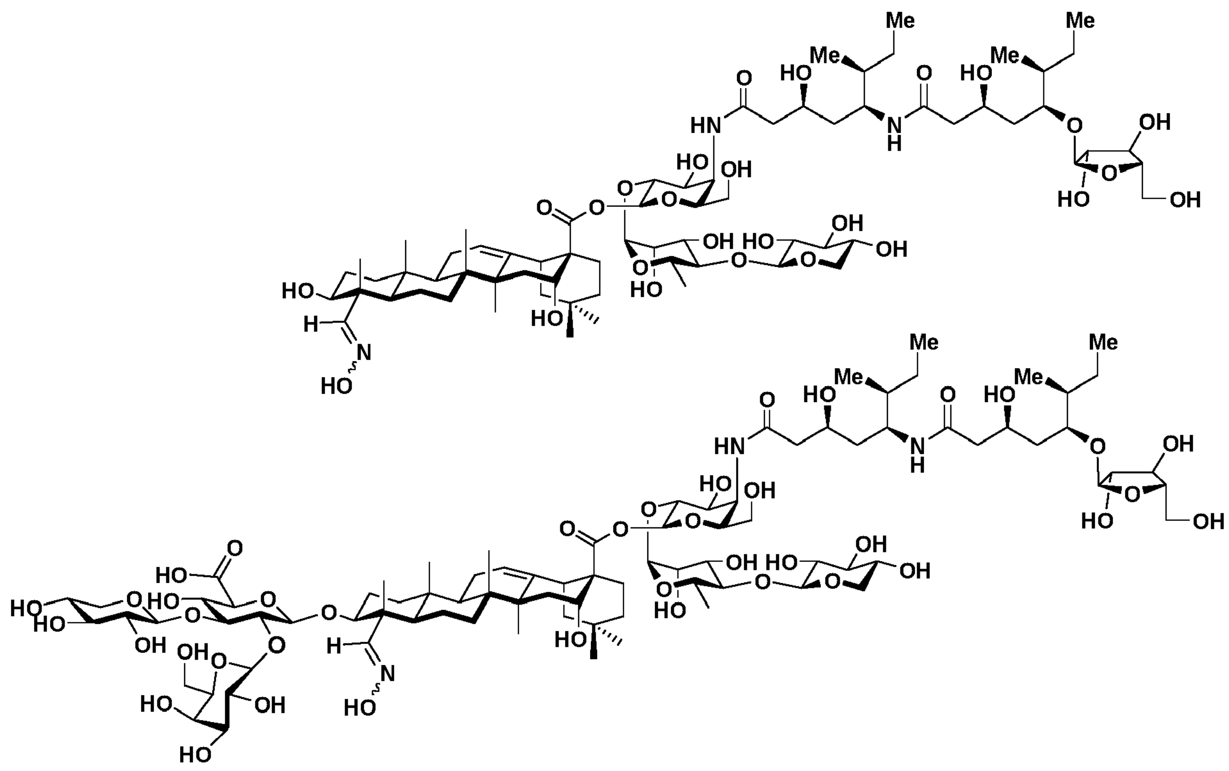

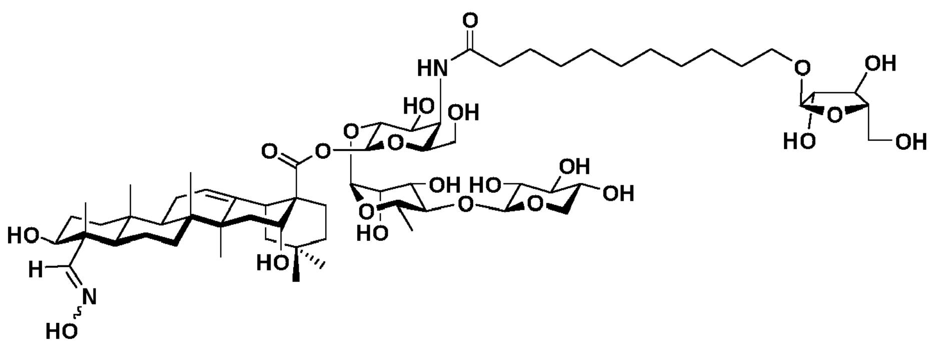

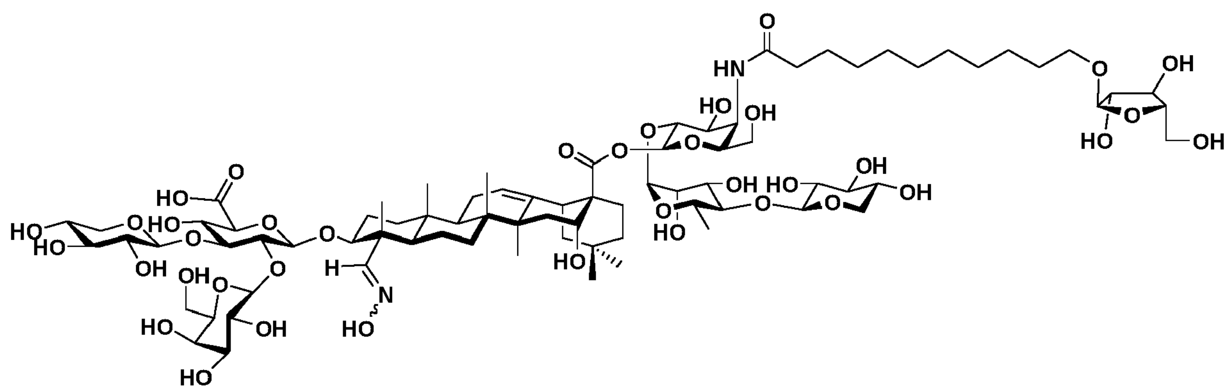

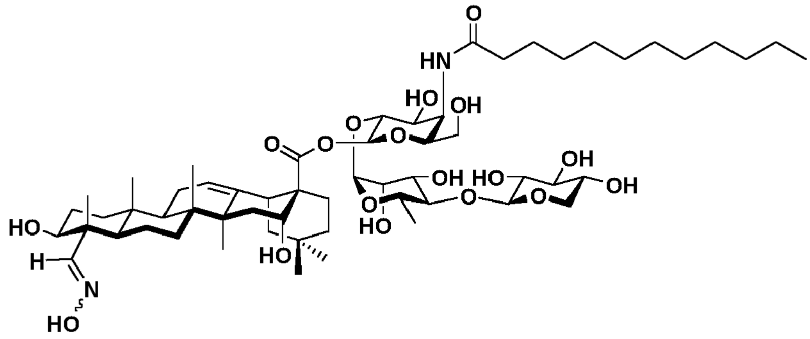

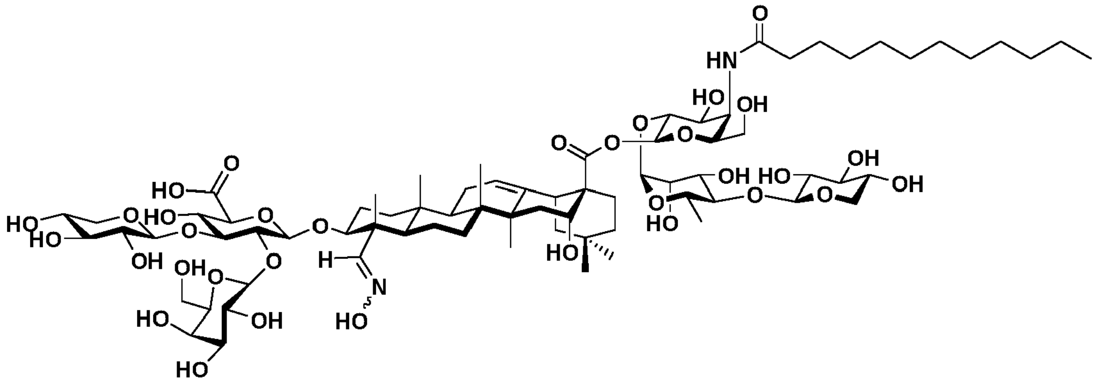

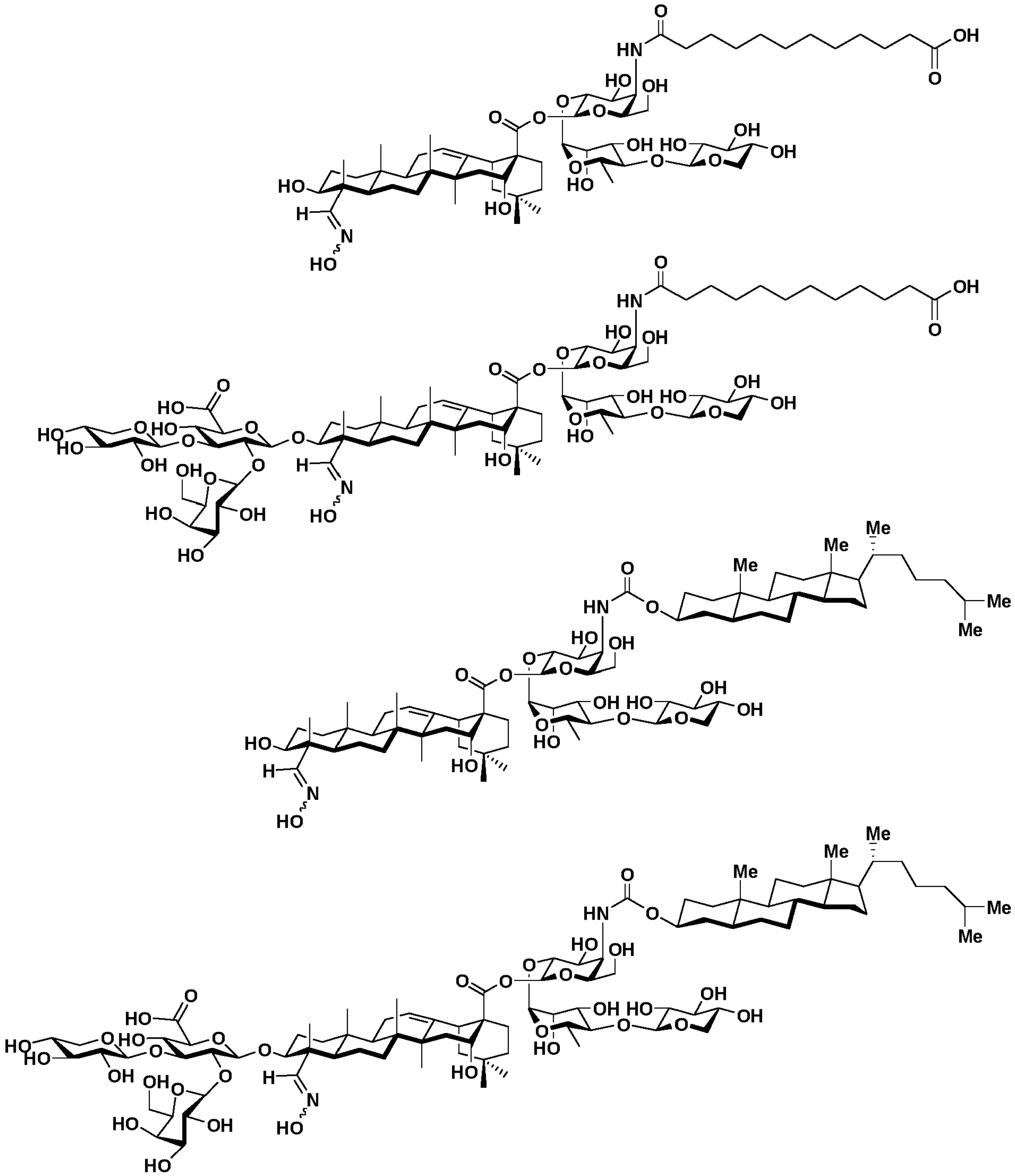

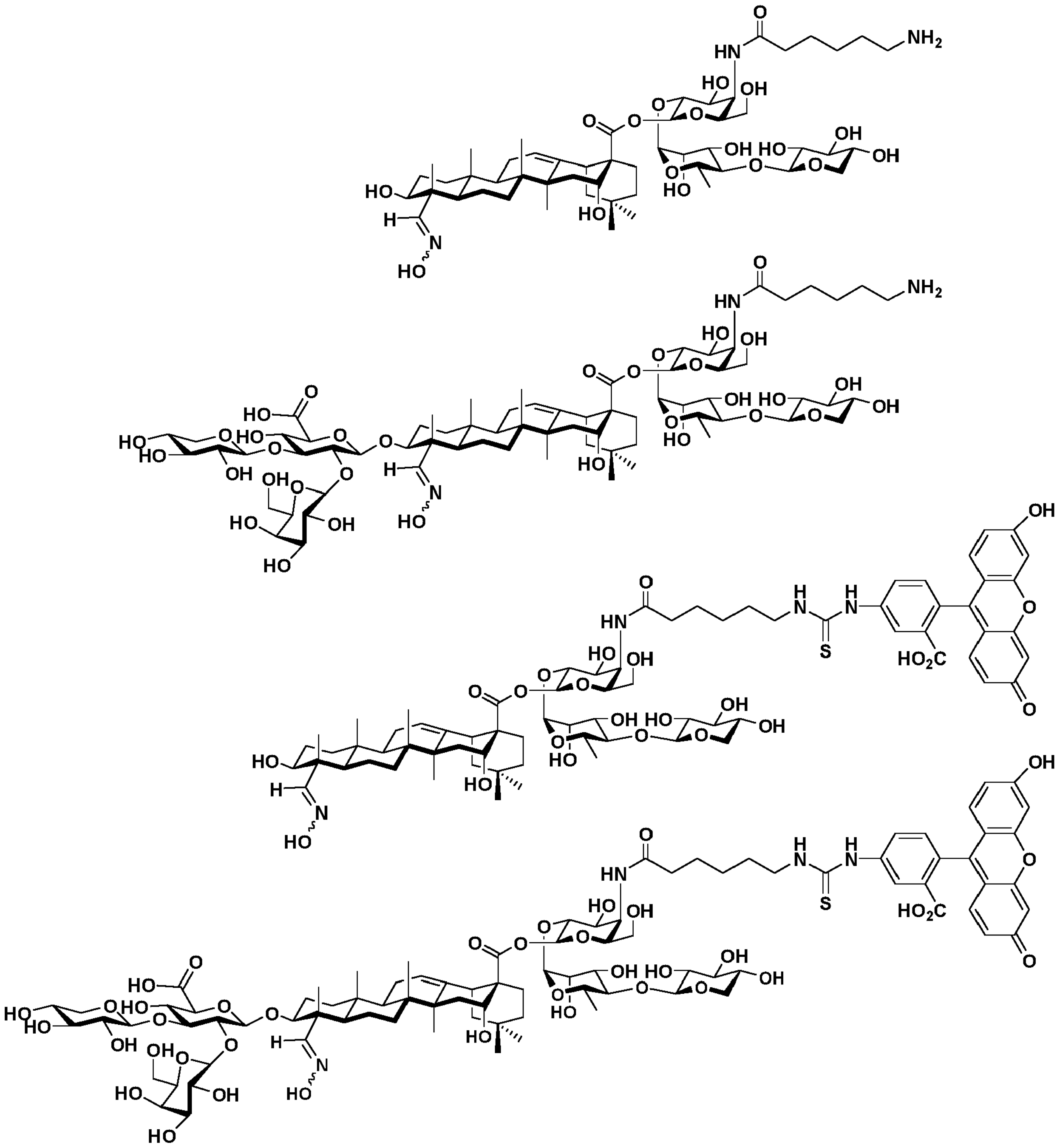

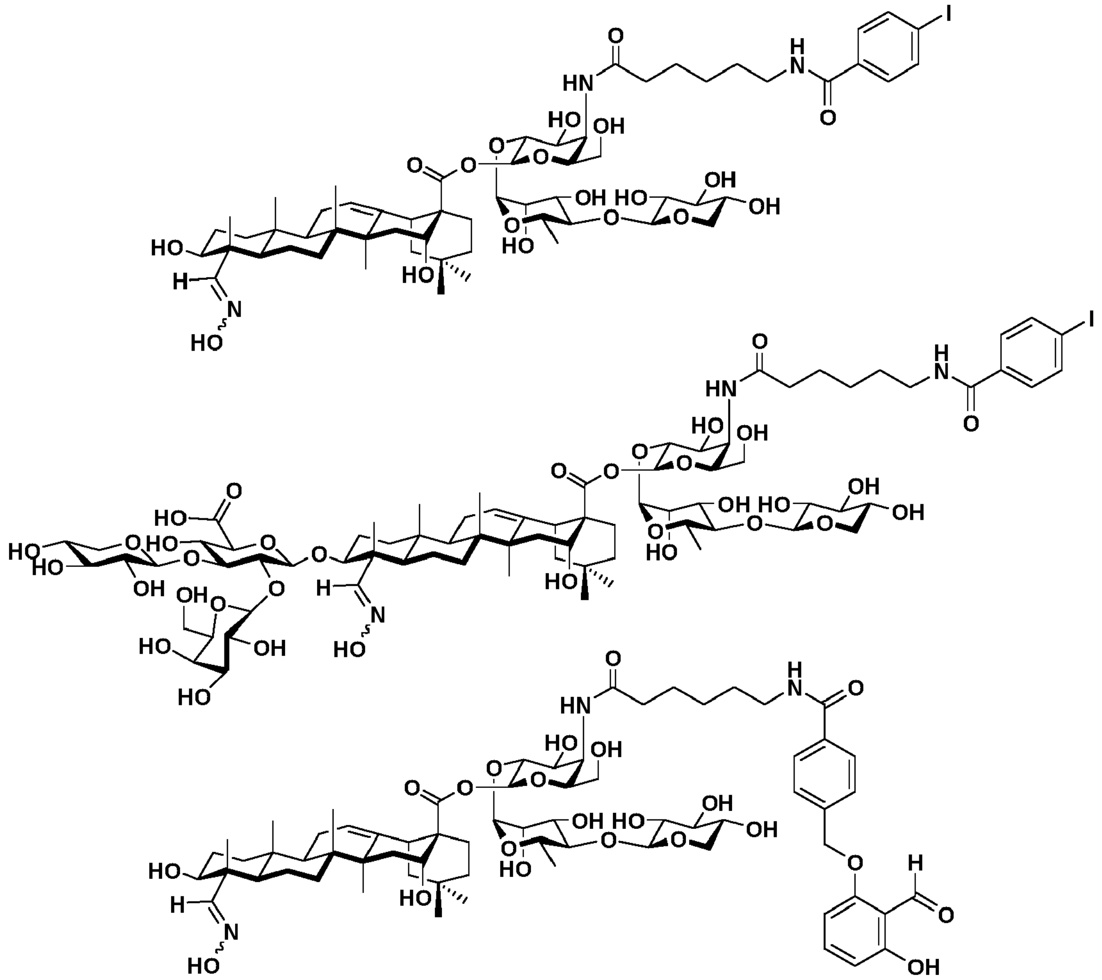

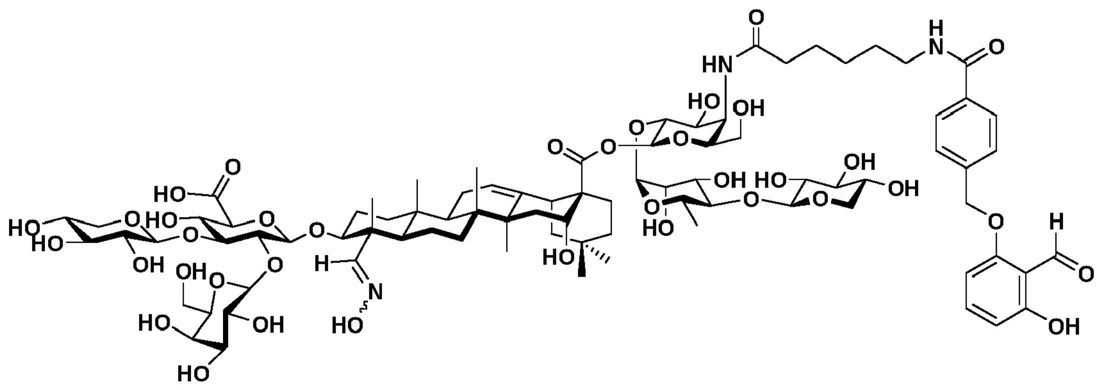

## Compounds of formula (II): self-adjuvanting vaccines

While traditional vaccines are formulated into mixtures of an antigen plus an adjuvant, vaccines in which the two moieties are contained within a single molecule are dubbed self-adjuvanting vaccines.

The invention provides compounds of general formula (II) or a pharmaceutically acceptable salt thereof useful as self-adjuvanting vaccines

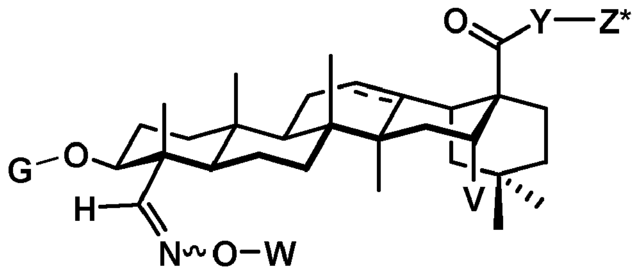

(II)

wherein

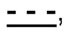,

, G, V and Y take the meanings and particular embodiments as in formula (I);

W is H or a moiety comprising either i) at least one T cell epitope selected from a helper T cell epitope or a CD8 epitope, ii) at least one B cell epitope or iii) at least one T cell epitope selected from a helper T cell epitope or a CD8 epitope, and at least one B cell epitope;

Z* represents that Z, as defined in formula (I), is optionally conjugated with a moiety comprising either i) at least one T cell epitope selected from a helper T cell epitope or a CD8 epitope, ii) at least one B cell epitope or iii) at least one T cell epitope selected from a helper T cell epitope or a CD8 epitope, and at least one B cell epitope;

with the proviso that the compound of formula (II) must comprise at least one T cell epitope and at least one B cell epitope.

Thus, the T cell epitope and the B cell epitope may be incorporated within the compound of formula (II) in different arrangements. For instance, the compounds of formula (II) may be provided at the C4-position of the triterpene domain with a moiety comprising at least one T cell epitope and at least one B cell epitope. In an alternative embodiment, the compounds of formula (II) may be provided through the chain of the carbohydrate domain with a moiety comprising at least one T cell epitope and at least one B cell epitope. In an alternative embodiment, the compounds of formula (II) may be provided at the C4-position of the triterpene domain with a moiety comprising at least one B cell epitope and through the chain of the carbohydrate domain with a moiety comprising at least one T cell epitope. In an alternative and preferred embodiment, the compounds of formula (II) may be provided at the C4-position of the triterpene domain with a moiety comprising at least one T cell epitope and through the chain of the carbohydrate domain with a moiety comprising at least one B cell epitope. Therefore, preferably W in the compounds of formula (II) is a moiety comprising at least one T cell epitope selected from a helper T cell epitope or a CD8 epitope and Z* represents that Z, as defined in formula (I), is conjugated with a moiety comprising at least one B cell epitope.

As used herein, the term "B cell epitope" refers to any antigen portion or region that is recognized by secreted antibodies or B-cell receptors and is able to trigger an immune response in a B cell. Methods and techniques to determine if a peptide/molecule is or contains a B cell epitope are well known to the skilled person in the art and described in the literature (Ahmad, T. A., et al. Trials in Vaccinology 2016, Volume 5, Pages 71-83).

As used herein, the T cell epitope is selected from a helper T cell epitope or a CD8 epitope.

The term "Helper T cell epitope" as used herein refers to peptides derived from antigens and recognized by the Helper T-cell receptor (TCR) when bound to class II Major Histocompatibility Complex (MHC-II) molecules displayed on the cell surface of Antigen Presenting Cells (APCs), which lead to the activation of the Helper T cells. Said peptides are the specific amino acid sequence of the antigen which is recognized by the TCR. Said peptides can also be used as "helper epitopes", epitopes which are known to be recognized by and activate Helper T cells, wherein said helper epitopes are fused to, or used in proximity of (such as co-expressed in the APC membrane), antigens of interest, more preferably B cell epitopes, being newly presented to Helper T cells in order to improve and enhance the immunological response of the Helper T cells. Methods to identify peptides which activate a response from helper T cells are well known in the art and examples of such methods can be found in the literature (Wang R. F. Methods. 2003, 29(3), 227-35; Singh R. et al. Immunology 2014, 141(4), 514-30).

The term "CD8 cell epitope" or "CD8 T cell epitope" as used herein refers to peptides derived from antigens recognized by CD8 T cell receptors when said antigens are bound to class I (MHC I) or class II (MHC II) Major Histocompatibility Complex (MHC) molecules on the surface of antigen presenting cells (APCs). When CD8 TCRs recognize the mentioned peptides, the cells get activated becoming cytotoxic T lymphocytes (CTLs). Methods to identify peptides which activate a response from CD8 T cells are well known in the art and examples of such methods can be found in the literature Reche, et al., 2004, Immunogenetics, vol. 56, 6, 405-419; Dönnes and Kohlbacher, 2005, Protein Sci., vol. 14, 8, 2132-2140; Doytchinova, et al., 2006, BMC Bioinformatics, vol. 7, 1, 131).

In some embodiments, the B cell epitope or the CD8 T cell epitope is selected from the group consisting of peptides, glycopeptides and carbohydrates capable of inducing an immune response against a neurodegenerative disease (e.g. the immunogenic region of a neurodegeneration-associated antigen), an infectious disease (e.g. the immunogenic region of a bacterial-, viral-, or protozoal-associated antigen) or a cancer cell (e.g. the immunogenic region of a cancer-associated antigen, also known as tumor-associated antigen or TACA).

In some embodiments, the B cell epitope or the CD8 T cell epitope is or is found within the immunogenic region of a cancer-associated antigen selected from the group consisting of:

- MUC1 peptide, TnMUC1 glycopeptide, Gb3 carbohydrate and Tn carbohydrate antigens such as Tn(Thr) antigen;
- Glycoproteins such as PSA; Mucins such as MUC1, MUC2, MUC4, MUC5AC, MUC6, MUC16; Mucin-derived carbohydrate antigens such as Tn, TF, STn; gangliosides such as GM2, GM3, GD2, GD3; globosides such as Gb4, Gb5, Globo-H;
- 5T4, 8H9, av beta 6 integrin, alphafetoprotein (AFP), B7-H6, CA-125, carbonic anhydrase 9 (CA9), CD19, CD20, CD22, CD30, CD33, CD38, CD44, CD44v6, CD44v7/8, CD52, CD123, CD171, carcionoembryonic antigen (CEA), EGFRvlll, epithelial glycoprotein-2 (EGP-2), epithelial glycoprotein-40 (EGP-40), ErbB1/EGFR, ErbB2/HER2/neu/EGFR2, ErbB3, ErbB4, epithelial tumor antigen (ETA), FBP, fetal acetylcholine receptor (AchR), folate receptor-a, G250/CAIX, ganglioside 2 (GD2), ganglioside 3 (GD3), HLA-A1, HLA-A2, high molecular weight melanoma-associated antigen (HMW-MAA), IL-13 receptor a2, KDR, k-light chain, Lewis Y (LeY), L1 cell adhesion molecule, melanoma-associated antigen (MAGE-A1), mesothelin, Murine CMV infected cells, mucin-1 (MUC1), mucin-16 (MUC16), natural killer group 2 member D (NKG2D) ligands, nerve cell adhesion molecule (NCAM), NY-ESO-1, Oncofetal antigen (h5T4), prostate stem cell antigen (PSCA), prostate-specific membrane antigen (PSMA), receptor-tyrosine kinase-like orphan receptor 1 (ROR1), TAA targeted by mAb IgE, tumor-associated glycoprotein-72 (TAG-72), tyrosinase, and vascular endothelial growth factor (VEGF) receptors;
- human Her2/neu, Her1/EGF receptor (EGFR), Her3, A33 antigen, B7H3, CD5, CD19, CD20, CD22, CD23 (IgE Receptor), C242 antigen, 5T4, IL-6, IL-13, vascular endothelial growth factor VEGF (e.g., VEGF-A) VEGFR-1, VEGFR-2, CD30, CD33, CD37, CD40, CD44, CD51, CD52, CD56, CD74, CD80, CD152, CD200, CD221, CCR4, HLA-DR, CTLA-4, NPC-1C, tenascin, vimentin, insulin-like growth factor 1 receptor (IGF-1R), alpha-fetoprotein, insulin-like growth factor 1 (IGF-1), carbonic anhydrase 9 (CA-IX), carcinoembryonic antigen (CEA), integrin av beta 3, integrin a5 beta 1, folate receptor 1, transmembrane glycoprotein NMB, fibroblast activation protein alpha (FAP), glycoprotein 75, TAG-72, MUC1, MUC16 (or CA-125), phosphatidylserine, prostate-specific membrane antigen (PMSA), NR-LU-13 antigen, TRAIL-R1, tumor necrosis factor receptor superfamily member 10b (TNFRSF10B or TRAIL-R2), SLAM family member 7 (SLAMF7), EGP40 pancarcinoma antigen, B-cell activating factor (BAFF), platelet-derived growth factor receptor, glycoprotein EpCAM (17-1A), Programmed Death-1, protein disulfide isomerase (PDI), Phosphatase of Regenerating Liver 3 (PRL-3), prostatic acid phosphatase, Lewis-Y antigen, GD2 (a disialoganglioside expressed on tumors of neuroectodermal origin), glypican-3 (GPC3), or mesothelin;
- associated antigens belonging to unique gene products of mutated or recombined cellular genes, in particular cyclin-dependent kinases, p15lnk4b, p53, AFP, B-catenin, caspase 8, p53, Bcr-abl fusion product, MUM-1 MUM-2, MUM-3, ELF2M, HSP70-2M, HST-2, KIAA0205, RAGE, myosin/m, 707-AP, CDC27/m, ETV6/AML, TEL/Amll, Dekcain, LDLR/FUT, Pm1-RARa, TEL/AMLI; Cancer-testis (CT) antigens, members of the MAGE-family, BAGE, DAM-6, DAM-10, members of the GAGE-family, NY-ESO-1, NA-88A, CAG-3, RCC-associated antigen G250, Tumor virus antigens, in particular human papilloma virus (HPV) -derived E6 E7 oncoproteins, Epstein Barr virus EBNA2-6, LMP-1, LMP-2; gp77, gp100, MART-1/Melan-A, p53, tyrosinase, tyrosinase-related protein 1 and 2, PSA, PSM, MC1R; ART4, CAMEL, CEA, CypB, epithelial cell adhesion molecule (EpCAM) HER2/neu, HER-3, hTERT, hTRT, ICE, Muc1, Muc2, PRAME RU1, RU2, SART-1, SART-2, SART-3, and WT1, and fragments and derivatives thereof;
- CD19; CD123; CD22; CD30; CD171; CS-1 (also referred to as CD2 subset 1, CRACC, SLAMF7, CD319, and 19A24); C-type lectin-like molecule-1 (CLL-1 or CLECLI); CD33; epidermal growth factor receptor variant III (EGFRvlll); ganglioside G2 (GD2); ganglioside GD3 (αNeuSAc(2-8)αNeuSAc(2-3)βDGaip(1-4)bDGlcp(1-1)Cer); ganglioside GM3 (αNeuSAc(2-3)βDGalp(1-4)βDGlcp(1-1)Cer); GM-CSF receptor; TNF receptor superfamily member 17 (TNFRSF17, BCMA); B-lymphocyte cell adhesion molecule; Tn antigen ((Tn Ag) or (GalNAc-α-Ser/Thr)); prostate-specific membrane antigen (PSMA); Receptor tyrosine kinase-like orphan receptor 1 (RORI); Tumor-associated glycoprotein 72 (TAG72); CD38; CD44v6; Carcinoembryonic antigen (CEA); Epithelial cell adhesion molecule (EPCAM); B7H3 (CD276); KIT (CD117); Interleukin-13 receptor subunit alpha-2 (IL-13Ra2 or CD213A2); Mesothelin; Interleukin 11 receptor alpha (IL-11Ra); prostate stem cell antigen (PSCA); Protease Serine 21 (Testisin or PRSS21); vascular endothelial growth factor receptor 2 (VEGFR2); HLA class I antigen A-2 alpha; HLA antigen; Lewis(Y)antigen; CD24; Platelet-derived growth factor receptor beta (PDGFR-beta); Stage-specific embryonic antigen-4 (SSEA-4); CD20; delta like 3 (DLL3); Folate receptor alpha; Folate receptor beta, GDNF alpha 4 receptor, Receptor tyrosine-protein kinase, ERBB2 (Her2/neu); Mucin 1, cell surface associated (MUC1); APRIL receptor; ADP ribosyl cyclase-1; Ephb4 tyrosine kinase receptor, DCAMKL1 serine threonine kinase, Aspartate beta-hydroxylase, epidermal growth factor receptor (EGFR); neural cell adhesion molecule (NCAM); Prostase; prostatic acid phosphatase (PAP); elongation factor 2 mutated (ELF2M); Ephrin B2; fibroblast activation protein alpha (FAP); insulin-like growth factor 1 receptor (IGF-I receptor), carbonic anhydrase IX (CAIX); Proteasome (Prosome, Macropain) Subunit, Beta Type, 9 (LMP2); glycoprotein 100 (gp100);oncogene fusion protein consisting of breakpoint cluster region (BCR) and Abelson murine leukemia viral oncogene homolog 1 (Abl) (bcr-abl); tyrosinase; ephrin type-A receptor 2 (EphA2); ephrin type-A receptor 3 (EphA3), Fucosyl GM1; sialyl Lewis adhesion molecule (sLe); transglutaminase 5 (TGS5); high molecular weight-melanomaassociatedantigen (HMWMAA); o-acetyl-GD2 ganglioside (OAcGD2); Folate receptor beta; tumor endothelial marker 1 (TEM1/CD248); tumor endothelial marker 7-related (TEM7R); six transmembrane epithelial antigen of the prostate I (STEAP1); claudin 6 (CLDN6); thyroid stimulating hormone receptor (TSHR); G protein-coupled receptor class C group 5, member D (GPRCSD); IL-15 receptor (IL-15); chromosome X open reading frame 61 (CXORF61); CD97; CD179a; anaplastic lymphoma kinase (ALK); Polysialic acid; placenta-specific 1 (PLAC1); hexasaccharide portion of globoH glycoceramide (GloboH); mammary gland differentiation antigen (NY-BR-1); uroplakin 2 (UPK2); Hepatitis A virus cellular receptor 1 (HAVCR1); adrenoceptor beta 3 (ADRB3); pannexin 3 (PANX3); G protein-coupled receptor 20 (GPR20); lymphocyte antigen 6 complex, locus K 9 (LY6K); Olfactory receptor 51E2 (ORS IE2); TCR Gamma Alternate Reading Frame Protein (TARP); Wilms tumor protein (WT1); Cancer/testis antigen 1 (NY-ESO-1); Cancer/testis antigen 2 (LAGE-Ia); Melanoma associated antigen 1 (MAGE-A1); Melanoma associated antigen 3 (MAGE-A3); Melanoma associated antigen 4 (MAGE-A4); T cell receptor beta 2 chain C; ETS translocation-variant gene 6, located on chromosome 12p (ETV6-AML); sperm protein 17 (SPA17); X Antigen Family, Member 1A (XAGE1); angiopoietin-binding cell surface receptor 2 (Tie 2); melanoma cancer testis antigen-1 (MADCT-1); melanoma cancer testis antigen-2 (MAD-CT-2); Fos-related antigen 1; tumor protein p53, (p53); p53 mutant; prostein; survivin; telomerase; prostate carcinoma tumor antigen-1 (PCTA-1 or Galectin 8), melanoma antigen recognized by T cells 1 (MelanA or MARTI); Rat sarcoma (Ras) mutant; human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N-Acetyl glucosaminyl-transferase V (NA17); paired box protein Pax-3 (PAX3); Androgen receptor; Cyclin-AI; Cyclin B1;v-myc avian myelocytomatosis viral oncogene neuroblastoma derived homolog (MYCN); Ras Homolog Family Member C (RhoC); Tyrosinase-related protein 2 (TRP-2); Cytochrome P450 1B1(CYP IBI); CCCTC-Binding Factor (Zinc Finger Protein)-Like (BORIS or Brother of the Regulator of Imprinted Sites), Squamous Cell Carcinoma Antigen Recognized By T Cells 3 (SART3); Paired box protein Pax-5 (PAXS); proacrosin binding protein sp32 (OY-TES I); lymphocyte-specific protein tyrosine kinase (LCK); A kinase anchor protein 4 (AKAP-4); Peptidoglycan recognition protein, synovial sarcoma, X breakpoint 2 (SSX2); Receptor for Advanced Glycation End products (RAGE-I); renal ubiquitous 1 (RUI); renal ubiquitous 2 (RU2); legumain; human papilloma virus E6 (HPV E6); human papilloma virus E7 (HPV E7); intestinal carboxyl esterase; heat shock protein 70-2 mutated (mut hsp70-2); CD79a; CD79b; CD72; Leukocyte-associated immunoglobulin-like receptor 1 (LAIRI); Fc fragment of IgA receptor (FCAR or CD89); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module containing mucin-like hormone receptor-like 2 (EMR2); lymphocyte antigen 75 (LY75); Glypican-2 (GPC2); Glypican-3 (GPC3); Fc receptor-like 5 (FCRL5); and immunoglobulin lambda-like polypeptide 1 (IGLL1); and

- CD150, 5T4, ActRIIA, B7, TNF receptor superfamily member 17 (TNFRSF17, BCMA), CA-125, CCNA1, CD123, CD126, CD138, CD14, CD148, CD15, CD19, CD20, CD200, CD21, CD22, CD23, CD24, CD25, CD26, CD261, CD262, CD30, CD33, CD362, CD37, CD38, CD4, CD40, CD4OL, CD44, CD46, CD5, CD52, CD53, CD54, CD56, CD66a-d, CD74, CD8, CD80, CD92, CE7, CS-1, CSPG4, ED-B fibronectin, EGFR, EGFRvlll, EGP-2, EGP-4, EPHa2, ErbB2, ErbB3, ErbB4, FBP, HER1-HER2 in combination, HER2-HER3 in combination, HERV-K, HIV-1 envelope glycoprotein gp120, HIV-1 envelope glycoprotein gp41, HLA-DR, HLA class I antigen alpha G, HM1.24, K-Ras GTPase, HMW-MAA, Her2, Her2/neu, IGF-1R, IL-11Ralpha, IL-13R-alpha2, IL-2, IL-22R-alpha, IL-6, IL-6R, la, li, L1-CAM, L1-cell adhesion molecule, Lewis Y, LI-CAM, MAGE A3, MAGE-A1, MART-1, MUC1, NKG2C ligands, NKG2D Ligands, NYESO-1, OEPHa2, PIGF, PSCA, PSMA, ROR1, T101, TAC, TAG72, TIM-3, TRAIL-R1, TRAIL-R1 (DR4), TRAIL-R2 (DR5), VEGF, VEGFR2, WT-I, a G-protein coupled receptor, alphafetoprotein (AFP), an angiogenesis factor, an exogenous cognate binding molecule (ExoCBM), oncogene product, anti-folate receptor, c-Met, carcinoembryonic antigen (CEA), cyclin (D 1), ephrinB2, epithelial tumor antigen, estrogen receptor, fetal acetylcholine e receptor, folate binding protein, gp100, hepatitis B surface antigen, Epstein-Barr nuclear antigen 1,

Latent membrane protein 1, Secreted protein BARF1, P2X7 purinoceptor, Syndecan-1, kappa chain, kappa light chain, kdr, lambda chain, livin, melanoma-associated antigen, mesothelin, mouse double minute 2 homolog (MDM2), mucin 16 (MUC16), mutated p53, mutated ras, necrosis antigens, oncofetal antigen, ROR2, progesterone receptor, prostate specific antigen, tEGFR, tenascin, β2-Microgiobulin, Fc Receptor-like 5 (FcRL5).

These cancer-associated antigens are shown in a list of different sub-lists merely for illustrative purposes as it should be appreciated that one or more cancer-associated antigens of a given sub-list could be combined with one or more cancer-associated antigents of one or more different sub-lists to form a different listing.

In some embodiments, the helper T cell epitope is a peptide containing less than or about 20 amino acids and/or amino acid analogs.

In some embodiments, the helper T cell epitope is selected from the group consisting of:

- Peptides derived from polio virus, such as (PV 103-115) KLFAVWKITYKDT;
- pan-DR binding (PADRE) peptides, such as DAla-Lys-Cha-Val-Ala-Ala-Trp-Thr-Leu-Lys-Ala-Ala-DAla;
- Peptides derived from tetanus toxin, such as (TT593-599) YSYFPSV, (TT830-843) QYIKANSKFIGITE, (TT830-844) QYIKANSKFIGITEL, (TT1084-1099) VSIDKFRIFCKANPK, (TT1174-1189) LKFIIKRYTPNNEIDS, (TT1064-1079) IREDNNITLKLDRCNN, and (TT947-967) FNNFTVSFWLRVPKVSASHLE;
- Peptides derived from *Neisseria meningitidis,* such as YAFKYARHANVGRNAFELFL ("YAF"); and
- Peptides derived from *P. falciparum* CSP such as, EKKIAKMEKASSVFNVNN.

In certain embodiments of the compounds of formula (II), $R^4$ is selected from:

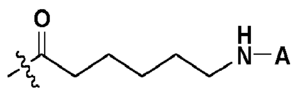

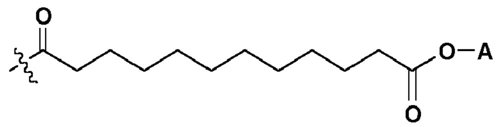

wherein A is H or a moiety comprising either i) at least one T cell epitope selected from a helper T cell epitope or a CD8 epitope, ii) at least one B cell epitope or iii) at least one T cell epitope selected from a helper T cell epitope or a CD8 epitope, and at least one B cell epitope. Preferably, A is a moiety comprising at least one B cell epitope.

In certain embodiments, the compound of formula (II) is selected from the group consisting of:

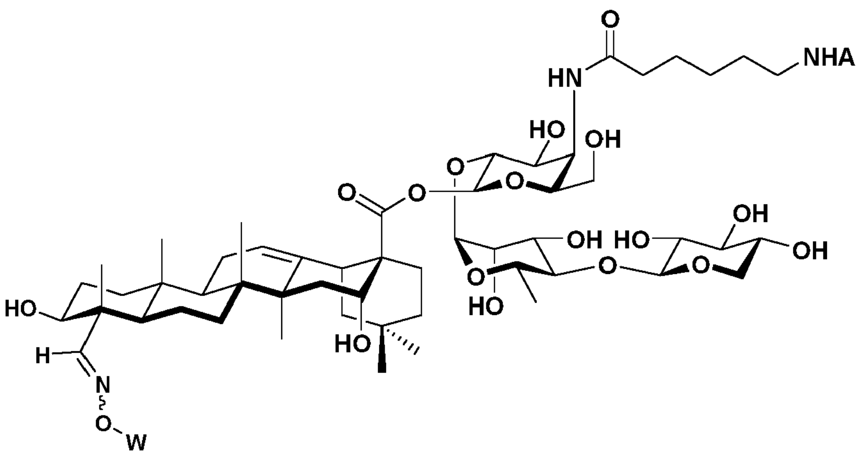

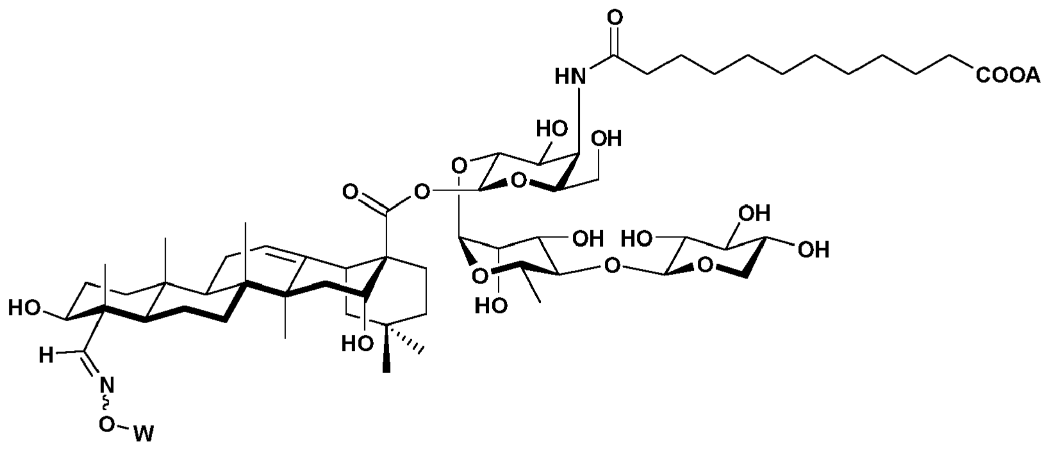

wherein W and A are independently H or a moiety comprising either i) at least one T cell epitope selected from a helper T cell epitope or a CD8 epitope, ii) at least one B cell epitope or iii) at least one T cell epitope selected from a helper T cell epitope or a CD8 epitope, and at least one B cell epitope;
with the proviso that the compound of formula (II) comprises at least one T cell epitope and at least one B cell epitope.

Preferably, A is a moiety comprising at least one B cell epitope and W is a moiety comprising at least one T cell epitope.

__**Compounds of formula (III) and (IV): intermediate compounds**__

The invention provides compounds of general formula (III) or a pharmaceutically acceptable salt thereof

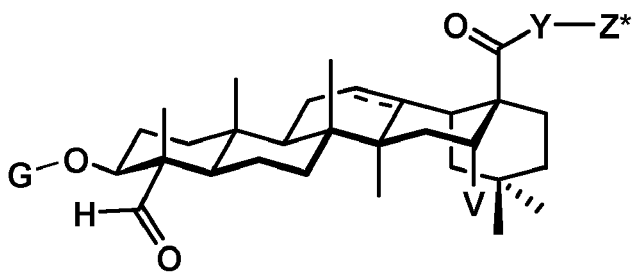

(III)

wherein

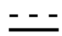

, G, V and Y take the meanings and particular embodiments as in formula (I) or formula (II) and Z* takes the meanings and particular embodiments as in formula (II).

The invention also provides compounds of general formula (IV) or a pharmaceutically acceptable salt thereof

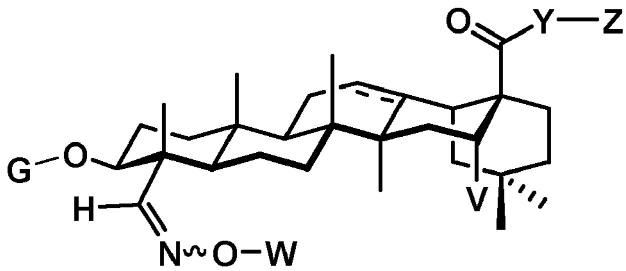

(IV)

wherein

- - -,

, G, V and Y take the meanings and particular embodiments as in formula (I) or (II), Z takes the meanings as in formula (I) and W take the meanings and particular embodiments as in formula (II).

Compounds of formula (III) and (IV) may be used as intermediate compounds for the preparation of self-adjuvanting vaccines of formula (II).

## Synthesis of Compounds

The compounds of formula (I) and (II) or a pharmaceutically salt thereof according to the present invention may be obtained from a compound of formula (V) or a pharmaceutically acceptable salt thereof

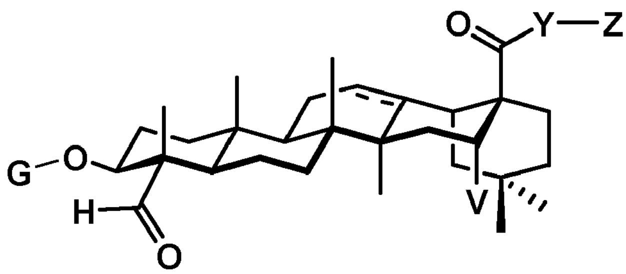

(V)

wherein

- - -,

, G, V, Y and Z take the same meanings and particular embodiments as in formula (I).

C4-aldehyde saponin compounds of formula (V) are available from known synthetic methods such as those disclosed in WO2009/126737, WO2015/184451, WO2017/079582, WO2017/106836, WO2018/191598, WO2018/200645 WO2018/200656 and WO2019/079160.

In some embodiments, the method of synthesizing a compound of formula (I) or a salt thereof comprises the steps of:

a) providing a compound of formula (V) or a salt thereof, and

b) reacting the compound of formula (V) or a salt thereof with $NH_2OH$ or a salt thereof to form a compound of formula (I) or a salt thereof.

In some embodiments, the method of synthesizing a compound of formula (II) or a salt thereof comprises the steps of:

a) providing a compound of formula (V) or a salt thereof,

b) conjugating the compound of formula (V) or a salt thereof with a moiety comprising either i) at least one T cell epitope selected from a helper T cell epitope or a CD8 epitope, ii) at least one B cell epitope or iii) at least one T cell epitope selected from a helper T cell epitope or a CD8 epitope and at least one B cell epitope, to form a compound of formula (III) or a salt thereof, and

c) reacting the compound of formula (III) or a salt thereof with a compound of formula

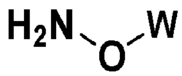

or a salt thereof, wherein W is H or a moiety comprising either i) at least one T cell epitope selected from a helper T cell epitope or a CD8 epitope, ii) at least one B cell epitope or iii) at least one T cell epitope selected from a helper T cell epitope or a CD8 epitope and at least one B cell epitope, to form a compound of formula (II) or a salt thereof.

In some embodiments, the method of synthesizing a compound of formula (II) or a salt thereof comprises the steps of:

a) providing a compound of formula (V),
b) reacting the compound of formula (V) or a salt thereof with a compound of formula

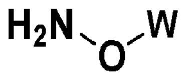

or a salt thereof, wherein W is H or a moiety comprising either i) at least one T cell epitope selected from a helper T cell epitope or a CD8 epitope, ii) at least one B cell epitope or iii) at least one T cell epitope selected from a helper T cell epitope or a CD8 epitope and at least one B cell epitope, to form a compound of formula (IV) or a salt thereof, and
c) if desired or required (as the compound of formula (IV) when W is a moiety comprising at least one T cell epitope and at least one B cell epitope is already encompassed within formula (II)), conjugating the compound of formula (IV) or a salt thereof with a moiety comprising either i) at least one T cell epitope selected from a helper T cell epitope or a CD8 epitope, ii) at least one B cell epitope or iii) at least one T cell epitope selected from a helper T cell epitope or a CD8 epitope and at least one B cell epitope, to form a compound to form a compound of formula (II) or a salt thereof.

In some embodiments, the conjugation through the chain of the carbohydrate domain with a moiety comprising at least one T cell epitope, at least one B cell epitope or both epitopes, i.e. the conversion of Z into Z*, is carried out via a click chemistry reaction.

The term 'click chemistry' has been introduced to describe specific chemical reactions, which are fast, reliable and can be selectively applied to the synthesis of functional materials and biomolecule conjugates. Click chemistry can be broadly defined as a ligation reaction in which two reactants are joined under mild or ambient conditions to provide the desired product in high chemical yield and short time.

Thus, in some embodiments, Z in the compounds of formula (I), formula (IV) or formula (V) is functionalized, preferably via the $R^4$ substituent, with a first functional group of a specific binding pair capable of forming a covalent bond with a complementary second functional group of said binding pair. This first functional group is suitable for conjugation purposes with a compound comprising an immune system activating agent (e.g. either T cell epitope or B cell epitope or both epitopes) functionalized with a complementary second functional group of said binding pair, capable of forming a covalent bond with said first functional group, thus forming Z* in the compounds of formula (II) and (III).

The term "binding pair" as used herein refers to a pair of different molecules (e.g. (i) the compounds of formula (I), formula (IV) or formula (V) and (ii) the compound comprising an immune system activating agent), each comprising its own specific functional group, both functional groups have particular specificity for (or complimentary to) each other. In other words, these groups, under normal conditions, are capable of covalently binding to each other in preference to binding to other molecules. Non-limiting examples of such binding pairs are carboxylic acid-amine, thiol-maleimide, azide-alkyne, aldehyde-hydroxylamine etc.

In general, a functional group is a specific group or moiety of atoms or bonds within molecules that is responsible for the characteristic chemical reactions of those molecules. In particular, a functional group, or a functional group of a binding pair, as defined herein, refers to a specific reactive group or moiety of atoms or bonds of the binding pair (hereinafter "a first functional group") capable of binding to another functional group of said binding pair (hereinafter "a second functional group"). As mentioned above, the first and the second functional groups are complementary to each other. In the above non-limiting examples, the first functional groups are carboxylic acid, thiol, azide or aldehyde and their complementary (second) functional groups are amine, maleimide, alkyne or hydroxylamine, respectively.

In a particular embodiment, i) the first functional group of the specific binding pair is aldehyde, ketone, isothiocyanate, carboxylic acid or derivative thereof such as ester, anhydride, acyl halide, tosyl and N-hydrosuccinimide (NHS), and the second functional group of said binding pair is amine, or vice versa; ii) the first functional group of the specific binding pair is alkyne or phosphine, and the second functional group of said binding pair is azide, or vice versa; iii) the first functional group of the specific binding pair is cycloalkene, cycloalkyne, cyclopropane, isonitrile (isocyanide) or vinyl boronic acid, and the second functional group of said binding pair is tetrazine, or vice versa; iv) the first functional group of the specific binding pair is alkyne or maleimide, and the second functional group of said binding pair is thiol, or vice versa; v) the first functional group of the specific binding pair is conjugated diene, and the second functional group of said binding pair is substituted alkene, or vice versa; vi) the first functional group of the specific binding pair is alkene, alkyne or copper acetylide, and the second functional group of said binding pair is nitrone, or vice versa; vii) the first functional group of the specific binding pair is aldehyde or ketone, and the second functional group of said binding pair is alkoxyamine, hydroxylamine, hydrazine or hydrazide, or vice versa, In a more particular embodiment, the first functional group of the specific binding pair is carboxylic acid or derivative thereof such as ester, anhydride, acyl halide, tosyl and N-hydrosuccinimide (NHS), and the second functional group of said binding pair is amine, or vice versa; In a more particular embodiment, the specific binding pair is carboxylic acid or derivative thereof such as ester ---- amine.

In a particular embodiment, the conjugation of Z with a moiety comprising an immune-system activating agent (e.g. either T cell epitope or B cell epitope or both epitopes) may be carried out via a divalent linker. Examples of divalent linkers are reactive PEG derivatives of formula

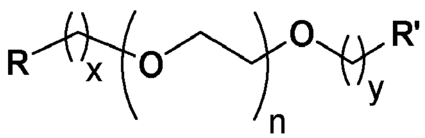

wherein n, x and y are positive integers and R and R' are reactive fuctional groups.

In a particular embodiment, n is selected from 1 to 20, preferably 1 to 10 and more preferably 1 to 5 such as 1, 2, 3, 4 or 5. In another particular embodiment, x and y are independently selected from 1, 2 or 3. In another particular embodiment, R and R' are independently selected from carboxylic acid or derivative thereof such as ester, anhydride, acyl halide, tosyl and N-hydrosuccinimide (NHS).

In a more particular embodiment, the linker is

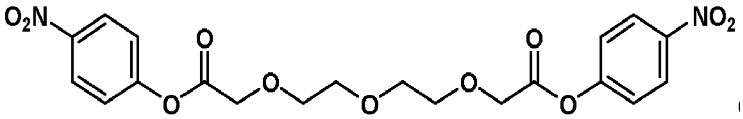

or a salt thereof.

In some embodiments, the functionalization at the C4-position of the triterpene domain with a moiety comprising either i) at least one T cell epitope, ii) at least one B cell epitope or iii) at least one T cell epitope and at least one B cell epitope may be achieved by reacting a saponin having a C4-aldehyde substituent (e.g. compound of formula (III) or formula (V)) with an immune-system activating agent comprising the following aminooxy reactive group

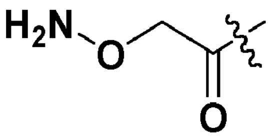

## Formulations

Another aspect of the present application relates to a formulation or pharmaceutical composition. The pharmaceutical composition comprises a compound or a pharmaceutically acceptable salt thereof according to the present invention, a pharmaceutically acceptable carrier and optionally an antigen.

In some embodiments, the pharmaceutical composition comprises a compound of formula (I) or a pharmaceutically acceptable salt thereof, a pharmaceutically acceptable carrier and an antigen.

As used herein, the term "antigen" refers to a substance that is able to generate a specific immune response and induce the formation of specific antibodies or specially sensitized T cells or both. In the context of the present invention the antigen is therefore capable of activating lymphocytes, and as such, is a complete antigen, i.e., possesses antigenic properties de novo, being able to generate an immune response by themselves. Said antigen is characterized by a molecular mass above 14 kDa, having a complex chemical composition and ideally contains aromatic radicals. Said antigens belong to four main groups, proteins, polysaccharides, nucleic acids and lipids, preferably proteins.

An "immune response" to an antigen or immunogenic composition is the development in a subject of a humoral and/or a cell-mediated immune response to molecules present in the antigen or vaccine composition of interest. For purposes of the present invention, a "humoral immune response" is an antibody-mediated immune response and involves the induction and generation of antibodies that recognize and bind with some affinity for the antigen in the immunogenic composition of the invention, while a "cell-mediated immune response" is one mediated by T-cells and/or other white blood cells.

A "cell-mediated immune response" also refers to the production of cytokines, chemokines and other such molecules produced by activated T-cells and/or other white blood cells, including those derived from CD4+ and CD8+ T-cells. The ability of a particular antigen or composition to stimulate a cell-mediated immunological response may be determined by a number of assays, such as by lymphoproliferation (lymphocyte activation) assays, CTL cytotoxic cell assays, by assaying for T-lymphocytes specific for the antigen in a sensitized subject, or by measurement of cytokine production by T cells in response to re-stimulation with antigen. Such assays are well known in the art. See, e.g., Erickson et al. (1993) J. Immunol. 151:4189-4199; and Doe et al. (1994) Eur. J. Immunol. 24:2369-2376.

The antigen may be selected for instance from the group consisting of a neurodegeneration-associated antigen, an infection-associated antigen (e.g. bacterial-, viral-, or protozoal-associated antigen) or a cancer-associated antigen, also known as tumor-associated antigen or TACA. Particular examples of TACAs for coadministration with a compound of formula (I) are those listed hereinbefore.

Further examples of antigens useful in the present invention are, without limitation, tetanus toxoid, egg albumin, thyroglobulin, recombinant hemagglutinin B (rHagB) antigen, recombinant protein from H1N1 influenza, protective BpOmpW from Burkholderia pseudomallei.

In some embodiments, the pharmaceutical composition comprises a compound of formula (II) or a pharmaceutically acceptable salt thereof and a pharmaceutically acceptable carrier.

In certain embodiments, the pharmaceutical composition includes a pharmaceutically acceptable amount of a compound of the present application. In certain embodiments, the pharmaceutical composition includes an immunologically effective amount of an antigen. In certain embodiments, the compounds of the application and an antigen form an active ingredient. In certain embodiments, the compound of the present application alone forms an active ingredient. The amount of active ingredient(s) which can be combined with a carrier material to produce a single dosage form will vary depending upon the host being treated, and the particular mode of administration. The amount of active ingredient(s) that can be combined with a carrier material to produce a single dosage form will generally be that amount of the compound which produces a therapeutic effect. Generally, this amount will range from about 1 % to about 99 % of active ingredient, preferably from about 5 % to about 70 %, most preferably from about 10 % to about 30 %, or from about 1 % to 99 %, preferably from 10 % to 90 %, 20 % to 80 %, 30 % to 70 %, 40 % to 60 %, 45 % to 55 %, or about 50 %.

In certain embodiments, formulations of the present application include injectable formulations.

In one aspect the present application provides formulations comprising a liposome formulation of MPL and a compound of the present invention. In another aspect the present application provides formuiations comprising MPL, a compound of the present invention and a squaiene emulsion. In another aspect the present application provides formulations comprising MPL, a compound of the present invention, and CpG 7909 or CpG 1018. MPL is a heterogeneous mixture of molecules from a biological source including both agonists and antagonists for TLR4. CpG 7909 is an immunomodulating synthetic oligonucleotide designed to specifically agonise the Toll-like receptor 9 (TLR9).

In another aspect the present application provides formulations comprising immune stimulating complexes (ISCOM) or ISCOM matrices of a compound of the present invention. In another aspect the present application provides formulations comprising ISCOM matrices of a compound of the present invention and an antigen. ISCOMs are open cage-like nanoparticulate structures comprising a saponin (here, a compound of the present invention), cholesterol, phospholipid and an antigen. ISCOM particules are typically spherical of approximately 40 nm diameter. ISCOMs deliver antigen to the cytosol, and have been demonstrated to promote antibody response and induction of T helper cell as well

as cytotoxic T lymphocyte responses in variety of experimental animal models. ISCOM matrices formulations (e.g. ISCOMATRIX and Matrix-M) contain the same components and have the same structure as the ISCOM but without the incorporated antigen.

Wetting agents, emuisifiers and lubricants, such as sodium lauryl sulfate and magnesium stearate, as well as coloring agents, release agents, coating agents, sweetening, flavoring and perfuming agents, preservatives and antioxidants can also be present in the compositions.

Non-limiting examples of pharmaceutically-acceptable antioxidants include: water soluble antioxidants, such as ascorbic acid, cysteine hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, alpha-tocopherol, and the like; and metal chelating agents, such as citric acid. ethylenediamine tetraacetic acid (EDTA), sorbitol, tartaric acid, phosphoric acid, and the like.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Non-limiting examples of suitable aqueous and nonaqueous carriers, which may be employed in the pharmaceutical compositions of the present application include water, alcohols (including but not limited to methanol, ethanol, butanol, etc.), polyols (including but not limited to glycerol, propylene glycol, polyethylene glycol, etc.), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain additives such as preservatives, wetting agents, emulsifying agents and dispersing agents. Prevention of the action of microorganisms upon the subject compounds may be ensured by the inclusion of various antibacterial and antifungal agents, for example, paraben, chiorobufanoi, phenol sorbic acid, and the like, it may also be desirable to include isotonic agents, such as sugars, sodium chloride, and the like into the compositions, in addition, prolonged absorption of the injectable pharmaceutical form may be brought about by the inclusion of agents which delay absorption such as aluminum monostearate and gelatin.

In some cases, in order to prolong the effect of a formulation, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution, which in turn, may depend upon crystal size and crystalline form.

Regardless of the route of administration selected, the compounds of the present application, which may be used in a suitable hydrated form, and/or the pharmaceutical compositions of the present application, are formulated into pharmaceutically acceptable dosage forms by conventional methods known to those of skill in the art.

Actual dosage levels of the active ingredients in the pharmaceutical compositions of the present application may be varied so as to obtain an amount of the active ingredient that is effective to achieve the desired therapeutic response for a particular patient, composition, and mode of administration, without being toxic to the patient.

The selected dosage level will depend upon a variety of factors including the activity of the particular compound of the present application employed, or the ester, salt or amide thereof, the route of administration, the time of administration, the rate of excretion or metabolism of the particular compound being employed, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the present application employed in the pharmaceutical composition at levels lower than that required to achieve the desired therapeutic effect and then gradually increasing the dosage until the desired effect is achieved.

In some embodiments, a compound or pharmaceutical composition of the present application is provided to a subject chronically. Chronic treatments include any form of repeated administration for an extended period of time, such as repeated administrations for one or more months, between a month and a year, one or more years, or longer. In many embodiments, a chronic treatment involves administering a compound or pharmaceutical composition of the present application repeatedly over the life of the subject. Preferred chronic treatments involve regular administrations, for example one or more times a day, one or more times a week, or one or more times a month, in general, a suitable dose, such as a daily dose of a compound of the present application, will be that amount of the compound that is the lowest dose effective to produce a therapeutic effect. Such an effective dose will generally depend upon the factors described above.

Generally, doses of the compounds of the present application for a patient, when used for the indicated effects, will range from about 0.0001 to about 100 mg per kg of body weight per day. Preferably the daily dosage will range from 0.001 to 50 mg of compound per kg of body weight, and even more preferably from 0.01 to 10 mg of compound per kg

of body weight. However, lower or higher doses can be used, in some embodiments, the dose administered to a subject may be modified as the physiology of the subject changes due to age, disease progression, weight, or other factors.

In some embodiments, provided adjuvant compounds of the present application are administered as pharmaceutical compositions or vaccines. In certain embodiments, it is contemplated that the amount of adjuvant compound administered will be 1-2000 μg. In certain embodiments, it is contemplated that the amount of adjuvant compound administered will be 1-1000 μg. In certain embodiments, it is contemplated that the amount of adjuvant compound administered will be 1-500 μg. in certain embodiments, it is contemplated that the amount of adjuvant compound administered will be 1-250 μg. In certain embodiments, it is contemplated that the amount of adjuvant compound administered will be 100-1000 μg. In certain embodiments, it is contemplated that the amount of adjuvant compound administered will be 100-500 μg. In certain embodiments, it is contemplated that the amount of adjuvant compound administered will be 100-200 μg. In certain embodiments, it is contemplated that the amount of adjuvant compound administered will be 250-500 μg. In certain embodiments, it is contemplated that the amount of adjuvant compound administered will be 10-1000 μg. In certain embodiments, it is contemplated that the amount of adjuvant compound administered will be 500-1000 μg. In certain embodiments, it is contemplated that the amount of adjuvant compound administered will be 50-250 μg. in certain embodiments, it is contemplated that the amount of adjuvant compound administered will be 50-500 μg.

In some embodiments, provided adjuvant compounds of the present application are administered as pharmaceutical compositions or vaccines. In certain embodiments, it is contemplated that the amount of adjuvant compound administered will be 1-2000 mg. In certain embodiments, it is contemplated that the amount of adjuvant compound administered will be 1-1000 mg. In certain embodiments, it is contemplated that the amount of adjuvant compound administered will be 1-500 mg. In certain embodiments, it is contemplated that the amount of adjuvant compound administered will be 1-250 mg. In certain embodiments, it is contemplated that the amount of adjuvant compound administered will be 100-1000 mg. In certain embodiments, it is contemplated that the amount of adjuvant compound administered will be 100-500 mg. In certain embodiments, it is contemplated that the amount of adjuvant compound administered will be 100-200 mg. In certain embodiments, it is contemplated that the amount of adjuvant compound administered will be 250-500 mg. In certain embodiments, it is contemplated that the amount of adjuvant compound administered will be 10-1000 mg. In certain embodiments, it is contemplated that the amount of adjuvant compound administered will be 500-1000 mg. In certain embodiments, it is contemplated that the amount of adjuvant compound administered will be 50-250 mg. In certain embodiments, it is contemplated that the amount of adjuvant compound administered will be 50-500 mg. In certain embodiments, it is contemplated that the amount of adjuvant compound administered will be 0.01-215.4 mg.

In certain embodiments, it is contemplated that the amount of adjuvant administered will be 1000-5000 μg/kg. In certain embodiments, it is contemplated that the amount of adjuvant administered will be 1000-4000 μg/kg. In certain embodiments, it is contemplated that the amount of adjuvant administered will be 1000-3000 μg/kg. In certain embodiments, it is contemplated that the amount of adjuvant administered will be 1000-2000 μg/kg. In certain embodiments, it is contemplated that the amount of adjuvant administered will be 2000-5000 μg/kg. In certain embodiments, it is contemplated that the amount of adjuvant administered will be 2000-4000 μg/kg. In certain embodiments, it is contemplated that the amount of adjuvant administered will be 2000-3000 μg/kg. In certain embodiments, it is contemplated that the amount of adjuvant administered will be 3000-5000 μg/kg. In certain embodiments, it is contemplated that the amount of adjuvant administered will be 3000-4000 μg/kg. In certain embodiments, it is contemplated that the amount of adjuvant administered will be 4000-5000 μg/kg. In certain embodiments, it is contemplated that the amount of adjuvant administered will be 1-500 μg/kg. In certain embodiments, it is contemplated that the amount of adjuvant administered will be 500-1000 μg/kg. In certain embodiments, it is contemplated that the amount of adjuvant administered will be 1000-1500 μg/kg. In certain embodiments, it is contemplated that the amount of adjuvant administered will be 1 mg/kg. In certain embodiments, it is contemplated that the amount of adjuvant administered will be 2 mg/kg. In certain embodiments, it is contemplated that the amount of adjuvant administered will be 3 mg/kg. In certain embodiments, it is contemplated that the amount of adjuvant administered will be 4 mg/kg. In certain embodiments, it is contemplated that the amount of adjuvant administered will be 5 mg/kg. In certain embodiments, it is contemplated that the amount of adjuvant administered will be 0.0029-5 mg/kg. In certain embodiments, the amount of adjuvant administered in females is less than the amount of adjuvant administered in males. In certain embodiments, the amount of adjuvant administered to infants is less than the amount of adjuvant administered to adults. In certain embodiments, the amount of adjuvant administered to pediatric recipients is less than the amount of adjuvant administered to adults. In certain embodiments, the amount of adjuvant administered to immunocompromised recipients is more than the amount of adjuvant administered to healthy recipients. In certain embodiments, the amount of adjuvant administered to elderly recipients is more than the amount of adjuvant administered to non-elderly recipients.

If desired, the effective dose of the active compound may be administered as two, three, four, five, six or more sub-doses administered separately at appropriate intervals throughout the day, optionally, in unit dosage forms. While it is possible for a compound of the present application to be administered alone, in certain embodiments the compound

is administered as a pharmaceutical formulation or composition as described above.

The compounds according to the present application may be formulated for administration in any convenient way for use in human or veterinary medicine, by analogy with other pharmaceuticals.

The present application provides kits comprising pharmaceutical formulations or compositions of a compound of the present application. In certain embodiments, such kits include the combination of a compound of formulae I and/or II and an antigen. The agents may be packaged separately or together. The kit optionally includes instructions for prescribing the medication, in certain embodiments, the kit includes multiple doses of each agent. The kit may include sufficient quantities of each component to treat one or more subject for a week, two weeks, three weeks, four weeks, or multiple months. The kit may include a full cycle of immunotherapy. In some embodiments, the kit includes a vaccine comprising one or more bacterial-, viral-, protozoal-, neurodegenerative disease- or cancer-associated antigens, and one or more provided compounds.

## Uses and methods of use of the compounds of the invention: Adjuvants and vaccines

Compounds of formula (I) or a salt thereof may be used as adjuvants in vaccines to increase the immune response to an antigen or enhance certain activities of cells from the immune system. Moreover, compounds of formula (II) or a salt thereof, which are covalently linked to a moiety comprising at least one B cell epitope and a moiety comprising at least one T cell epitope or to a moiety comprising both epitopes, may be used as a self-adjuvanting vaccine.

Another aspect of the present application relates to a compound of the present invention, e.g. compounds of formula (I) and/or (II), or a pharmaceutical compositon thereof for use in medicine, and more particularly, for use in the treatment and/or prevention of cancer, an infectious disease or a neurodegenerative disease.

It will be understood that, when referring to the compounds of formula (I), which do not contain within their structure an antigen or epitope, their use in therapy requires that the compound is administered in combination with the antigen to which a response is desired. In a preferred embodiment, the compounds of formula (I) can be formulated with the antigen into an immunogenic composition. In this case, any reference to the medical use of the compounds of formula (I) has to be understood as a reference to medical use of an immunogenic composition comprising a compound of formula (I) and an antigen. Similarly, any reference to a pharmaceutical composition of the compounds of formula (I) has to be understood as a composition comprising an antigen. Conversely, since the compounds of formula (II) are self-adjuvanted, these compounds can be included into a pharmaceutical composition soley in the presence of a pharmaceutically acceptable vehicle without the need of an antigen.

Another aspect of the present application relates to a method for the treatment and/or prevention of a disorder in a subject said method comprising the administration of an effective amount of a compound of the present invention, e.g. compounds of formula (I) and/or (II), or a pharmaceutical compositon thereof to the subject, wherein the disorder is cancer, an infectious disease or a neurodegenerative disease.

Another aspect of the present application relates to a compound of the present invention, e.g. compounds of formula (I) and/or (II), or a pharmaceutical compositon thereof for use in the immunization of a subject.

Another aspect of the present application relates to a method for immunizing a subject, said method comprising administering to the subject an effective amount of a compound of the present invention, e.g. compounds of formula (I) and/or (II), or a pharmaceutical composition thereof.

Any animal that may experience the beneficial effects of the compositions of the present application is within the scope of subjects that may be treated, in some embodiments, the subjects are mammals. In some embodiments, the subjects are humans.

The vaccines of the present application may be used to confer resistance to infection by either passive or active immunization. When the vaccines of the present application are used to confer resistance through active immunization, a vaccine of the present application is administered to an animal to elicit a protective immune response which either prevents or attenuates a proliferative or infectious disease. When the vaccines of the present application are used to confer resistance to infection through passive immunization, the vaccine is provided to a host animal (e.g., human, dog, or mouse), and the antisera elicited by this vaccine is recovered and directly provided to a recipient suspected of having an infection or disease or exposed to a causative organism.

The present application thus concerns and provides a means for preventing or attenuating a proliferative disease resulting from organisms or tumor cells which have antigens that are recognized and bound by antisera produced in response to the immunogenic antigens included in vaccines of the present application. As used herein, a vaccine is said to prevent or attenuate a disease if its administration to an animal results either in the total or partial attenuation (i.e., suppression) of a symptom or condition of the disease, or in the total or partial immunity of the animal to the disease.

The administration of the vaccine (or the antisera which it elicits) may be for either a "prophylactic" or "therapeutic" purpose. When provided prophylacticaily, the vaccine(s) are provided in advance of any symptoms of proliferative disease. The prophylactic administration of the vaccine(s) serves to prevent or attenuate any subsequent presentation of the disease. When provided therapeutically, the vaccine(s) is provided upon or after the detection of symptoms which

indicate that an animal may be infected with a pathogen or have a certain cancer. The therapeutic administration of the vaccine(s) serves to attenuate any actual disease presentation. Thus, the vaccines may be provided either prior to the onset of disease proliferation (so as to prevent or attenuate an anticipated infection or cancer) or after the initiation of an actual proliferation.

Thus, in one aspect the present application provides vaccines comprising one or more antigens (e.g. one or more bacterial, viral, protozoal, neurodegenerative disease or tumor-related antigens) in combination with one or more inventive compounds. In some embodiments, the vaccine comprises a single bacterial, viral, protozoal, neurodegenerative disease or tumor-related antigen in combination with one inventive compound. In some embodiments, the vaccine comprises two or more bacterial, viral, protozoal, neurodegenerative disease or tumor-related antigens in combination with a single inventive compound. In some embodiments, the vaccine comprises two or more bacterial, viral, protozoal, neurodegenerative disease or tumor-related antigens in combination with two or more inventive compounds. In some embodiments, the vaccine comprises a single bacterial, viral, protozoal, neurodegenerative disease or tumor-related antigens in combination with two or more inventive compounds.

In some embodiments, one or more antigens of provided vaccines are bacterial-associated antigens.

In certain embodiments, one or more antigens of provided vaccines are viral-associated antigens.

In certain embodiments, one or more antigens of provided vaccines are protozoal-associated antigens.

In certain embodiments, one or more antigens of provided vaccines are neurodegenerative disease-associated antigens.

In certain embodiments, one or more antigens of provided vaccines are cancer- or tumor-associated antigens including for instance those listed hereinbefore.

In certain embodiments, one or more antigens are included within the structure of the inventive compounds, e.g. in compounds of formula (II), and therefore although possible it is not required the administration of additional antigens.

One of ordinary skill in the art will appreciate that vaccines may optionally include a pharmaceutically acceptable excipient or carrier. Thus, according to another aspect, provided vaccines may comprise one or more antigens that are optionally conjugated to a pharmaceutically acceptable excipient or carrier. In some embodiments, said one or more antigens are conjugated covalently to a pharmaceutically acceptable excipient. In other embodiments, said one or more antigens are non-covalentiy associated with a pharmaceutically acceptable excipient.

As described above, adjuvants may be used to increase the immune response to an antigen. According to the present application, provided vaccines may be used to invoke an immune response when administered to a subject. In certain embodiments, an immune response to an antigen may be potentiated by administering to a subject a provided vaccine in an effective amount to potentiate the immune response of said subject to said antigen.

As described above, the compounds of the present application may be used in cancer vaccines as adjuvants in combination with tumor-associated antigens such as those listed hereinbefore. In certain embodiments, said vaccines may be used in the treatment or prevention of neoplasms. In certain embodiments, the neoplasm is a benign neoplasm. In other embodiments, the neoplasm is a malignant neoplasm. Any cancer may be treated using compounds of the invention with an antigen.

In certain embodiments, the malignancy is a hematological malignancy.

Other cancers besides hematological malignancies may also be treated using compounds of formulae I and II. In certain embodiments, the cancer is a solid tumor.

In certain embodiments, compounds and pharmaceutical compositions of the present application can be employed in combination therapies, that is, the compounds and pharmaceutical compositions can be administered concurrently with, prior to, or subsequent to, one or more other desired therapeutics or medical procedures. The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder (e.g., an inventive compound may be administered concurrently with another antiproliferative agent), or they may achieve different effects (e.g., control of any adverse effects).

For example, other therapies or anticancer agents that may be used in combination with the inventive anticancer agents of the present application include surgery, radiotherapy (gamma-radiation, neutron beam radiotherapy, electron beam radiotherapy, proton therapy, brachytherapy, and systemic radioactive isotopes, to name a few), endocrine therapy, biologic response modifiers (interferons, interleukins, and tumor necrosis factor (TNF) to name a few), hyperthermia and cryotherapy, agents to attenuate any adverse effects (e.g., antiemetics), and other approved chemotherapeutic drugs, including, but not limited to, alkylating drugs (mechlorethamine, chlorambucil, Cyclophosphamide, Melphalan, Ifosfamide), antimetabolites (Methotrexate), purine antagonists and pyrimidine antagonists (6-Mercaptopurine, 5-Fluorouracil, Cytarabile, Gemcitabine), spindle poisons (Vinblastine, Vincristine, Vinorelbine, Paclitaxel), podophyllotoxins (Etoposide, Irinotecan, Topotecan), antibiotics (Doxorubicin, Bleomycin, Mitomycin), nitrosoureas (Carmustine, Lomustine), inorganic ions (Cisplatin, Carboplatin), enzymes (Asparaginase), and hormones (Tamoxifen, Leuprolide, Flutamide, and Megestrol), to name a few. Additionally, the present invention also encompasses the use of certain cytotoxic or

anticancer agents currently in clinical trials and which may ultimately be approved by the FDA (including, but not limited to, epothilones and analogues thereof and geldanamycins and analogues thereof). For a more comprehensive discussion of updated cancer therapies see, www.nci.nih.gov, a list of the FDA approved oncology drugs at www.fda.gov/cder/cancer/druglistframe.htm, and The Merck Manual, Seventeenth Ed.1999, the entire contents of which are hereby incorporated by reference.

In another aspect, the present application provides a method of treating infectious disease in a subject comprising administering to the subject a therapeutically effective amount of a compound of formula (I) and an antigen or a therapeutically effective amount of a compound of formula (II). In some embodiments, the infection is bacterial. In some embodiments, the infection is viral. In some embodiments, the infection is protozoal. Examples of infectiuous diseases include, but are not limited to, malaria, acquired immunodeficiency syndrome, hepatitis and tuberculosis. In some embodiments, the subject is human.

In another aspect, the present application provides a method of treating neurodegenerative diseases in a subject comprising administering to the subject a therapeutically effective amount of a compound of formula (I) and an antigen or a therapeutically effective amount of a compound of formula (II). In some embodiments, the present application can be used in the treatment of neurodegenerative diseases (e.g. Alzheimer's disease).

It should be understood that the scope of the present disclosure includes all the possible combinations of embodiments disclosed herein.

The following examples are merely illustrative of certain embodiments of the invention and cannot be considered as restricting it in any way.

## EXAMPLES

### GENERAL INFORMATION - MATERIALS & METHODS

All commercially available materials were used without further purification except boron trifluoride diethyl etherate and trifluoromethanesulfonic anhydride, which were distilled from calcium hydride and phosphorus pentoxide, respectively, at 1 atm under $N_2$. All manipulations with air-sensitive reagents and chemical reactions were carried out under a dry argon atmosphere using standard Schlenk techniques. Air- and moisture-sensitive liquids and solutions were transferred via syringe. When anhydrous conditions were required, the appropriate reagents were dried via azeotropic removal of water with dry toluene. Molecular sieves were activated at 350 °C and were crushed immediately prior to use, then dried under vacuum. Organic solutions were concentrated under reduced pressure by rotary evaporation below 40 °C. Column chromatography was performed employing 230-400 mesh silica gel. Analytical thin-layer chromatography (TLC) was performed using aluminum-backed sheets pre-coated with 230-400 mesh silica gel 60 containing fluorescent indicator (F254). Preparative TLC (Analtech Uniplates) was performed using glass-backed sheets pre-coated with 500 micron silica gel containing fluorescent indicator (F254). TLC plates were visualized under UV light (254 nm) and by staininig with cerium ammonium molybdate (CAM), phosphomolybidic acid (PMA), or 5% sulfuric acid in ethanol solutions.

**$^1$H, APT $^{13}$C, COSY and HSQC Nuclear magnetic resonance (NMR).** $^1$H, APT$^{13}$C, COSY and HSQC spectra were recorded on a Bruker Avance III instrument ($^1$H NMR at 600 MHz and APT $^{13}$C NMR at 151 MHz). Chemical shifts are expressed in parts per million ($\delta$ scale) downfield from tetramethylsilane and are referenced to residual proton in the NMR solvent ($CDCl_3$: $\delta$ 7.26 for $^1$H NMR, $\delta$ 77.00 for $^{13}$C NMR; methanol-$d_4$: $\delta$ 3.31 for $^1$H NMR, $\delta$ 49.15 for $^{13}$C NMR). Data are presented as follows: chemical shift, multiplicity (s = singlet, br s = broad singlet, d = doublet, t = triplet, q = quartet, m = multiplet and/or multiple resonances), coupling constant in Hertz (Hz), integration, assignment.

**RP-HPLC/LC-MS and ESI$^+$-MS.** All reverse-phase RP-HPLC analyses/purifications were carried out on a Waters 1525 binary gradient system (Solv. A = 0.05% TFA in $H_2O$; Solv. B = 0.05% TFA in $CH_3CN$) equipped with a Waters 2998 photodiode array detector (PDA) combined with a low-resolution single quadrupole mass spectrometer (SQD2 Waters Corporation). Absorbances were monitored at wavelengths of 190-400 nm or 210-600 nm. Low-resolution mass spectra were recorded on the Waters SQD2 with electrospray ionization source (ESI) coupled with the Waters RP-HPLC system mentioned above.

**HR-ESI-MS.** High resolution electrospray ionization mass spectrometry (HR-ESI-MS) was performed on a Waters LCT Premier XE (Waters, Milford, MA, USA) in W-optics positive ionization scan mode. Mass spectromatry parameters were optimized to achieve the best signal-to-noise ratio: capillary voltage 1 kV, sample cone voltage 100 V, desolvation gas flow 600 $Lh^{-1}$, cone gas flow 50 $Lh^{-1}$, desolvation temperature 350 °C, source temperature 150 °C . The instrument was calibrated over the range m/z 200-2000 before measurement using a standard NaI solution (1 $\mu$M). In order to minimize the accuracy in the measurements, leucine-enkephalin was used as lockmass reference [2M+Na], m/z 1111,5459. Data analysis was performed with Masslynx software version 4.1 (Waters, Milford, MA, USA). Characterization by MS was confirmed after comparing the experimental isotopic pattern with the theoretical one.

**MALDI-TOF-HR-MS:** Compounds **23** and **24** were desalted using ZipTip® C4 micro-columns (Millipore) with

elution using 0.5μL SA (sinapinic acid, 10 mg/ml in [70:30] acetonitrile:trifluoroacetic acid 0.1%) matrix onto a GroundSteel massive 384 target (Bruker Daltonics). Matrix-assisted laser desorption/ionization-time of flight high resolution mass spectrometry (MALDI-TOF-HR-MS) analyses were carried out on an Autoflex III Samrtbeam MALDI-TOF spectrometer (Bruker Daltonics) which was used in linear mode with the following settings: 8.000-100.000 Th window, linear positive mode, ion source 1: 20 kV, ion source 2: 18.5 kV, lens: 9 kV, pulsed ion extraction of 120 ns, high gating ion suppression up to 1000 Mr. Mass calibration was performed externally with protein 1 standard calibration mixture (Bruker Daltonics) in the same range as the samples. Data acquisition was performed using FlexControl 3.0 software (Bruker Daltonics), and peak peaking and subsequent spectra analysis was performed using FlexAnalysis 3.0 software (Bruker Daltonics).

**Solid-Phase Peptide Synthesis (SPPS).** All (glyco)peptides were synthesized using the standard 9-fluorenylmethoxycarbonyl (Fmoc)/ tert-butyloxycarbonyl (tBu) protection strategy. Automated SPPS was carried out on a CEM Liberty Blue™ microwave-assisted synthesizer, using default cycles. Fmoc-deprotection cycles were performed with 20% piperidine in DMF, under microwave irradiation at 85-90 °C for 1 minute. Amino acid (AA) couplings involved reaction of 5 eq. of AA (0.2 M), in the presence of *N,N'*-diisopropylcarbodiimide (DIC) and ethyl cyano(hydroxyimino)acetate (Oxyma Pure), under microwave irradiation at 85-90 °C for 2 minutes. Upon completion of the sequence, the resin was transferred in a solid-phase peptide synthesis vessel provided with a sintered glass filter, and abundantly washed with MeOH prior treatment with the cleavage cocktail. Glyco-amino acid building block **17** (Scheme S2) was coupled manually, by suspending the resin in the solid-phase peptide synthesis vessel, following reagents addition and gentle suspension mixing via a stream of $N_2$.

## SYNTHETIC EXAMPLES

**Example 1: Prior Art Compound 13** [CP-III-066]

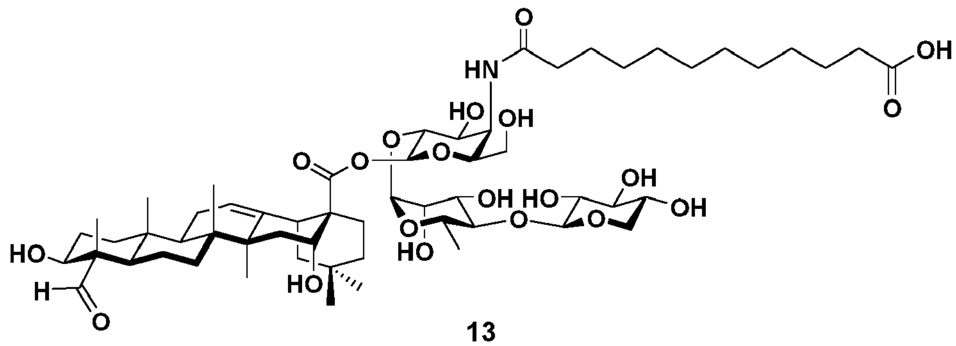

**13**

Data for **13** [CP-III-066] ($C_{59}H_{95}NO_{20}$) was in accordance with that of the previous report by Ghirardello et al. (Chem. Commun. 2020, 56 (5), 719-722).

**HPLC:** $t_R$ = 17.57 min, gradient = 20-100% solv. B over 30 min $\lambda_{max}$ = 200 nm. **LRMS (ESI+-MS):** *m/z* (monoisotopic) calcd for $[M+H]^+$ 1138.65, found 1138.72. **HRMS (MALDI-TOF):** *m/z* (monoisotopic) calcd for $[M+Na]^+$ 1160.6336, found 1160.6293. **$^1$H-NMR** (600 MHz, $CD_3OD$) characteristic resonances: δ 9.31 (s, 1H), 5.38 (d, *J* = 1.9 Hz, 1H), 5.34 (d, *J* = 7.9 Hz, 1H), 5.31 (t, *J* = 3.5, 1H), 4.50-4.47 (m, 2H), 4.34-4.30 (m, 1H), 3.95 - 3.90 (m, 3H), 3.87-3.75 (m, 4H), 3.69 (td, *J* = 6.6, 1.7 Hz, 1H), 3.57 - 3.49 (m, 2H), 3.46 (ddd, *J* = 10.4, 8.9, 5.4 Hz, 1H), 3.41 (dd, *J* = 11.6, 6.9 Hz, 1H), 3.23 - 3.17 (m, 2H), 2.94 (dd, *J* = 14.3, 4.6 Hz, 1H), 1.41 (s, 3H), 1.02 (s, 3H), 1.01 (s, 3H), 0.96 (s, 3H), 0.88 (s, 3H), 0.78 (s, 3H).

**Example 2: Synthesis of compound 25 (under general formula (I))**

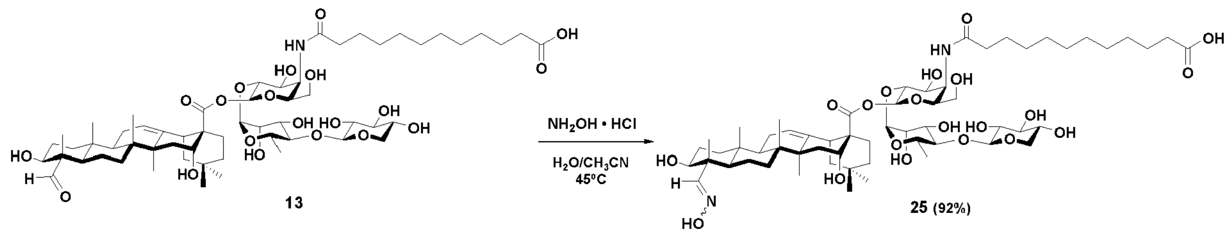


**QA(oxime) adjuvant 25 [RF-IV-045]. A solution of $NH_2OH{\cdot}HCl$ (10.3 mg, 21.4 eq.) and** NaOAc (5.8 mg, 32.3 eq.) in $H_2O/CH_3CN$ (1:3, 2.4 mL) was added to saponin variant **13** (4.4 mg, 1.0 eq) followed by $NH_2OH$ aq. 50% (100 $\mu$L, 490 eq.). The reaction mixture was stirred at 45 °C for 4 h and then directly purified via RP-HPLC (30% acetonitrile in water with 0.05% TFA for 5 min, then 30-64% over 12 min). The main peak was collected and lyophilized, providing the saponin oxime **25** (4.1 mg, 92% yield) as a white foam.

**HPLC:** $t_R$ = 17.25 min, gradient = 20-100% acetonitrile/water over 30 min, $\lambda_{max}$ = 200 nm. **LRMS (ESI+-MS):** *m/z* (monoisotopic) calcd for $[M+2H]^{2+}$ 577.33, found: 577.49; calcd for $[M+H]^+$ 1153.66, found: 1153.65. **$^1$H NMR** (400 MHz, methanol-$d_4$): $\delta$ 7.13 (s, $^1$H, CHNOH), 5.37 (d, *J* = 1.9 Hz, 1H, H-1 Rha), 5.34 (d, *J* = 7.7 Hz, 1H, H-1 N-Gal), 5.30 (t, *J* = 3.6 Hz, 1H, H-12 QA), 4.51 - 4.46 (m, 2H, H-1 Xyl, H-16 QA), 4.34 - 4.31 (m, 1H, H-4 N-Gal), 3.96 - 3.90 (m, 3H, H-3 & H-2 N-Gal, H-2 Rha), 3.90 - 3.75 (m, 3H, H-5a Xyl, H-5 & H-3 Rha), 3.69 (td, *J* = 6.6, 1.7 Hz, 1H, H-5 N-Gal), 3.58 - 3.45 (m, 4H, H-4 Rha, H-3 QA, H-4 Xyl, H-6a N-Gal), 3.45 - 3.38 (m, 1H, H-6b N-Gal), 3.36 - 3.11 (m, 3H, H-3 & H-2 Xyl, H-5b Xyl), 2.94 (dd, *J* = 14.2, 4.5 Hz, $^1$H, H-18 QA), 2.40 - 2.23 (m, 5H, H-19a QA, $CH_2$(a)CONH & $CH_2$(a')$CO_2$H acyl), 2.02 - 1.87 (m, 4H, H-11a,b, H-22a & H-21a QA), 1.87 - 1.74 (m, $^1$H, H-22b QA), 1.74 - 1.55 (m, 9H, H-2a,b, H-9, H-1a & H-15a QA, $CH_{2(b)}CH_2CONH$ & $CH_{2(b')}CH_2CO_2H$ acyl), 1.55-1.40 (m, 3H, H-7a, H-15b & H-6a QA), 1.39 - 1.24 (m, 20H, [1.39 s, 3H, $CH_3$ C-27 QA], H-7b & H-6b QA, $CH_3$ Rha, 6 $\times$ $CH_{2(c)}$ internal acyl), 1.21 - 1.11 (m, $^1$H, H-21 QA), 1.11 - 0.98 (m, 9H, [1.03 s, 3H, $CH_3$ C-24 QA], [1.01 s, 3H, $CH_3$ C-25 QA], H-1b, H-19b & H-5 QA), 0.96 (s, 3H, $CH_3$ C-30 QA), 0.88 (s, 3H, $CH_3$ C-29 QA), 0.77 (s, 3H, $CH_3$ C-26 QA). **$^{13}$C NMR** (101 MHz, methanol-$d_4$): $\delta$ 178.5 (CONH acyl), 177.8 ($CO_2H$ acyl), 177.1 (CO [C-28] QA), 161.0 (CNOH), 144.8 (C-13 QA), 123.3 (C-12 QA), 107.0 (C-1 Xyl), 101.4 (C-1 Rha), 95.5 (C-1 N-Gal), 84.2 (C-4 Rha), 78.3 (C-3 Xyl), 76.5 (C-3 QA), 76.3 (C-5 N-Gal), 76.1 (C-2 Xyl), 74.8 (C-3 & C-2 N-Gal), 74.7 (C-16 QA), 72.2 (C-3 Rha), 71.9 (C-2 Rha), 71.0 (C-4 Xyl), 68.9 (C-5 Rha), 67.3 (C-5 Xyl), 61.7 (C-6 N-Gal), 52.9 (C-5 QA), 52.5 (C-4 N-Gal), 50.0 (C-17 QA), 48.2 (C-9 QA), 48.0 (C-19 QA), 47.4 (C-4 QA), 42.7 (C-14 QA), 42.3 (C-18 QA), 41.1 (C-8 QA), 39.7 (C-1 QA), 37.7 (C-10 QA), 36.8 ($CH_{2(a)}CONH$ acyl), 36.5 (C-15 & C-21 QA), 35.0 ($CH_{2(a')}CO_2H$ acyl QA), 33.8 (C-7 QA), 33.4 ($CH_3$ C-29), 32.0 (C-22 QA), 31.3 (C-20 QA), 30.6, 30.5, 30.4, 30.3, 30.2 (6 $\times$ $CH_{2(c)}$ internal acyl), 27.23 ($CH_{2(b)}CH_2CONH$ acyl), 27.20 ($CH_3$ C-27 QA), 27.0 (C-2 QA), 26.1 ($CH_{2(b')}CO_2H$ acyl), 24.9 ($CH_3$ C-30 QA), 24.5 (C-11 QA), 20.9 (C-6 QA), 18.4 ($CH_3$ Rha), 17.8 ($CH_3$ C-26 QA), 16.6 ($CH_3$ C-25 QA), 12.0 ($CH_3$ C-24 QA).

**Example 3: Synthesis of compounds 1-2 (under general formula (III)) and 3-4 (under general formula (II))**

The synthesis of compounds **1-4** is depicted in the following Scheme.

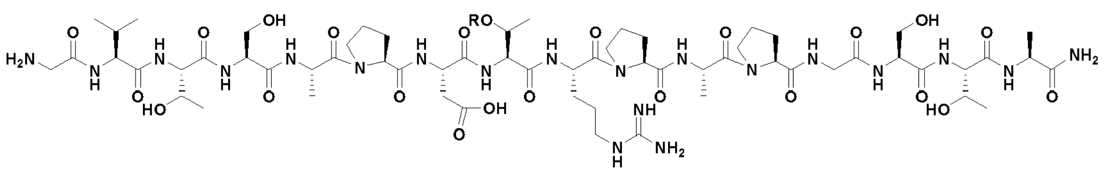

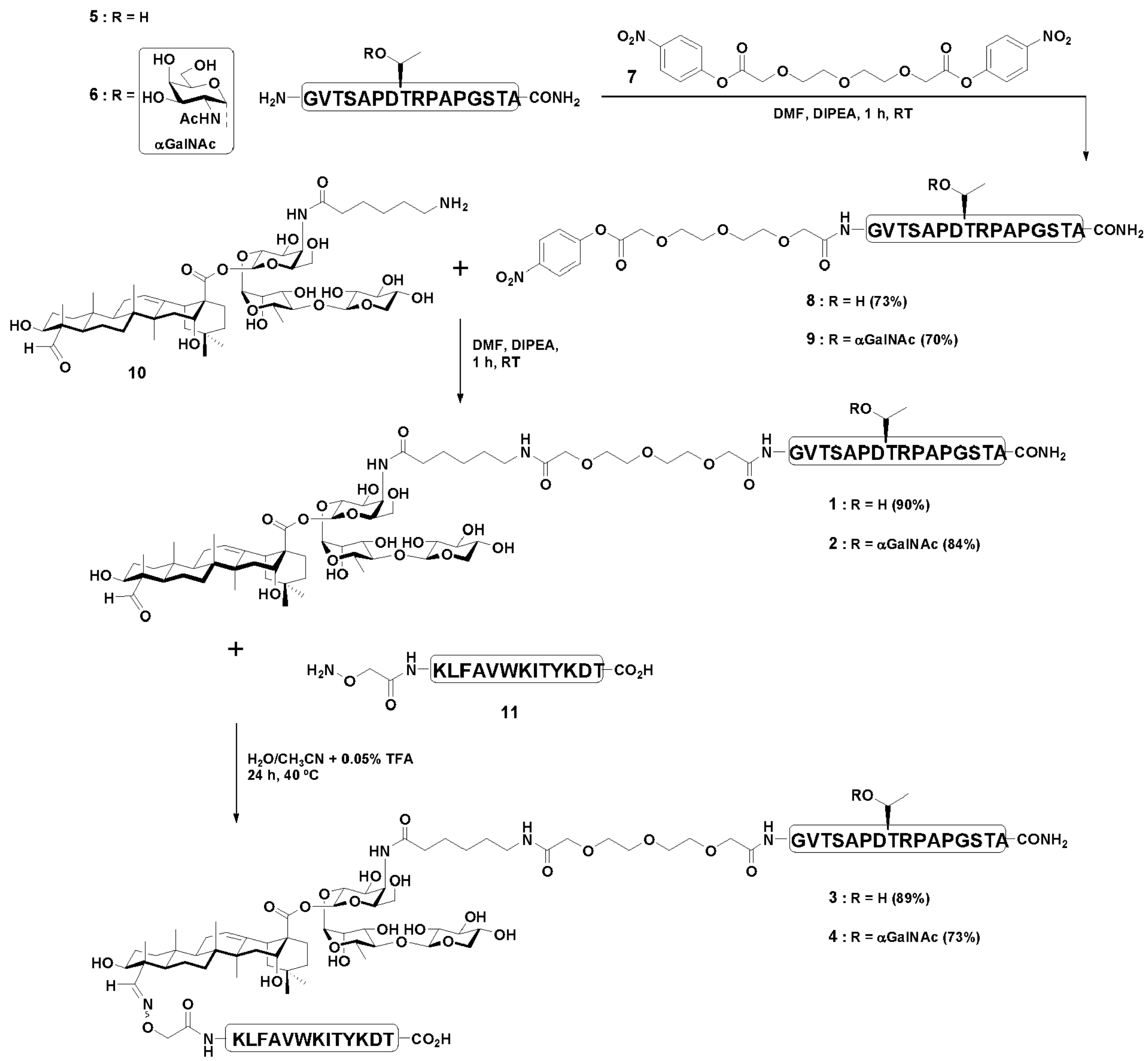


Legend: DMF (dimethylformamide), DIPEA (N,N-diisopropylethylamine), RT (room temperature), TFA (trifluoroacetic acid). Yields after RP-HPLC purification and lyophilisation are reported between brackets.

**3.1. Preparation of MUC1 and TnMUC1 B-cell epitopes + polyethylene glycol (PEG) spacer**

**MUC1 peptide "GVTSAPDTRPAPGSTA" (5)** [CP-I-018, CP-I-052].

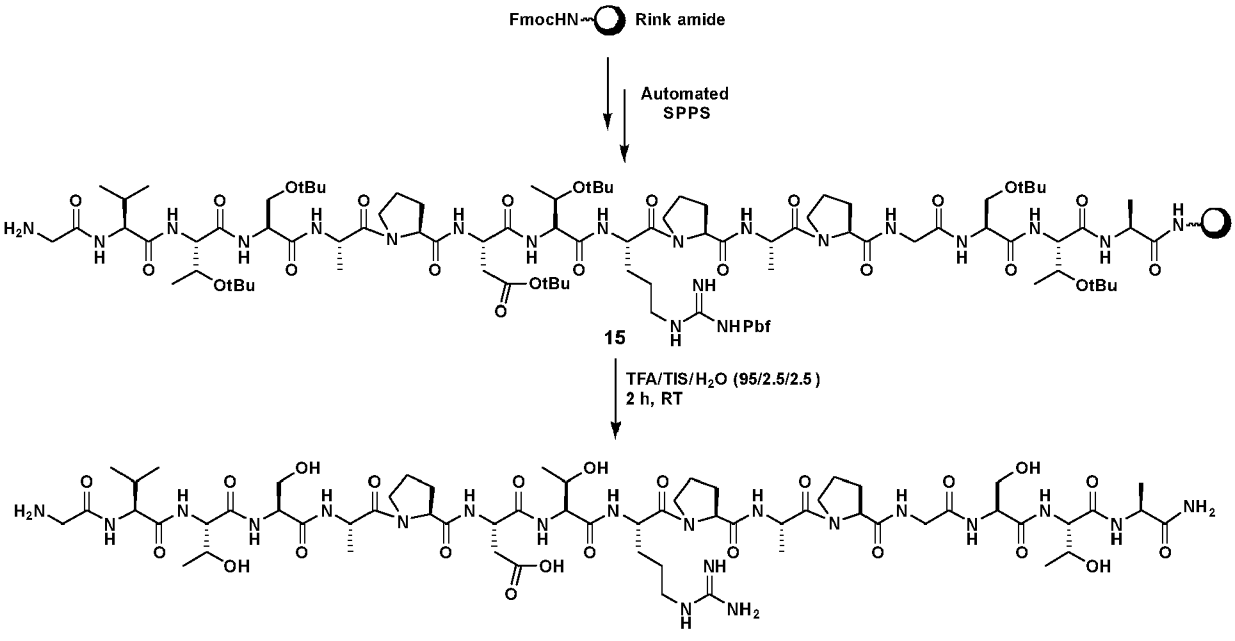


15

**5**
**(44% overall yield)**

Resin-bound sequence **15** was synthesized on 192 mg of Rink Amide MBHA resin (loading = 0.520 mmol/g) via microwave-assisted automated SPPS (CEM Liberty Blue™), using commercially available amino acids (Novabiochem®). Upon sequence completion, the resin was washed with MeOH (5 x 10 mL), transferred in a 50 mL falcon tube and treated with 10 mL of a TFA/TIS/$H_2O$ (95/2.5/2.5) cocktail. After for 2 hours of gentle stirring (rocker platform) at room temperature, the filtrate was added to ice-cold $Et_2O$ to induce precipitation of crude peptide **5.** Following three cycles of (i) centrifugation (3000 rpm at room temperature), (ii) removal of the supernatant, and (iii) resuspension in ice-cold $Et_2O$, the precipitate was dried under vacuum to provide 150 mg of crude **5** as a white powder. Purification of the crude was performed via RP-HPLC (5% solv. B for 5 min, then 5-15% over 25 min.), the collected fractions were lyophilized, affording 65.28 mg of **5** ($C_{61}H_{102}N_{20}O_{23}$) as a white foam in a 44% overall yield.

**HPLC:** $t_R$ = 13.03 min, gradient = 5-40% solv. B over 30 min $\lambda_{max}$ = 200 nm. **LRMS (ESI+-MS):** *m/z* (monoisotopic) calcd for $[M+2H]^{2+}$ 742.38, found 742.41; calcd for $[M+H]^+$ 1483.75, found 1483.61. **HRMS (ESI+-MS):** m/z (monoisotopic) calcd for $[M+Na]^+$ 1505.7324, found 1505.7296.

## Compound 17

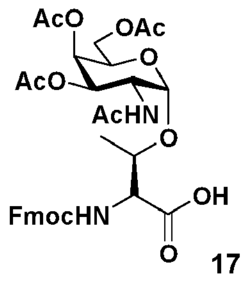

**17**

Data for Fmoc-Thr($Ac_3$-$\alpha$-GalNAc)-$CO_2H$ **17** [CP-I-024, CP-I-054] ($C_{33}H_{38}N_2O_{13}$) was in accordance with that of the previous report by Payne et al. (J. Am. Chem. Soc. 2007, 129 (44), 13527-13536).

**HPLC:** $t_R$ = 18.27 min, gradient = 20-100% solv. B over 30 min $\lambda_{max}$ = 200 nm. **LRMS (ESI+-MS):** *m/z* (monoisotopic) calcd for $[M+H]^+$ 671.25, found 671.32. **HRMS (ESI+-MS):** *m/z* (monoisotopic) calcd for $[M+Na]^+$ 693.2272, found 693.2315. **$^1$H-NMR** (600 MHz, $CDCl_3/CD_3OD$ 1:1 v/v, major rotamer reported) $\delta$ 7.76 (d, *J* = 7.6 Hz, 2H), 7.66 - 7.62 (m, 2H), 7.39 - 7.35 (m, 2H), 7.32 - 7.28 (m, 2H), 5.37 - 5.36 (m, 1H), 5.03 (dd, *J* = 11.5, 3.3 Hz, 1H), 4.94 (d, *J* = 3.9 Hz, 1H), 4.54 - 4.35 (m, 4H), 4.30 - 4.03 (m, 6H), 2.14 (s, 3H), 2.03 (s, 3H), 1.96 (s, 3H), 1.92 (s, 3H), 1.24 (d, *J* = 6.5 Hz, 3H).

**TnMUC1 glycopeptide "GVTSAPDT(aGalNAc)RPAPGSTA" (6)** [CP-I-050, CP-I-056, CP-I-058, CP-I-062,

CP-I-068].

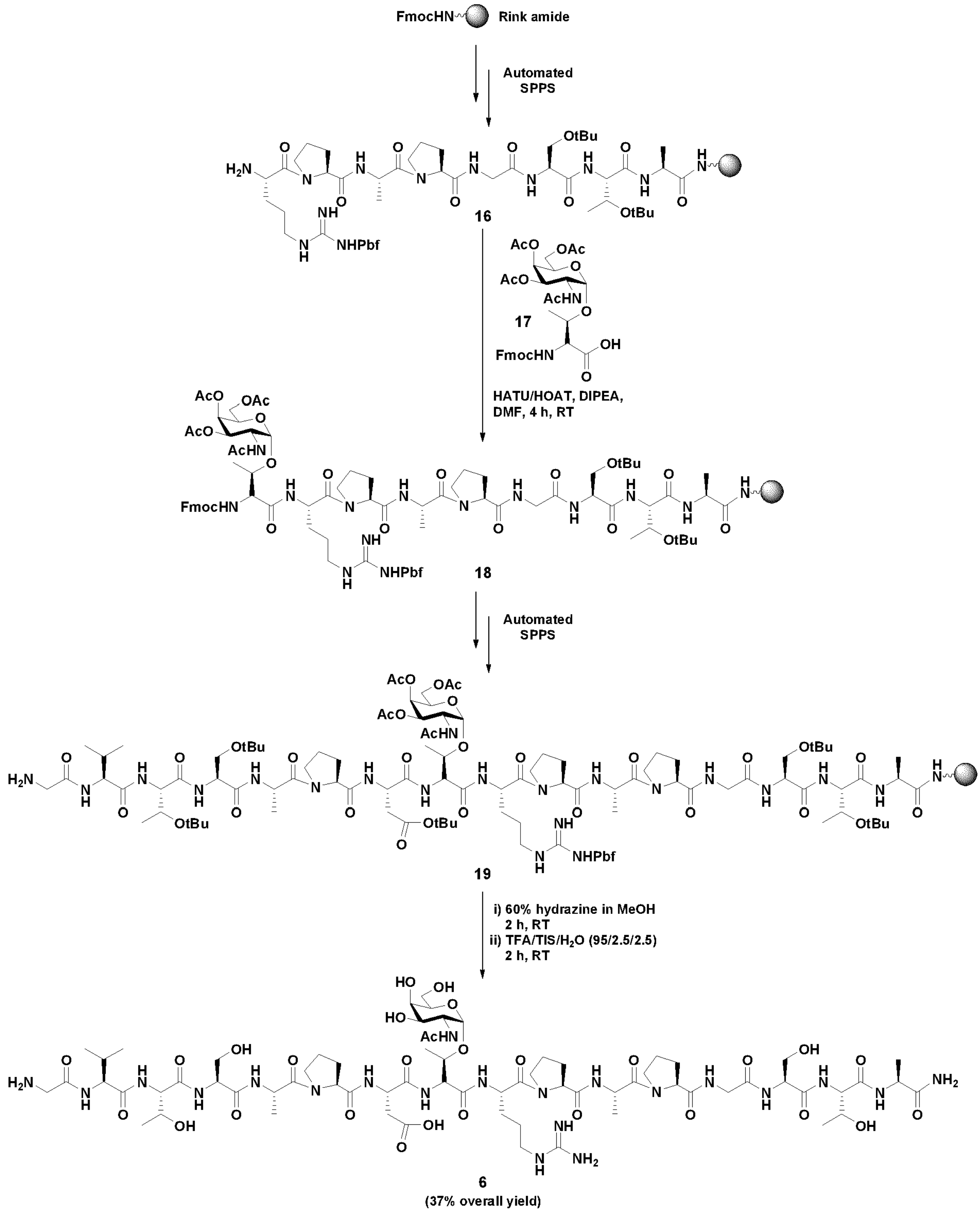


Resin-bound sequence **16** was synthesized on 192 mg of Rink Amide MBHA resin (loading = 0.520 mmol/g) via microwave-assisted automated SPPS (CEM Liberty Blue™), using commercially available amino acids (Novabiochem®). The functionalized resin was then transferred in a solid-phase peptide synthesis vessel provided with a sintered glass filter to manually perform the coupling of glycoamino acid Fmoc-Thr($Ac_3$-α-GalNAc)-$CO_2H$ **17** (Z. Wu, et al. Chem. Commun. 2010, 46, 5773-5774). After two cycles of swelling (10 min.)/solvent filtration, first with $CH_2Cl_2$, then with DMF, sequence **16** was suspended in 2.0 mL of DMF. In a separate flask, a mixture of DIPEA (80 μL, 0.46 mmol, 4.6 eq.), HATU (60.0 mg, 0.158 mmol, 1.6 eq.), HOAt (21.0 mg, 0.154 mmol, 1.5 eq.) and **17** (112.65 mg, 0.168 mmol, 1.7 eq.)

in 5.0 mL DMF was stirred at room temperature for 10 minutes, then added to the reactor. The suspension was stirred using a stream of $N_2$, after 4 hours at room temperature the solvent was removed through the filter and the resin washed with $CH_2Cl_2$(5 x 10 mL) and DMF (5 x 10 mL). Resin-bound sequence **18** was then transferred back to the peptide synthesizer, and fragment **19** was completed via automated SPPS. The resin was transferred in the glass reactor, washed with MeOH (5 x 10 mL), and treated with a 60% hydrazine in MeOH for 2 hours at room temperature under a stream of $N_2$ to remove the acetyl protecting groups. After washing with MeOH (5 x 10 mL) and $CH_2Cl_2$ (5 x 10 mL), the resin was lastly transferred in a 50 mL falcon tube and treated with 10 mL of a TFA/TIS/$H_2O$ (95/2.5/2.5) cocktail. After for 2 hours of gentle stirring (rocker platform) at room temperature, the filtrate was added to ice-cold $Et_2O$ to induce precipitation of crude glycopeptide **6.** Following three cycles of (i) centrifugation (3000 rpm at room temperature), (ii) removal of the supernatant, and (iii) resuspension in ice-cold $Et_2O$, the precipitate was dried under vacuum to provide 170 mg of crude 6 as a white powder. Purification of the crude was performed via RP-HPLC (5% solv. B for 5 min, then 5-15% over 25 min.), the collected fractions were lyophilized, affording 62.41 mg of TnMUC1 glycopeptide **6** ($C_{69}H_{115}N_{21}O_{28}$) as a white foam in a 37% overall yield.

**HPLC:** $t_R$ = 12.74 min, gradient = 5-40% solv. B over 30 min $\lambda_{max}$ = 200 nm. **LRMS (ESI+-MS):** *m*/*z* (monoisotopic) calcd for $[M+3H]^{3+}$ 562.95, found 563.07; calcd for $[M+2H]^{2+}$ 843.92, found 844.15; calcd for $[M+H]^+$ 1686.83, found 1686.30. **HRMS (MALDI-TOF):** *m*/*z* (monoisotopic) calcd for $[M+H]^+$ 1686.8289, found 1686.8384.

## Compound 20

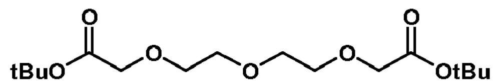

20

Data for 20 [CP-III-030] ($C_{16}H_{30}O_7$) was in accordance with that of the previous report by Wittmann et al. (J. Org. Chem. 1998, 63 (15), 5137-5143).

**HPLC:** $t_R$ = 18.42 min, gradient = 20-100% solv. B over 30 min $\lambda_{max}$ = 200 nm. **LRMS (ESI+-MS):** *m*/*z* (monoisotopic) calcd for $[M+H]^+$ 335.21, found 335.27, calcd for $[M+Na]^+$ 357.19, found 357.31. **HRMS (ESI+-MS):** *m*/*z* (monoisotopic) calcd for $[M+Na]^+$ 357.1925, found 357.1889. **$^1$H-NMR** (600 MHz, $CDCl_3$) $\delta$ 4.01 (s, 4H), 3.75 - 3.66 (m, 8H), 1.46 (s, 18H).

**Para-nitrophenol-activated, diethylenglycol (DEG)-derived homobifunctional linker (7)** [CP-III-032].

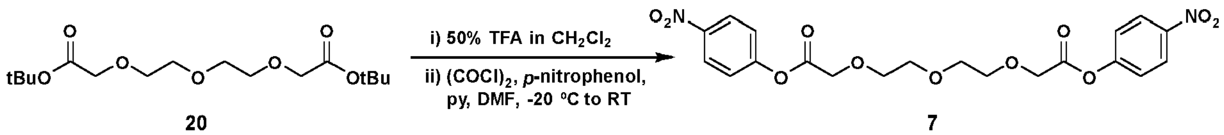


20

7 (59%)

A solution of 3,6,9-trioxaundecane-1,11-dioic acid di-*tert*-butyl ester **20** (1.038 g, 3.07 mmol, 1.0 eq.) in a TFA/$CH_2Cl_2$ (1:1, 3.0 mL) mixture was stirred at room temperature for 4 hours. The solvent mixture was removed under high vacuum trough a Schlenk line, and co-evaporated with dry toluene (3 x 1 mL). The resulting di-acid was solubilized in dry $CH_2Cl_2$ (7.0 mL), and the resulting solution cooled to 0 °C. Oxalyl chloride (1.6 mL, 18.63 mmol, 6.1 eq.) and a catalytic amount of dry DMF (60 $\mu$L) were added, and the mixture was stirred at 0 °C for 10 minutes before allowing it to passively warm to room temperature during 1 hour. In order to remove volatiles, the reaction mixture was concentrated under reduced pressure via the Schlenk line, then dry $CH_2Cl_2$ (7.0 mL) was added. This operation was repeated two times, then the reaction mixture was cooled again to 0 °C. In a separated flask, azeotropically dried (dry toluene: 3 x 1 mL) para-nitrohpenol (940 mg, 6.76 mmol, 2.2 eq.) was solubilized in a $CH_2Cl_2$/THF (1:1, 14 mL) mixture, and the resulting solution was cooled to -20 °C. To this solution, the mixture containing the activated di-acid was added via cannula, followed by dry pyridine (550 $\mu$L, 6.94 mmol, 2.3 eq.). The reaction mixture was stirred at -20 °C for 15 minutes and then allowed to passively warm to room temperature. After 2 hours, the reaction mixture was diluted with $CH_2Cl_2$ (120 mL) and washed with 1% aq. AcOH (50 mL). The organic phase was dried over $MgSO_4$, filtered, and the solvent removed under vacuum to afford 1.54 g of brown solid as crude. The residue was purified via silica gel flash chromatography (eluent: 0 to 20% AcOEt in toluene), affording 836 mg of linker **7** ($C_{20}H_{20}N2O_{11}$) as white solid in a 59% yield over two steps.

**HPLC:** $t_R$ = 23.56 min, gradient = 20-100% solv. B over 30 min $\lambda_{max}$ = 200 nm. **LRMS (ESI$^+$-MS):** *m*/*z* (monoisotopic) calcd for $[M+H]^+$ 465.11, found 465.12. **HRMS ($ESI^+$-MS):** *m/z* (monoisotopic) calcd for $[M+Na]^+$ 487.0965, found 487.0955. **TLC**: $R_f$ = 0.32 (toluene/AcOEt, 7:3, v/v). **$^1$H-NMR** (600 MHz, $CDCl_3$) δ 8.28 - 8.25 (m, 4H, Ph), 7.33 - 7.29 (m, 4H, Ph), 4.45 (s, 4H, $COCH_2O$) 3.86 - 3.83 (m, 4H, O-C$H_2$$CH_2$-O), 3.78 - 3.76 (m, 4H, O-$CH_2$C$H_2$-O). **$^{13}$C NMR** (151 MHz, $CDCl_3$): δ = 168.26 (CO), 154.98 (Ph), 145.72 (Ph), 125.41 (Ph), 122.36 (Ph), 71.38 (O-C$H_2$$CH_2$-O), 70.87(O-$CH_2$C$H_2$-O), 68.72 (COC$H_2$).

**Para-nitrophenol-activated, DEG-containing, MUC1- and TnMUC1 sequences (8 and 9)** [CP-I-118, CP-III-016, CP-III-046, CP-III-090, CP-III-092].

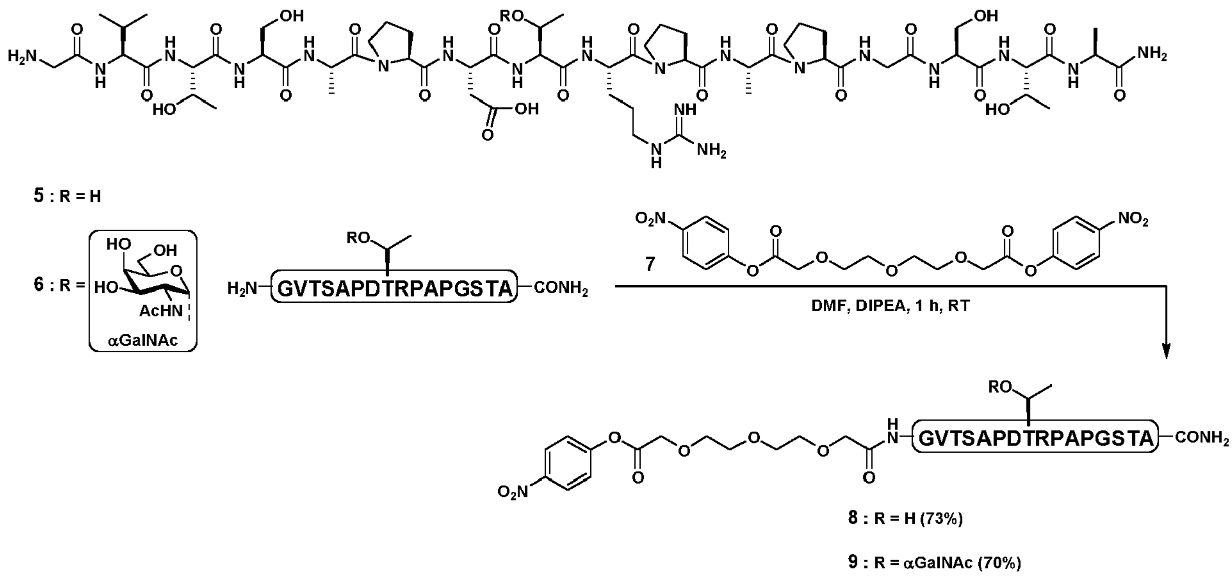


To a stirred solution of **5** or **6** (1.0 eq.) in dry DMF containing DIPEA (2.5 eq.), a solution of **7** (5.0 eq.) in dry DMF was added (final conc. = 32 mM with respect to **7**). The reaction mixture readily tuned to yellow and was kept under stirring at room temperature. After 1 hour, the reaction was quenched by slowly adding a 0.05% TFA solution in $H_2O/CH_3CN$ (1:1) until disappearance of the yellow coloration, then directly purified via RP-HPLC (15% solv. B for 5 min, then 15-50% over 25 min.). The collected fractions were lyophilized, affording the title compounds as a white foam.

Data for **8** ($C_{75}H_{117}N_{21}O_{31}$): prepared on a 9.62 μmol scale, yield: 12.71 mg, 73%. **HPLC:** $t_R$ = 17.10 min, gradient = 5-70% solv. B over 30 min $\lambda_{max}$ = 200 nm. **LRMS ($ESI^+$-MS):** *m/z* (monoisotopic) calcd for $[M+3H]^{3+}$ 603.62, found 603.52; calcd for $[M+2H]^{2+}$ 904.92, found 904.68; calcd for $[M+H]^+$ 1808.83, found 1808.49. **HRMS (MALDI-TOF):** *m/z* (monoisotopic) calcd for $[M+H]^+$ 1808.8293, found 1808.8181.

Data for **9** ($C_{83}H_{130}N_{22}O_{36}$): prepared on a 9.47 μmol scale, yield: 13.40 mg, 70%. **HPLC:** $t_R$ = 16.73 min, gradient = 5-70% solv. B over 30 min $\lambda_{max}$ = 200 nm. **LRMS ($ESI^+$-MS):** *m/z* (monoisotopic) calcd for $[M+3H]^{3+}$ 671.31, found 671.38; calcd for $[M+2H]^{2+}$ 1006.46, found 1006.47; calcd for $[2M+3H]^{3+}$ 1341.61, found 1341.51; calcd for $[3M+4H]^{4+}$ 1509.18, found 1508.87; calcd for $[M+H]^+$ 2011.91, found 2012.21. **HRMS ($ESI^+$-MS):** *m/z* (monoisotopic) calcd for $[M+H]^+$ 2011.9087, found 2011.9045.

### 3.2. Preparation of di-component conjugates QA-MUC1 (1) and QA-TnMUC1 (2)

**Prior Art Compound 10** [CP-I-100]

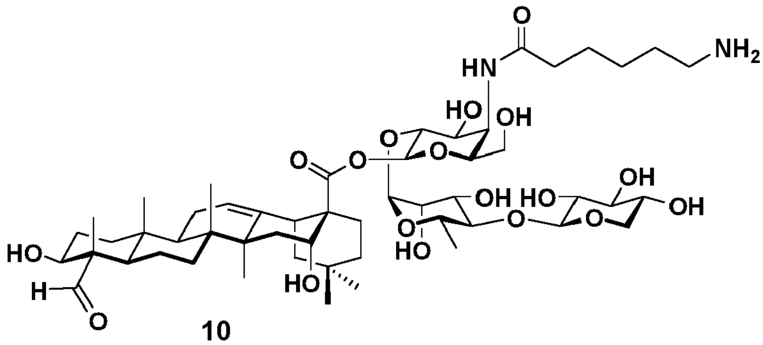

10

Data for **10** [CP-I-100] ($C_{53}H_{86}N_2O_{18}$) was in accordance with that of the previous report by Fernández-Tejada et al. (Nat. Chem. 2014, 6 (7), 635-643).

**HPLC:** $t_R$ = 12.64 min, gradient = 20-100% solv. B over 30 min $\lambda_{max}$ = 200 nm. **LRMS (ESI$^+$-MS):** *m/z* (monoisotopic) calcd for $[M+H]^+$ 1039.60, found 1039.68. **HRMS (MALDI-**TOF): *m/z* (monoisotopic) calcd for $[M+Na]^+$ 1061.5764, found 1061.5720. **$^1$H-NMR** (600 MHz, $CD_3OD$) characteristic resonances: δ 9.31 (s, 1H), 5.42 (d, *J* = 1.8 Hz, 1H), 5.34 (t, *J* = 3.5 Hz, 1H), 5.31 (d, *J* = 3.6 Hz, 1H), 4.51 - 4.48 (m, 2H), 4.37 (dd, *J* = 4.7, 1.7 Hz, 1H), 3.96 (dd, *J* = 9.5, 4.7 Hz, 1H), 3.91 - 3.80 (m, 5H), 3.79 - 3.76 (m, 1H), 3.72 (td, *J* = 6.5, 1.8 Hz, 1H), 3.59 - 3.51 (m, 2H), 3.49 - 3.41 (m, 2H), 3.24 - 3.17 (m, 2H), 2.94 - 2.91 (m, 3H), 2.43 - 2.27 (m, 3H), 2.02 - 1.90 (m, 4H), 1.41 (s, 3H), 1.34 (d, *J* = 6.1 Hz, 3H), 1.08 - 1.04 (m, 1H), 1.02 (s, 3H), 1.01 (s, 3H), 0.95 (s, 3H), 0.88 (s, 3H), 0.77 (s, 3H).

**MUC1- and TnMUC1-containing, di-component synthetic vaccines (1 and 2)** [CP-I-128, CP-III-052, CP-III-098, CP-III-124, CP-III-128].

To a vial containing amino-terminating saponin **10** (1.0 eq.) and para-nitrophenol-activated (glyco)peptide **8** or **9** (1.5 eq.), a solution of dry DMF containing DIPEA (3.0 eq.) was added (final conc. = 2.5 mM, with respect to **10**). The reaction mixture rapidly turned to a yellow solution, which was stirred at room temperature for 1 hour. The reaction was quenched by slowly adding a 0.05% TFA solution in $H_2O/CH_3CN$ (1:1) until disappearance of the yellow coloration, then directly purified via RP-HPLC (20% solv. B for 5 min, then 20-100% over 25 min.). The collected fractions were lyophilized, affording the title compounds as a white foam.

Data for **1** ($C_{122}H_{198}N_{22}O_{46}$): prepared on a 3.18 μmol scale, yield: 7.72 mg, 90%. **HPLC:** $t_R$ = 11.98 min, gradient = 20-100% solv. B over 30 min $\lambda_{max}$ = 200 nm. **LRMS (ESI$^+$-MS):** *m/z* (monoisotopic) calcd for $[M+3H]^{3+}$ 903.47, found 903.45; calcd for $[M+2H]^{2+}$ 1354.70, found 1355.00; calcd for $[2M+3H]^{3+}$ 1805.93, found 1805.96; calcd for $[3M+4H]^{4+}$ 2031.55, found 2031.06; calcd for $[4M+5H]^{5+}$ 2166.91, found 2166.90; calcd for $[5M+6H]^{6+}$ 2257.16, found 2257.63; calcd for $[M+H]^+$ 2708.39, found 2708.56. **HRMS (MALDI-TOF):** *m/z* (monoisotopic) calcd for $[M+H]^+$ 2708.3900, found 2708.3990.

Data for **2** ($C_{130}H_{211}N_{23}O_{51}$): prepared on a 3.59 μmol scale, yield: 8.75 mg, 84%. HPLC: $t_R$ = 12.00 min, gradient = 20-100% solv. B over 30 min $\lambda_{max}$ = 200 nm. LRMS **(ESI$^+$-MS):** *m/z* (monoisotopic) calcd for $[M+3H]^{3+}$ 971.16, found 971.02; calcd for $[3M+5H]^{5+}$ 1747.29, found 1746.49; calcd for $[2M+3H]^{3+}$ 1941.31, found 1940.99; calcd for $[3M+4H]^{4+}$ 2183.85, found 2184.16; calcd for $[4M+5H]^{5+}$ 2329.38, found 2329.22; calcd for $[5M+6H]^{6+}$ 2426.39, found 2426.70; calcd for $[M+H]^+$ 2911.47, found 2911.94. **HRMS (MALDI-TOF):** *m/z* (monoisotopic) calcd for $[M+H]^+$ 2911.4703, found 2911.4693.

### 3.3. Preparation of tri-component conjugates QA(PV)-MUC1 (3) and QA(PV)-TnMUC1 (4)

**Aminooxy-functionalized $PV_{103-115}$ peptide** (11) [CP-III-034].

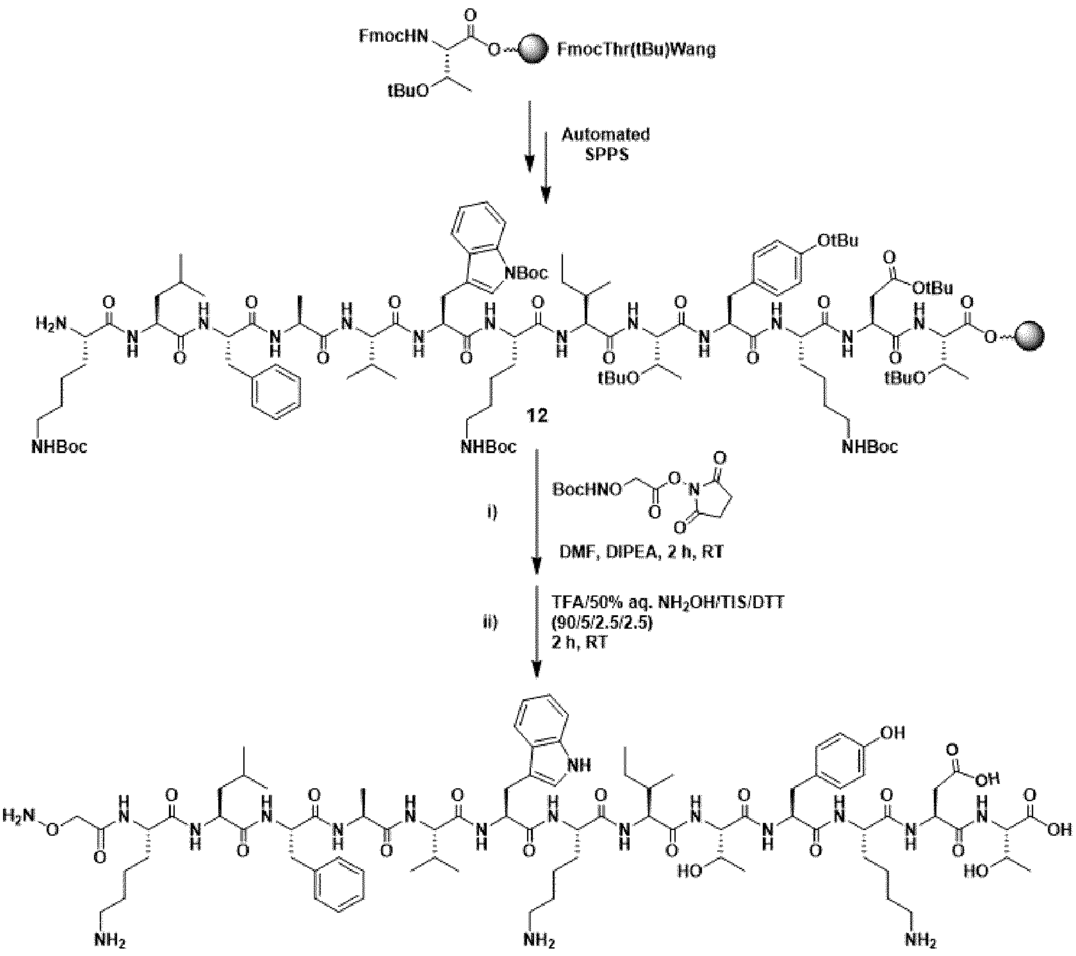


11
(36% overall yield)

Resin-bound sequence **12** was synthesized on 94 mg of FmocThr(*t*Bu)Wang resin (loading = 0.530 mmol/g) via microwave-assisted automated SPPS (CEM Liberty Blue™), using commercially available amino acids (Novabiochem®). The functionalized resin was then transferred in a solid-phase peptide synthesis vessel provided with a sintered glass filter, washed with MeOH (5 x 5 mL) and two cycles of swelling (10 min.)/solvent filtration, first with $CH_2Cl_2$, then with DMF, were performed. Resin **12** was suspended in 2.0 mL of DMF, then DIPEA (8.75 μL, 0.05 mmol, 1.0 eq.) and Boc-aminooxyacetic acid *N*-hydroxysuccinimide ester (57.65 mg, 0.20 mmol, 4.0 eq.) were added, and the suspension was stirred using a stream of $N_2$. After 2 hours at room temperature, the resin was washed with DMF (5 x 5 mL), MeOH (5 x 5 mL) and $CH_2Cl_2$ (5 x 5 mL), and then transferred in a 50 mL falcon tube and treated with 5 mL of a TFA/50% aq. $NH_2OH$/TIS/DTT $H_2O$ (90/5/2.5/2.5) cocktail. After for 2 hours of gentle stirring (rocker platform) at room temperature, the filtrate was added to ice-cold $Et_2O$ to induce precipitation of crude peptide **11.** Following three cycles of (i) centrifugation (3000 rpm at room temperature), (ii) removal of the supernatant, and (iii) resuspension in ice-cold $Et_2O$, the precipitate was dried under vacuum, re-suspended in 10 mL of 1% aq. AcOH and lyophilized to provide 103 mg of crude **11** as a white powder. Purification of the crude was performed via RP-HPLC (5% solv. B for 5 min, then 5-65% over 25 min.), the collected fractions were lyophilized, affording 30.35 mg of **11** ($C_{81}H_{124}N_{18}O_{21}$) as a white foam in a 36% overall yield.

**HPLC:** $t_R$ = 10.49 min, gradient = 20-100% solv. B over 30 min $\lambda_{max}$ = 200 nm. **LRMS (ESI$^+$-MS):** *m/z* (monoisotopic) calcd for $[M+3H]^{3+}$ 562.65, found 562.78; calcd for $[M+2H]^{2+}$ 843.47, found 843.57; calcd for $[M+H]^+$ 1685.93, found 1685.94. **HRMS (ESI$^+$-MS):** *m/z* (monoisotopic) calcd for $[M+H]^+$ 1685.9266, found 1685.9308.

**MUC1- and TnMUC1-containing, tri-component synthetic vaccines including the $PV_{103-115}$ peptide sequence (3 and 4)** [CP-III-040, CP-III-058, CP-III-112, CP-III-126].

A stirred solution of **1** or **2** (1.0 eq, 1.5 mM) and aminooxy-functionalized $PV_{103-115}$ peptide **11** (2.0 eq.) in a $H_2O/CH_3CN$ (2:1) mixture containing 0.05% TFA was heated at 40 °C for 24 h. The reaction mixture was directly purified via RP-HPLC (30% solv. B for 5 min, then 30-80% over 25 min.). The collected fractions were lyophilized, affording the title compounds as a white foam.

Data for **3** ($C_{203}H_{320}N_{40}O_{66}$): prepared on a 1.14 μmol scale, yield: 4.46 mg, 89%. **HPLC:** $t_R$ = 12.90 min,

gradient = 20-100% solv. B over 30 min $\lambda_{max}$ = 200 nm. **LRMS (ESI$^+$-MS):** *m/z* (monoisotopic) calcd for $[M+6H]^{6+}$ 730.06, found 730.32; calcd for $[M+5H]^{5+}$ 875.87, found 876.04; calcd for $[M+4H]^{4+}$ 1094.58, found 1094.78; calcd for $[M+3H]^{3+}$ 1459.10, found 1459.34; calcd for $[2M+5H]^{5+}$ 1750.72, found 1750.70; calcd for $[M+2H]^{2+}$ 2188.15, found 2188.30. **HRMS (ESI$^+$-MS):** *m/z* (monoisotopic) calcd for $[M+H]^+$ 4375.2982, found 4375.2856.

Data for **4** ($C_{211}H_{333}N_{41}O_{71}$): prepared on a 0.86 μmol scale, yield: 2.87 mg, 73%. **HPLC:** $t_R$ = 12.59 min, gradient = 20-100% solv. B over 30 min $\lambda_{max}$ = 200 nm. **LRMS (ESI$^+$-MS):** *m/z* (monoisotopic) calcd for $[M+6H]^{6+}$ 763.90, found 764.32; calcd for $[M+5H]^{5+}$ 916.48, found 916.86; calcd for $[M+4H]^{4+}$ 1145.35, found 1145.46; calcd for $[M+3H]^{3+}$ 1526.80, found 1527.14; calcd for $[2M+5H]^{5+}$ 1831.96, found 1832.14; $[M+2H]^{2+}$ 2289.69, found 2289.18. **HRMS (MALDI-TOF):** *m/z* (monoisotopic) calcd for $[M+H]^+$ 4578.3785, found 4578.3799.

**Example 4: Synthesis of synthetic saponin-$PV_{103-115}$ conjugate (14) (compound under formula IV)** [CP-III-136]

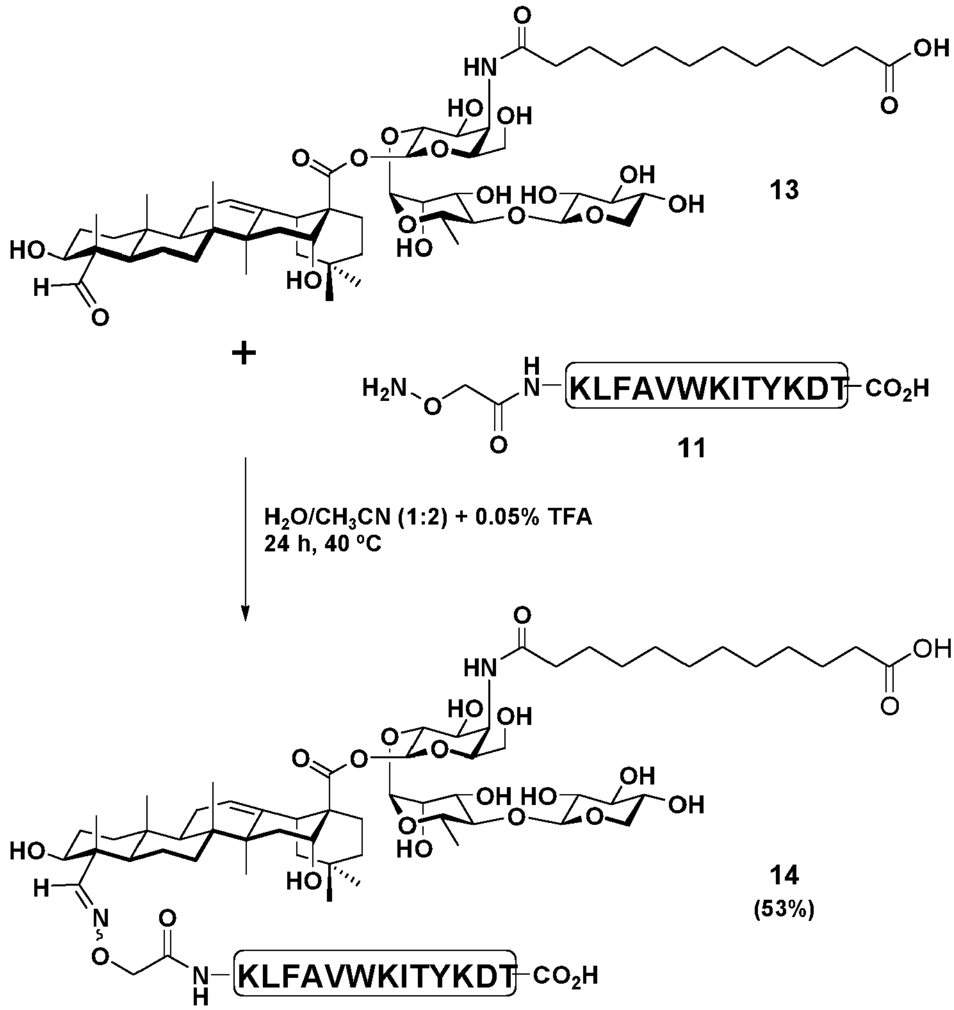


A stirred solution of **13** (2.07 mg, 1.82 μmol, 1.0 eq.) and aminooxy-functionalized $PV_{103-115}$ peptide **11** (6.48 mg, 3.84 μmol, 2.1 eq.) in a $H_2O/CH_3CN$ (1:2) mixture containing 0.05% TFA (final conc. = 1.5 mM with respect to **13**) was heated at 40 °C for 24 h. The reaction mixture was directly purified via RP-HPLC (20% solv. B for 5 min, then 20-100% over 25 min.). The collected fractions were lyophilized, affording 2.72 mg of **14** ($C_{140}H_{217}N_{19}O_{40}$) as a white foam in a 53% yield.

**HPLC:** $t_R$ = 15.80 min, gradient = 20-100% solv. B over 30 min $\lambda_{max}$ = 200 nm. **LRMS (ESI+-MS):** *m/z* (monoisotopic) calcd for $[M+3H]^{3+}$ 935.86, found 935.64; calcd for $[M+2H]^{2+}$ 1403.28, found 1403.58; calcd for $[2M+3H]^{3+}$ 1870.71, found 1871.08; calcd for $[3M+4H]^{4+}$ 2104.42, found 2105.25; calcd for $[4M+5H]^{5+}$ 2244.65, found 2245.01; calcd for $[M+H]^+$ 2805.56, found 2805.39; (most intense peak of isotopic cluster) calcd for $[M+H]^+$ 2806.56, found 2806.55. **HRMS (MALDI-TOF):** *m/z* (monoisotopic) calcd for $[M+Na]^+$ 2827.5419, found 2827.5482.

**Example 5: PV$_{103\text{-}115}$ peptide (22)** [CP-III-042]

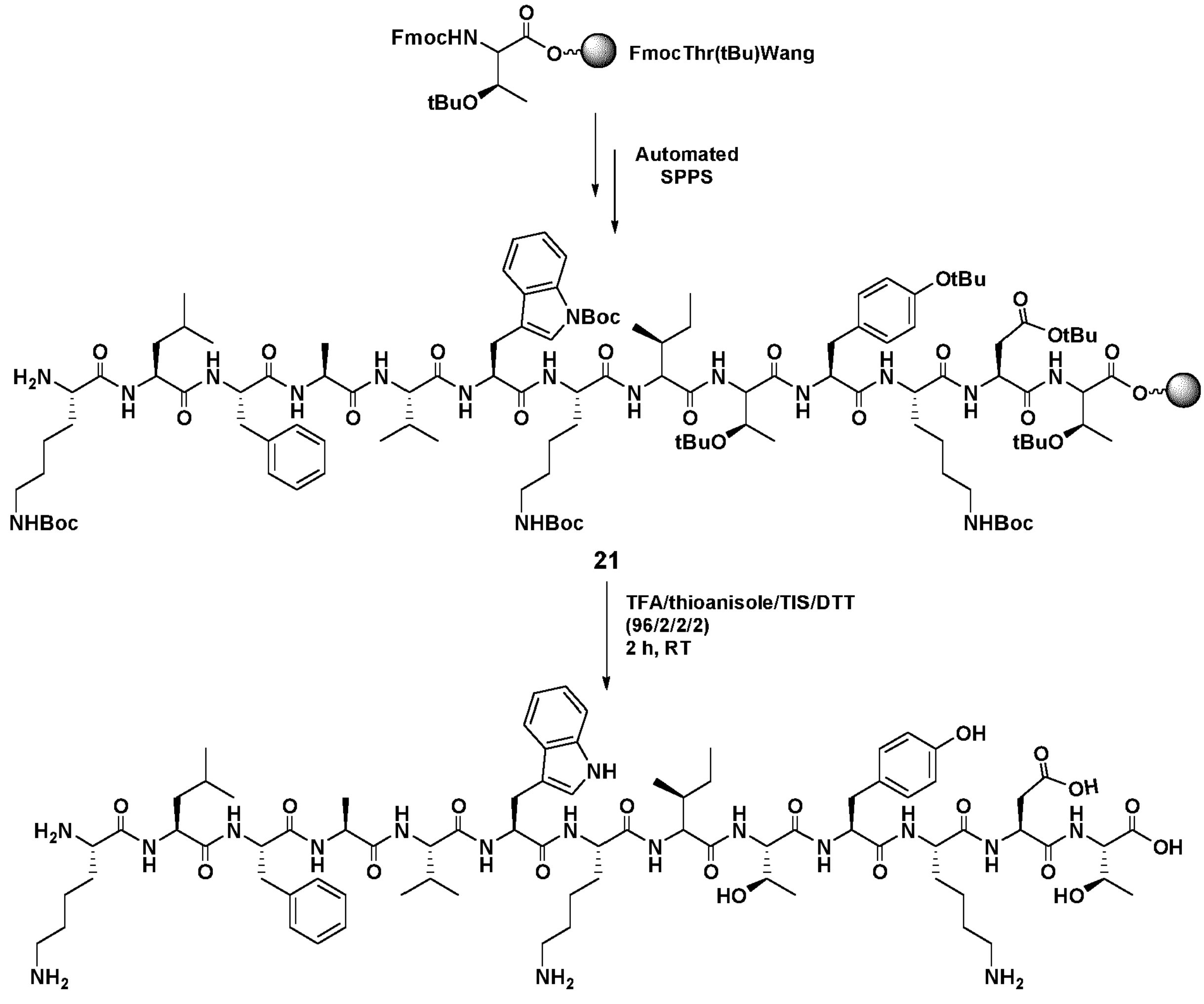


**22**
**(55% overall yield)**

Resin-bound sequence **21** was synthesized on 94 mg of FmocThr(*t*Bu)Wang resin (loading = 0.530 mmol/g) via microwave-assisted automated SPPS (CEM Liberty Blue™), using commercially available amino acids (Novabiochem®). Upon sequence completion, the resin was washed with MeOH (5 x 5 mL), transferred in a 50 mL falcon tube and treated with 5 mL of a TFA/thioanisole/TIS/DTT (96/2/2/2) cocktail. After for 2 hours of gentle stirring (rocker platform) at room temperature, the filtrate was added to ice-cold $Et_2O$ to induce precipitation of crude peptide **22.** Following three cycles of (i) centrifugation (3000 rpm at room temperature), (ii) removal of the supernatant, and (iii) resuspension in ice-cold $Et_2O$, the precipitate was dried under vacuum, re-suspended in 10 mL of 1% aq. AcOH and lyophilized to provide 101 mg of crude **22** as a white powder. Purification of the crude was performed via RP-HPLC (15% solv. B for 5 min, then 15-85% over 25 min.), the collected fractions were lyophilized, affording 44.35 mg of **22** ($C_{79}H_{121}N_{17}O_{19}$) as a white foam in a 55% overall yield.

**HPLC:** $t_R$ = 9.61 min, gradient = 20-100% solv. B over 30 min $\lambda_{max}$ = 200 nm. **LRMS (ESI$^+$-MS):** *m/z* (monoisotopic) calcd for $[M+3H]^{3+}$ 538.31, found 538.42; calcd for $[M+2H]^{2+}$ 806.96, found 806.81; calcd for $[2M+3H]^{3+}$ 1075.61, found 1075.93; calcd for $[M+H]^+$ 1612.91, found 1612.78. **HRMS (MALDI-TOF):** *m/z* (monoisotopic) calcd for $[M+H]^+$ 1612.9103, found 1612.9175.

**Example 6: MUC1- and TnMUC1-functionalized BSA (23 and 24)** [CP-III-018-020-068]

Figure 3 shows the preparation of BSA conjugates **23** and **24.** To a stirred solution of BSA (Fisher Scientific,

CAS: 9048-46-8, 1.0 eq.) in PBS (10 mM, pH 7.4) at a 37.5 μM concentration, a solution of **8** or **9** (30 eq.) in DMF (200 μL) was added. The reaction mixture was stirred at room temperature and progressively turned to yellow. After 24 hours, the reaction was quenched by slowly adding a 0.05% TFA solution in $H_2O$ until disappearance of the yellow coloration, then directly purified via RP-HPLC (5% solv. B for 5 min, then 5-70% over 25 min.). The collected fractions were lyophilized, affording the title compounds as a white foam.

Data for **23:** prepared on a 0.030 μmol scale of BSA, yield: 1.85 mg, n ≈ 6.3.

Data for **24:** prepared on a 0.045 μmol scale of BSA, yield: 2.86 mg, n ≈ 3.0.

"n" was calculated as follows: $n = \frac{\text{MALDI} - \text{BSA}}{\text{EPITOPE}}$ ; where "MALDI" is the most intense peak observed via MALDI-MS, "BSA" corresponds to the molecular weight of the native protein (66463 Da), and "EPITOPE" corresponds to mass increase of the conjugate for 1 unit of either MUC1 (1669 Da) or TnMUC1 (1872 Da) epitopes.

**Example 7: Synthesis of compounds 26 and 28 (under general formula (III)) and 27 and 29 (under general formula (II))**

Di- and tri-component saponin conjugates based on tumor-associated carbohydrate antigens (Tn and Gb3) were synthesized.

### 7.1. Preparation of Tn antigen + polyethylene glycol ($PEG_3$) spacer

The following scheme shows the synthesis of the Tn antigen moiety functionalized with a PEG-based, carboxy-terminated linker.

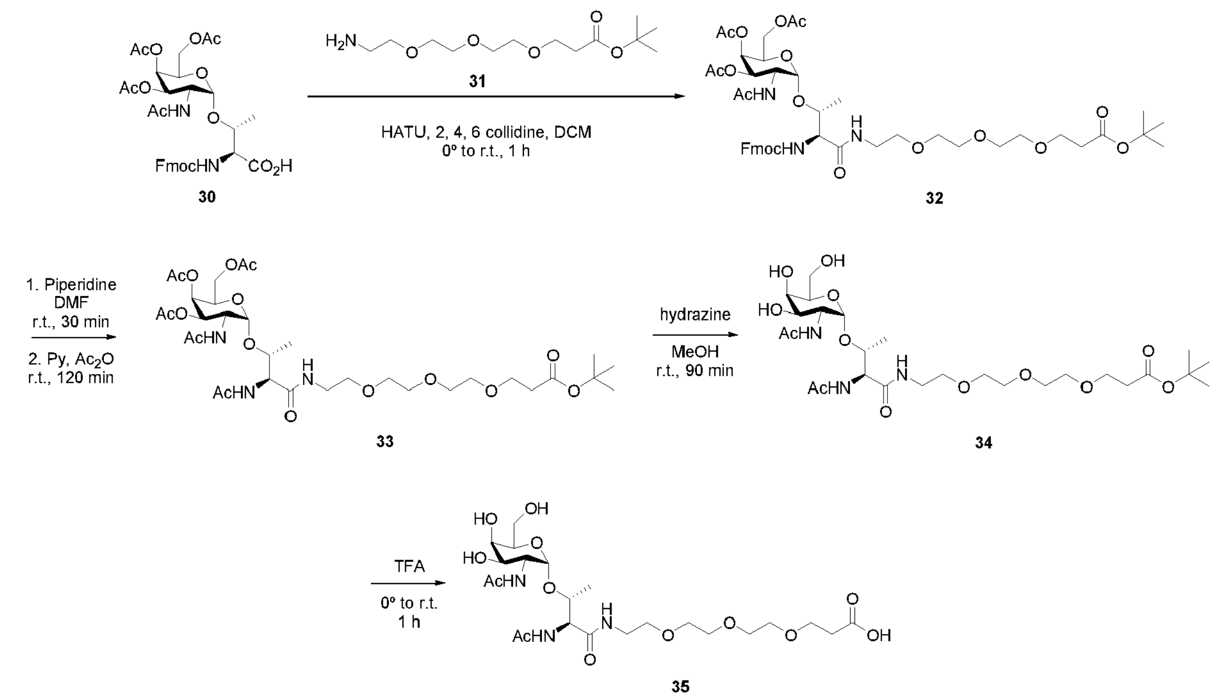


**Protected Fmoc-Tn(Thr)-$PEG_3$ *tert*-butyl ester (32).**

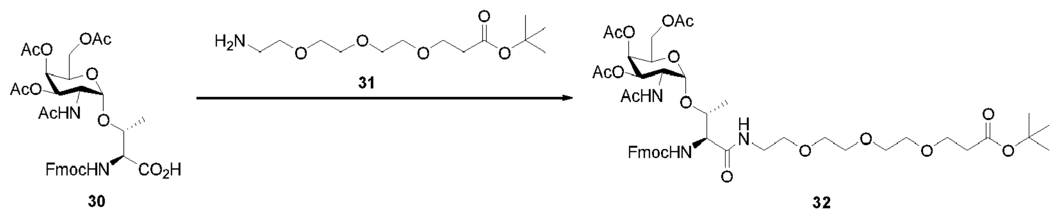

To a solution of Tn-Thr acid **30** (T. Buskas, et al. Angew. Chem. Int. Ed. 2005, 44, 5985-5988; Z. Wu, et al. Chem. Commun. 2010, 46, 5773-5774) (47 mg, 70.2 μmol) in DCM (0.9 mL), HATU (29.4 mg, 77.3 μmol, 1.1 equiv) and the amino-$TEG_3$-*t*-butyl ester linker **31** (22.07 μL, 84.3 μmol, 1.2 equiv) were added. The mixture was cold to 0°C and 2,4,6-collidine (10.2 μL, 77.3 μmol, 1.1 equiv) was added. After stirring for 1 h at 0°C, the reaction was allowed to warm to r.t. and 1 h later TLC (95:5 DCM/methanol) confirmed consumption of starting material. The resulting crude was concentrated and dried under vacuum, and the residue was purified by silica gel column chromatography (DCM to 97:3 DCM/methanol) affording the Tn(Thr)-$TEG_3$ *tert*-butyl spacer **32** (53.9 mg, 82% yield) as a glassy solid.

**TLC:** $R_f$ 0.40 (DCM); **$^1$H NMR** (400 MHz, $CDCl_3$): $\delta$ 7.74 (d, J = 7.5 Hz, 2H, Ar-H Fmoc), 7.65-7.59 (m, 3H, Ar-H Fmoc, NH, NHCO [Tn-TEG]), 7.42-7.34 (m, 2H, Ar-H Fmoc), 7.33-7.26 (m, 2H, Ar-H Fmoc), 6.77 (dd, J = 9.7, 3.0 Hz, 1H, NH, NHAc), 6.28 (d, J = 8.8 Hz, 1H, NH, NHFmoc), 5.35 (d, J = 2.2 Hz, 1H, H-4), 5.05 (dd, J = 11.3, 3.2 Hz, 1H, H-3), 4.86 (d, J = 3.6 Hz, 1H, H-1), 4.53-4.44 (m, 2H, H-2, CH*Ha* Fmoc), 4.41-4.33 (m, 1H, CH*Hb* Fmoc), 4.30-4.12 (m, 4H, CH Fmoc, C*H*α NHFmoc, H-5, C*H*α $CH_3$ [Thr]), 4.11-3.98 (m, 2H, H-6a,b), 3.65 (t, J = 6.4 Hz, 2H, $CH_{2(y)}$ TEG), 3.61-3.52 (m, 8H, 4 × $CH_{2(z)}$ TEG), 3.52 - 3.42 (m, 2H, $CH_{2(y')}$ TEG), 3.42-3.36 (m, 2H, , $CH_{2(x')}$ TEG), 2.45 (t, J = 6.4 Hz, 2H, $CH_{2(x)}$ TEG), 2.13 (s, 3H, $CH_3$ [AcO C-4]), 1.99 (s, 3H, $CH_3$ [AcO C-6]), 1.97 (s, 3H, $CH_3$ [NHAc C-2 Gal Tn]), 1.96 (s, 3H, $CH_3$ [AcO C-3]), 1.40 (s, 9H, 3 × ($CH_3$) [$^t$Bu]), 1.25 (d, J = 6.3 Hz, 3H, $CH_3$ [Thr]). **$^{13}$C NMR** (101 MHz, $CDCl_3$): $\delta$ 171.2 ($CO_2{}^tBu$), 171.1 (CONH [C-2 Gal Tn]), 171.0 (CO [AcO C-3]), 170.62 (CO [AcO C-4]), 170.57 (CO [AcO C-6]), 170.5 (NHCO [Tn-TEG]), 157.0 (CO [Fmoc]), 143.8, 141.3 (C quatern [Fmoc]), 127.8, 127.2, 125.2, 125.1, 120.04, 120.02 (Ar Fmoc), 100.3 (C-1), 80.9 (C quatern [$^t$Bu]), 78.2 (CHa $CH_3$ [Thr]), 70.5, 70.4, 70.2, 70.1 (4 × $CH_{2(z)}$ TEG), 69.5 ($CH_{2(y')}$ TEG), 68.9 (C-3), 67.5 (C-4), 67.23 ($CH_2$ Fmoc), 67.20 (C-5), 66.8 ($CH_{2(y)}$ TEG), 62.2 (C-6), 58.7 (CHa NHFmoc), 47.5 (C-2), 47.2 (CH Fmoc), 39.5 ($CH_{2(x')}$ TEG), 36.2 ($CH_{2(x)}$ TEG), 28.1 (3 × ($CH_3$) [$^t$Bu]), 22.9 (AcNH [C-2 Gal]), 20.8 ($CH_3$ [AcO C-3]), 20.7 ($CH_3$ [AcO C-4]), 20.6 ($CH_3$ [AcO C-6]), 18.1 ($CH_3$ [Thr]). **HRMS (MALDI)** *m/z*: Calcd for $[C_{46}H_{63}N_3O_{17}Na]^+$ $[M+Na]^+$ 952.4048, found 952.4033

**Protected AcHN-Tn(Thr)-$PEG_3$ *tert*-butyl ester (33).**

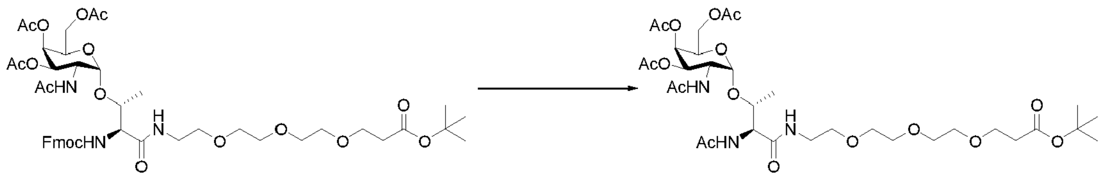

32 33

Piperidine (240 μL, 0.04 equiv) was added dropwise to a solution of Fmoc-protected Tn(Thr) moiety **32**(53.4 mg, 57.4 μmol) in DMF (1.0 mL). After 45 min, TLC (EtOAc) revealed consumption of starting material and solvent was removed under vacuum. Upon further azeotropic drying with toluene, the crude residue was dissolved in pyridine (900 μL) and acetic anhydride (300 μL) was added dropwise. When 2 h later TLC (EtOAc) indicated full conversion of the intermediate, the reaction mixture was concentrated to dryness and further dried with toluene. Purification by silica gel column chromatography (DCM/methanol 97.5:2.5 to 96.5:3.5), afforded AcHN-Tn(Thr)-$TEG_3$ spacer **33** (40.2 mg, 93% yield) as a white solid.

**TLC:** $R_f$ 0.75 (9:1 DCM/MeOH); **$^1$H NMR** (400 MHz, $CDCl_3$): $\delta$ 7.32 (t, *J* = 5.4 Hz, $^1$H, NH, NHCO [Tn-TEG]), 6.66 (d, *J* = 8.9 Hz, 1H, N*H*, NHAc [Thr]), 6.59 (d, *J* = 9.5 Hz, 1H, NH, NHAc [C-2 Gal Tn]), 5.34 (d, *J* = 3.6 Hz, 1H, H-4), 5.05 (dd, *J* = 11.3, 3.2 Hz, 1H, H-3), 4.88 (d, *J* = 3.6 Hz, 1H, H-1), 4.60 (dd, *J* = 9.1, 2.4 Hz, 1H, CHa NHAc [Thr]), 4.57 - 4.49 (m, 1H, H-2), 4.23 (t, *J* = 6.5 Hz, 1H, H-5), 4.16 (qd, *J* = 6.3, 2.3 Hz, 1H, C*H*α $CH_3$ [Thr]), 4.11 - 3.98 (m, 2H, H-6a,b), 3.69 (t, *J* = 6.3 Hz, 2H, $CH_{2(y)}$ TEG), 3.63-3.55 (m, 8H, 4 × $CH_{2(z)}$ TEG), 3.55-3.44 (m, 2H, $CH_{2(y')}$ TEG), 3.44-3.37 (m, 2H, $CH_{2(x')}$ TEG), 2.48 (t, *J* = 6.3 Hz, 2H, $CH_{2(x)}$ TEG), 2.13 (s, 3H, $CH_3$ [AcO C-4]), 2.09 (s, 3H, $CH_3$ [NHAc Thr]), 1.99 (s, 3H, $CH_3$ [AcO C-6]), 1.99 (s, 3H, $CH_3$ [NHAc C-2 Gal Tn]), 1.96 (s, 3H, $CH_3$ [AcO C-3]), 1.42 (s, 9H, 3 × ($CH_3$) [$^t$Bu]), 1.25 (d, *J* = 6.3 Hz, 3H, $CH_3$[Thr]). **$^{13}$C NMR** (101 MHz, $CDCl_3$): $\delta$ 171.2 ($CO_2{}^tBu$), 171.0 (CO [AcO C-3]), 170.8 (CONH [NHAc Thr]), 170.78 (CONH [C-2 Gal Tn]), 170.5 (CO [AcO C-4 & C-6]), 170.4 (NHCO [Tn-TEG]), 100.5 (C-1), 80.9 (C quatern [$^t$Bu]), 78.4 (CHa $CH_3$ [Thr]), 70.6, 70.5, 70.4, 70.3 (4 × $CH_{2(z)}$ TEG), 69.5 ($CH_{2(y')}$ TEG), 69.1 (C-3), 67.5 (C-4), 67.3 (C-5), 66.9 ($CH_{2(y)}$ TEG), 62.3 (C-6), 56.6 (CHa NHAc [Thr]), 47.5 (C-2), 39.5 ($CH_{2(x')}$ TEG), 36.3 ($CH_{2(x)}$ TEG), 28.2 (3 × ($CH_3$) [$^t$Bu]), 23.3 (AcNH [Thr]), 23.1 (AcNH [C-2 Gal]), 20.88 ($CH_3$ [AcO C-3]), 20.86 ($CH_3$ [AcO C-4]), 20.7 ($CH_3$ [AcO C-6]), 18.3 ($CH_3$ [Thr]). **HRMS (MALDI)** *m/z*: Calcd for $[C_{33}H_{55}N_3O_{16}Na]^+$ $[M+Na]^+$ 772.3473, found 772.3416.

**AcHN-Tn(Thr)-$PEG_3$ *tert*-butyl ester (34).**

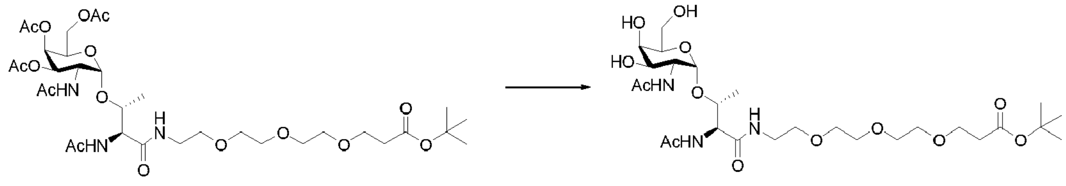

33 34

To a suspension of **33** (33 mg, 44 μmol) in MeOH (1.0 mL), hydrazine (50 μL) was added, resulting in a clear solution. The reaction was monitored by HPLC and after 90 min toluene was added. The mixture was concentrated and the residue was purified by silica gel chromatography using a Büchi Pure C-850 automated system (DCM/MeOH 90:10 to 82:18), providing the deacetylated Tn(Thr)-$TEG_3$ *tert*-butyl ester **34** (37.2 mg, 84% yield) as a glassy solid.

**TLC:** $R_f$ 0.22 (9:1 DCM/MeOH); **$^1$H NMR** (600 MHz, methanol-$d_4$): $\delta$ 4.85 (d, $J$ = 3.7 Hz, 1H, H-1), 4.52 (d, $J$ = 2.6 Hz, 1H, CHa NHAc [Thr]), 4.26-4.19 (m, 2H, H-2, C$H\alpha$ $CH_3$ [Thr]), 3.91-3.86 (m, 2H, H-4, H-5), 3.75 (dd, $J$ = 11.0, 3.2 Hz, 1H, H-3), 3.73-3.68 (m, 4H, H-6a,b, C$H_{2(y)}$ TEG), 3.66-3.57 (m, 8H, 4 × C$H_{2(z)}$ TEG), 3.55 - 3.50 (m, 2H, C$H_{2(y')}$ TEG), 3.50-3.45 (m, 1H, CH$Ha_{(x')}$ TEG), 3.31 - 3.27 (m, 1H, CH$Hb_{(x')}$ TEG), 2.48 (t, $J$ = 6.2 Hz, 2H, C$H_{2(x)}$ TEG), 2.09 (s, 3H, C$H_3$ [NHAc Thr]), 2.07 (s, 3H, C$H_3$ [NHAc C-2 Gal Tn]), 1.45 (s, 9H, 3 × ($CH_3$) [$^t$Bu]), 1.27 (d, $J$ = 6.4 Hz, 3H, $CH_3$ [Thr]). **$^{13}$C NMR** (151 MHz, methanol-$d_4$): $\delta$ 174.1 (CONH [C-2 Gal Tn]), 173.6 (CONH [NHAc Thr]), 172.8 ($CO_2{}^t$Bu), 172.3 (NHCO [Tn-TEG]), 101.0 (C-1), 81.7 (C quatern [$^t$Bu]), 77.7 (CHa $CH_3$ [Thr]), 72.9 (C-5), 71.53, 71.47, 71.4, 71.3 (4 × $CH_{2(z)}$ TEG), 70.40 (C-3), 70.38 ($CH_{2(y')}$ TEG), 70.26 (C-4), 67.9 ($CH_{2(y)}$ TEG), 62.7 (C-6), 58.4 (CHa NHAc [Thr]), 51.4 (C-2), 40.5 ($CH_{2(x')}$ TEG), 37.2 ($CH_{2(x)}$ TEG), 28.4 (3 × ($CH_3$) [$^t$Bu]), 23.2 (AcNH [C-2 Gal]), 22.5 (AcNH [Thr]), 19.2 ($CH_3$ [Thr]). **HRMS (ESI)** m/z: Calcd for $[C_{27}H_{49}N_3O_{13}Na]+$ [M+Na]+ 646.3154, found 646.3128.

**AcHN-Tn(Thr)-$PEG_3$ acid (35).**

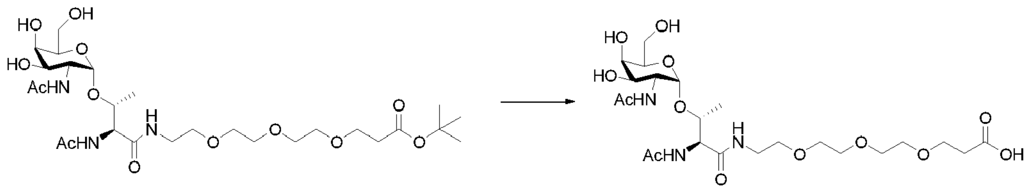

34 35

*Tert*-butyl ester **34** (26.5 mg, 42 μmol) at 0 °C was dissolved in a precooled (0 °C) solution of TFA (0.8 mL). The reaction mixture was stirred at this temperature and its progress was monitored by HPLC. After 1 h, DCM was added and the contents were evaporated to dryness. The crude was purified by reverse phase (RP) chromatography using a Büchi Pure C-850 automated system [0-100% acetonitrile/water (0.05% TFA)], affording the deprotected Tn(Thr)-$TEG_3$ carboxylic acid **35** (20.5 mg, 86% yield) as a white solid.

**HPLC:** $t_R$ = 13.56 min, $\lambda_{max}$ =194.52 nm; **$^1$H NMR** (600 MHz, methanol-$d_4$): $\delta$ 4.85 (d, $J$ = 3.8 Hz, 1H, H-1), 4.52 (d, $J$ = 2.6 Hz, 1H, CHa NHAc [Thr]), 4.26 - 4.20 (m, 2H, H-2, CHa $CH_3$ [Thr]), 3.91 - 3.87 (m, 2H, H-4, H-5), 3.77 - 3.70 (m, 5H, H-3, H-6a, H-6b, C$H_{2(y)}$ TEG), 3.65 - 3.58 (m, 8H, 4 × C$H_{2(z)}$ TEG), 3.54 - 3.50 (m, 2H, C$H_{2(y')}$ TEG), 3.50 - 3.45 (m, 1H, CH$Ha_{(x')}$ TEG), 3.32 - 3.28 (m, 1H, CH$Hb_{(x')}$ TEG), 2.58 - 2.54 (m, 2H, C$H_{2(x)}$ TEG), 2.09 (s, 3H, C$H_3$ [NHAc Thr]), 2.07 (s, 3H, C$H_3$ [NHAc C-2 Gal Tn]), 1.27 (d, $J$ = 6.4 Hz, 3H, $CH_3$ [Thr]). **$^{13}$C NMR** (151 MHz, methanol-$d_4$): $\delta$ 175.4 ($CO_2H$), 174.2 (CONH [C-2 Gal Tn]), 173.7 (CONH [NHAc Thr]), 172.4 (NHCO [Tn-TEG]), 101.0 (C-1), 77.7 (CHa $CH_3$ [Thr]), 72.9 (C-5), 71.54, 71.45, 71.4, 71.3 (4 × $CH_{2(z)}$ TEG), 70.42 (C-3), 70.40 ($CH_{2(y')}$ TEG), 70.3 (C-4), 67.8 ($CH_{2(y)}$ TEG), 62.7 (C-6), 58.5 (CHa NHAc [Thr]), 51.4 (C-2), 40.5 ($CH_{2(x')}$ TEG), 35.8 ($CH_{2(x)}$ TEG), 23.2 (AcNH [C-2 Gal]), 22.5 (AcNH [Thr]), 19.2 ($CH_3$ [Thr]). **HRMS (ESI)** m/z: Calcd for $[C_{23}H_{41}N_3O_{13}Na]^+$ $[M+Na]^+$ 590.2485, found 590.2528.

**7.2. Preparation of Tn-containing vaccines**

**Aminoacyl quillaic acid-Tn(Thr) saponin [QA-Tn] (26)** [RF-III-162].

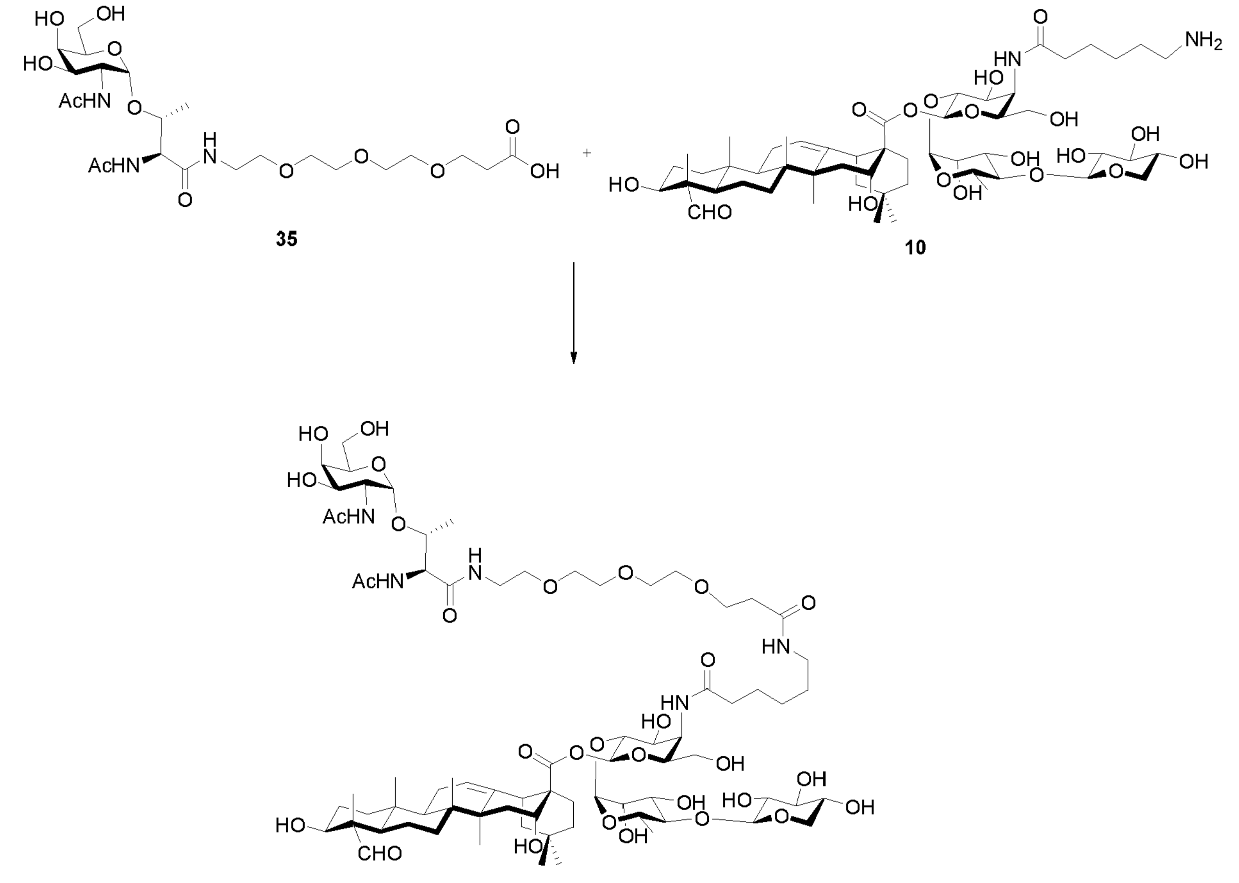

35 + 10

26

To a stirred solution of Tn(Thr)-spacer acid **35** (3.9 mg, 6.9 μmol, 1.5 equiv), benzotriazol-1-yl-oxy]tripyrrolidinophosphonium hexafluorophosphate (PyBOP) (3.8 mg, 7.3 μmol, 1.6 equiv) and quillaic acid saponin amine **10** (4.8 mg, 4.6 μmol, 1.0 equiv) in DMF (1.6 mL), diisopropylethylamine (DIPEA) (2.4 μL, 13.8 μmol, 3.0 equiv) was added dropwise. The reaction mixture was stirred at r.t. for 1 h and then concentrated under high vacuum. The resulting residue was dissolved in 50% acetonitrile/water, filtered through 0.2 μm PTFE filter disk and purified by RP-HPLC on a XBridge prep BEH300 C18 column (5 μm, 19 × 250 mm) using a linear gradient of 20-100% acetonitrile/water (0.05% TFA) over 30 min at a flow rate of 17 mL/min. The fraction containing the major peak was collected and lyophilized to dryness, providing the desired quillaic acid saponin-Tn conjugate **26** (6.2 mg, 85% yield) as a white solid.

HPLC: $t_R$ = 22.56 min, $\lambda_{max}$ =193.5 nm. **$^1$H NMR** (600 MHz, methanol-$d_4$): $\delta$ 9.31 (s, 1H, H-23 [CHO] QA), 8.16 (t, J = 5.6 Hz, 1H, NH [CONH Thr-TEG]), 8.10 (d, J = 9.1 Hz, 1H, NH [NHAc Thr]), 7.97 (d, J = 9.2 Hz, 1H, NH [CONH C-4 N-Gal]), 7.44 (d, J = 9.2 Hz, 1H, NH [NHAc C-2 Gal Tn]), 5.39 (d, J = 1.8 Hz, 1H, H-1 Rha), 5.34 (d, J = 7.8 Hz, 1H, H-1 N-Gal), 5.31 (t, J = 3.7 Hz, 1H, H-12 QA), 4.86-4.83 (m, 1H, H-1 Gal Tn), 4.56-4.51 (m, 1H, C$H\alpha$ NHAc [Thr]), 4.51 - 4.47 (m, 2H, H-1 Xyl, H-16 QA), 4.37 - 4.32 (m, 1H, H-4 N-Gal), 4.27-4.19 (m, 2H, H-2 Gal Tn, C$H\alpha$ $CH_3$ [Thr]), 3.97 - 3.87 (m, 5H, H-3 & H-2 N-Gal, H-2 Rha, H-4 & H-5 Gal Tn), 3.87 - 3.78 (m, 3H, H-3 & H-5 Rha, H-5a Xyl), 3.78 - 3.68 (m, 7H, H-3 QA, H-6a,b & H-3 Gal Tn, C$H_{2(y)}$ TEG, H-5 N-Gal), 3.65 - 3.58 (m, 8H, 4 × C$H_{2(z)}$ TEG), 3.58 - 3.45 (m, 6H, H-6a N-Gal, CH$Ha_{(x')}$ & C$H_{2(y')}$ TEG, H-4 Xyl, H-4 Rha), 3.45 - 3.40 (m, 1H, H-6b N-Gal), 3.35 - 3.27 (m, 2H, CH$Hb_{(x')}$ TEG, H-3 Xyl), 3.24 - 3.16 (m, 4H, C$H_{2(a')}$NHCO acyl, H-5b & H-2 Xyl), 2.94 (dd, J = 14.3, 4.6 Hz, 1H, H-18 QA), 2.44 (t, J = 6.2 Hz, 2H, C$H_{2(x)}$ TEG), 2.37 - 2.28 (m, 3H, C$H_{2(a)}$CONH acyl, H-19a QA), 2.09 (s, 3H, $CH_3$ [NHAc Gal]), 2.07 (s, 3H, $CH_3$ [NHAc Thr]), 2.00 - 1.89 (m, 4H, H-11a,b, H-22a & H-21a QA), 1.83 - 1.62 (m, 8H, C$H_{2(b)}$$CH_2$CONH acyl, H-22b, H-9, H-1a, H-2a,b & H-15a QA), 1.60 - 1.43 (m, 5H, C$H_{2(b')}$$CH_2$NHCO acyl, H-6a, H-7a & H-15b QA), 1.43 - 1.30 (m, 10H, C$H_{2(c)}$$CH_2$$CH_2$CONH acyl, H-7b & H-5 QA, [1.41 s, $CH_3$ C-27 QA], [1.33 d, *J* = 6.3 Hz, 3H, $CH_3$ Rha]), 1.27 (d, J = 6.4 Hz, 3H, $CH_3$ Thr), 1.20 - 1.09 (m, 2H, H-21b & H-1b QA), 1.09 - 1.03 (m, $^1$H, H-19b QA), 1.02 (s, 3H, $CH_3$ C-24 QA), 1.01 (s, 3H, $CH_3$ C-25 QA), 0.95 (s, 3H, $CH_3$ C-30 QA), 0.94 - 0.90 (m, 1H, H-6b), 0.88 (s, 3H, $CH_3$ C-29 QA), 0.77 (s, 3H, $CH_3$ C-26 QA). **$^{13}$C NMR** (151 MHz, methanol-$d_4$): $\delta$ 208.8 (CHO QA), 178.2 (CONH acyl [C-4 N-Gal]), 176.9 (CO [C-28] QA), 174.2 (CONH [C-2 Gal Tn]), 173.8 (CONH [TEG-acyl]), 173.7 (CONH [NHAc Thr]), 172.4 (NHCO [Tn-TEG]), 144.8 (C-13 QA), 123.2 (C-12 QA), 107.0 (C-1 Xyl), 101.3 (C-1 Rha), 101.0 (C-1 Gal Tn), 95.6 (C-1 N-Gal), 84.1 (C-4 Rha), 78.2 (C-3 Xyl), 77.7 (CHa $CH_3$ [Thr]), 76.4 (C-5 N-Gal), 76.2 (C-2 Xyl), 74.9 (C-3 N-Gal), 74.6 (C-16

QA), 74.4 (C-2 N-Gal), 72.94 (C-5 Gal Tn), 72.87 (C-3 QA), 72.2 (C-3 Rha), 71.9 (C-2 Rha), 71.54, 71.46, 71.32, 71.30 (4 × $CH_{2(z)}$ TEG), 71.1 (C-4 Xyl), 70.4 (C-3 Gal Tn & $CH_{2(y')}$ TEG), 70.3 (C-4 Gal Tn), 68.9 (C-5 Rha), 68.3 ($CH_{2(y)}$ TEG), 67.3 (C-5 Xyl), 62.7 (C-6 Gal Tn), 61.7 (C-6 N-Gal), 58.5 (CHa NHAc [Thr]), 56.8 (C-4 QA), 52.5 (C-4 N-Gal), 51.4 (C-2 Gal Tn), 50.0 (C-17 QA), 48.9 (C-5 QA), 48.05 (C-19 QA), 48.02 (C-9 QA), 42.8 (C-14 QA), 42.4 (C-18 QA), 41.1 (C-8 QA), 40.5 ($CH_{2(x')}$ TEG), 40.3 ($CH_{2(a')}$NHCO acyl), 39.5 (C-1 QA), 37.7 ($CH_{2(x)}$ TEG), 37.0 (C-10 QA), 36.6 ($CH_{2(a)}$CONH acyl), 36.54 (C-21 QA), 36.53 (C-15 QA), 33.6 (C-7 QA), 33.4 ($CH_3$ C-29 QA), 32.0 (C-22 QA), 31.3 (C-20 QA), 30.1 ($\mathit{CH}_{2(b')}CH_2$NHCO acyl), 27.5 ($\mathit{CH}_{2(c)}CH_2CH_2$CONH acyl), 27.2 ($CH_3$ C-27 QA), 27.0 (C-2 QA), 26.8 ($\mathit{CH}_{2(b)}CH_2$CONH acyl), 24.9 ($CH_3$ C-30 QA), 24.5 (C-11 QA), 23.2 ($CH_3$ [NHAc, Thr]), 22.6 ($CH_3$ [NHAc, Gal]), 21.9 (C-6 QA), 19.2 ($CH_3$ Thr), 18.4 ($CH_3$ Rha), 17.8 ($CH_3$ C-26 QA), 16.3 ($CH_3$ C-25 QA), 9.5 (CH3 C-24 QA). **HRMS (MALDI)** m/z: Calcd for $[C_{76}H_{125}N_5O_{30}Na]^+$ $[M+Na]^+$ 1610.8300, found 1610.8349.

**PV-aminoacyl quillaic acid-Tn(Thr) saponin [QA(PV)-Tn] (27)** [RF-III-165].

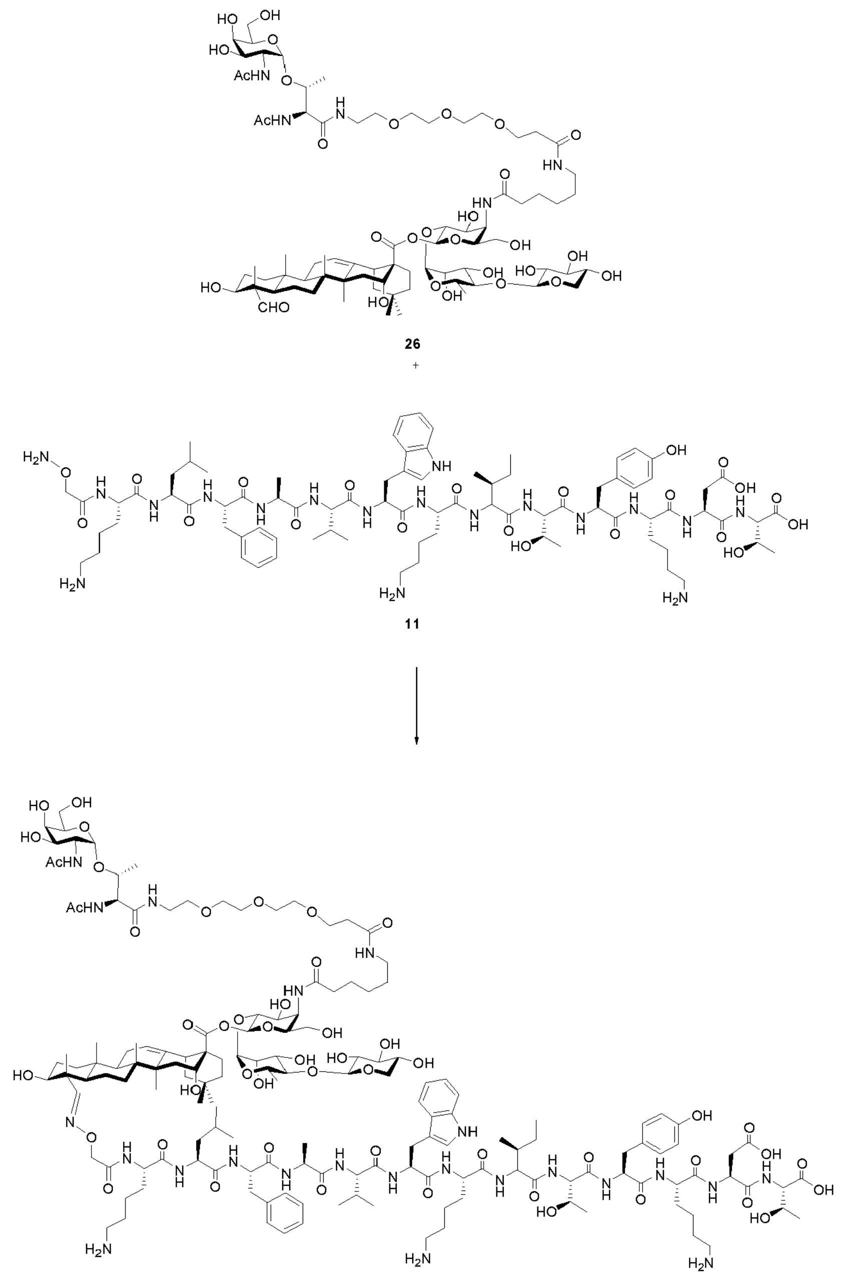

26

+

11

27

QA saponin-Tn conjugate **26** (4.6 mg, 2.8 μmol, 1.0 equiv) was coupled with aminooxy peptide PV 11 (9.0 mg, 5.3 μmol, 1.8 equiv) in acetonitrile/water (2:3, 0.05% TFA, 1.5 mL). After stirring for 16 h at 40 °C, the reaction mixture was HPLC purified [10-100% acetonitrile/water (0.05% TFA)]. The fraction containing the major peak was collected and

lyophilized to dryness, providing the desired QA(PV)-Tn conjugate **27** (4.9 mg, 52% yield) as a white solid.

**HPLC:** $t_R$ = 25.02 min, $\lambda_{max}$ = 195.52 nm. **MS (ESI)** *m/z*: Calcd for $[C_{157}H_{259}N_{23}O_{50}]^{+2}$ [M+2H]/$2^{+2}$ 1628.38, found 1629.31, $[C_{163}H_{260}N_{21}O_{59}]^{+3}$ [M+3H]/$3^{+3}$ 1085.92, found 1086.52.

### 7.3. Preparation of Gb3 antigen + diethyleneglycol (DEG) spacer

The following Scheme shows the synthesis of the Gb3 antigen moiety functionalized with a PEG-based, amino-reactive linker.

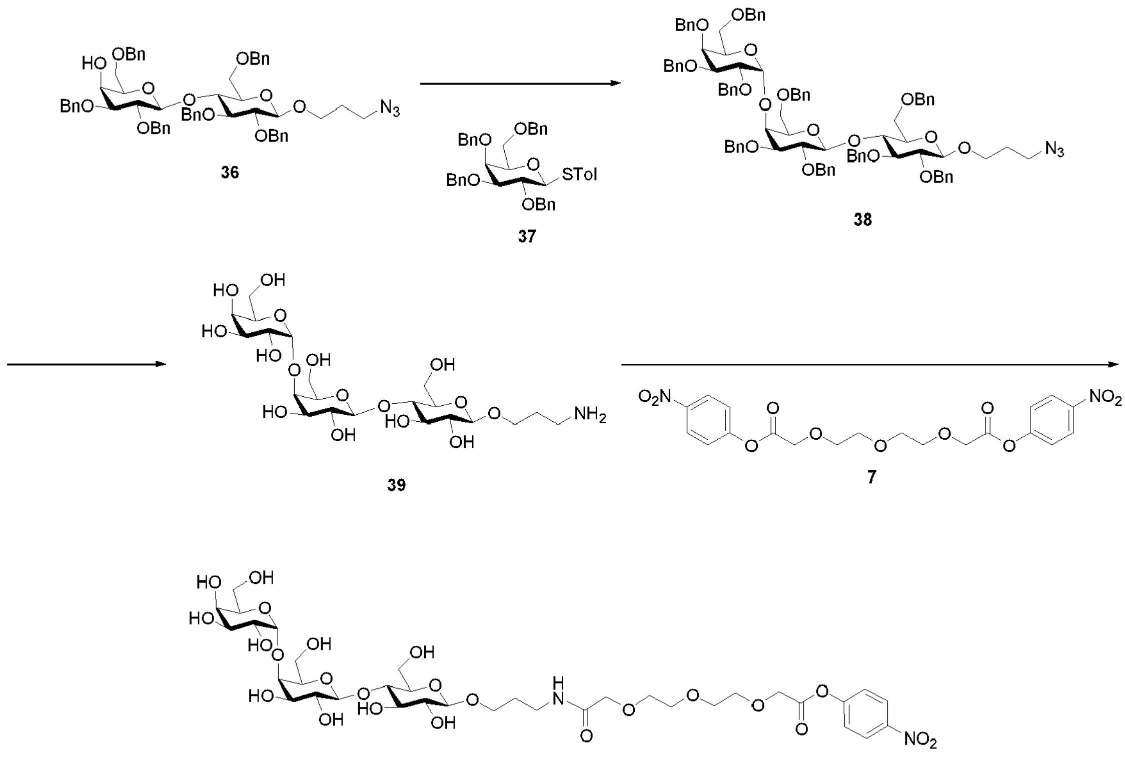

36 37 38 39 7

40

**3-Azidopropyl 2,3,4,6-Tetra-O-benzy)-α-d-galactopyranosyl-(1→4)-2,3,6-tri-O-benzyl-β-d galactopyranosyl-(1→4)-2,3,6-tri-O-benzyl-β-d-glucopyranoside (38)** (RF-II-112).

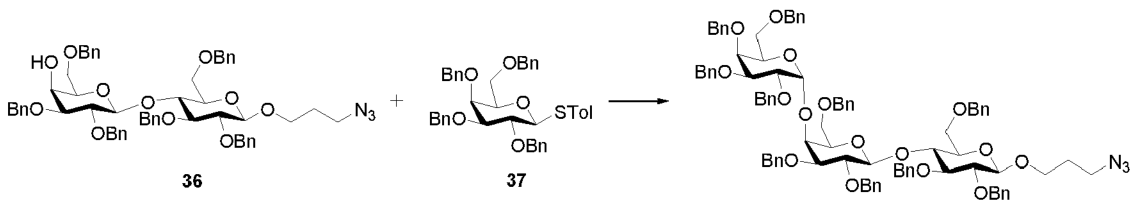

36 + 37

38

Aceptor disaccharide **36** (20.18 mg, 0.02 mmol, 1 equiv.) and donor STolyl monosaccharide **37** (14.16 mg, 0.021 mmol, 1.05 equiv.) were azeotroped with toluene. Activated molecular sieves (20 mg) was then added followed by dry DCM/$Et_2O$ 2:1 (0.9 mL). After stirring for 5 min, the mixture was cooled to -78°C and an activated AgOTf (azeotropically dried with toluene) (16.23 mg, 0.063 mmol, 3.16 equiv.) solution in dry $Et_2O$ was added with a syringe, followed by addition of ρ-TolSCl without touching the glass walls. The reaction was stirred for 1 h while allowing to warm up to room temperature, observing consumption of starting material. The reaction mixture was filtered through celite rinsing with two portions of DCM, and concentrated. The resulting crude was dissolved in DCM and washed with NaHCO3, and brine. The organic layer was dried over $MgSO_4$, filtered and concentrated. Purification by silica gel chromatography (96:4 Toluene/EtOAc) afforded the product **(38)** (20.95 mg, 70% yield) as a clear oil that can eventually crystallize.

**TLC:** $R_f$ 0.64 (7:3 hexane/EtOAc); **$^1$H NMR** (600 MHz, $CDCl_3$) δ 7.42 - 7.10 (m, 45H), 5.07 (d, J = 11.1 Hz, 1H),

5.06 (d, J = 3.5 Hz, 1H), 4.86 (dd, J = 11.3, 6.7 Hz, 2H), 4.80 - 4.67 (m, 7H), 4.54 - 4.42 (m, 6H), 4.38 - 4.33 (m, 3H), 4.30 - 4.23 (m, 2H), 4.20 - 4.15 (m, 1H), 4.13 - 4.06 (m, 3H), 4.05 - 4.01 (m, 2H), 4.00 - 3.93 (m, 3H), 3.82 (dd, J = 10.8, 4.3 Hz, 1H), 3.70 (dd, J = 10.8, 1.6 Hz, 1H), 3.66 - 3.54 (m, 3H), 3.53 - 3.48 (m, 2H), 3.39 - 3.28 (m, 6H), 3.18 (dd, J = 8.3, 4.6 Hz, 1H), 1.93 - 1.81 (m, 2H). **$^{13}$C NMR** (151 MHz, $CDCl_3$) δ 139.21, 139.07, 138.89, 138.82, 138.73, 138.56, 138.49, 138.17, 128.65, 128.49, 128.41, 128.38, 128.34, 128.27, 128.25, 128.07, 127.95, 127.80, 127.75, 127.73, 127.68, 127.60, 127.55, 127.53, 127.47, 127.27, 103.62, 102.95, 100.86, 82.74, 81.77, 79.56, 77.23, 76.74, 75.36, 75.23, 75.18, 75.13, 75.01, 74.94, 73.86, 73.42, 73.32, 73.19, 73.15, 72.58, 72.21, 69.57, 68.35, 67.97, 67.85, 66.59, 48.46, 29.38.

**3-Aminopropyl 2,3,4,6-Tetrol-α-d-galactopyranosyl-(1→4)-2,3,6-triol-β-d galactopyranosyl-(1→4)-2,3,6-triol-β-d-glucopyranoside (39)** (RF-III-053).

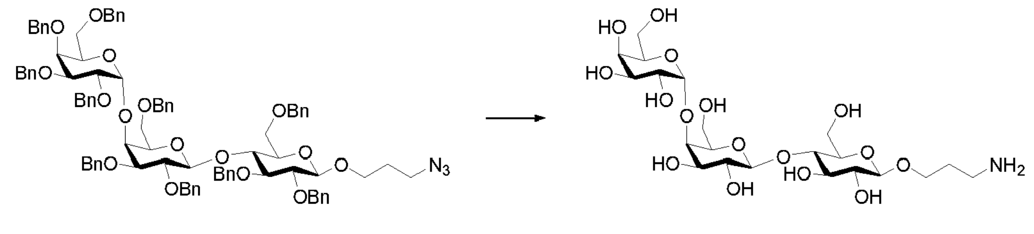

38 39

Azidopropyl Gb3 **38** (30 mg, 0.02 mmol) and Pd/C 10% (108 mg, 0.10 mmol, 5 equiv.) were dissolved/suspended in a mixture of MeOH/DCM 3:1 (12 mL). A $H_2$ balloon was connected to the flask and the atmosphere was purged and refilled with $H_2$ 5 times. After the mixture was stirred overnight, monitoring by direct injection on the mass spectrometer confirmed total conversion. The mixture was then filtered through a syringe equipped with a 0.45 μm PTFE disc filter, concentrated and lyophilized, affording the deprotected aminopropyl Gb3 **39** (10.8 mg, 96% yield) as a white solid.

**$^1$H NMR** (600 MHz, $D_2O$) δ 4.95 (d, J = 3.9 Hz, 1H), 4.52 (d, J = 2.8 Hz, 1H), 4.51 (d, J = 2.6 Hz, 1H), 4.36 (t, J = 6.5 Hz, 1H), 4.09 - 3.97 (m, 4H), 3.96 - 3.88 (m, 2H), 3.87 - 3.77 (m, 5H), 3.77 - 3.68 (m, 3H), 3.67 - 3.56 (m, 4H), 3.35 - 3.30 (m, 1H), 3.17 (t, J = 7.0 Hz, 2H), 2.05 - 1.99 (m, 2H). **$^{13}$C NMR** (151 MHz, $D_2O$) δ 103.9, 102.7, 100.9, 79.3, 78.0, 76.1, 75.5, 75.0, 73.5, 72.8, 71.5, 71.5, 69.8, 69.6, 69.2, 68.5, 61.1, 61.0, 60.6, 38.2, 27.3. **MS (ESI)** *m/z*: Calcd for $[C_{21}H_{39}NO_{16}]^+$ $[M+H]^+$ 562.23, found 562.33.

**Gb3-DEG linker (40)** (RF-III-057).

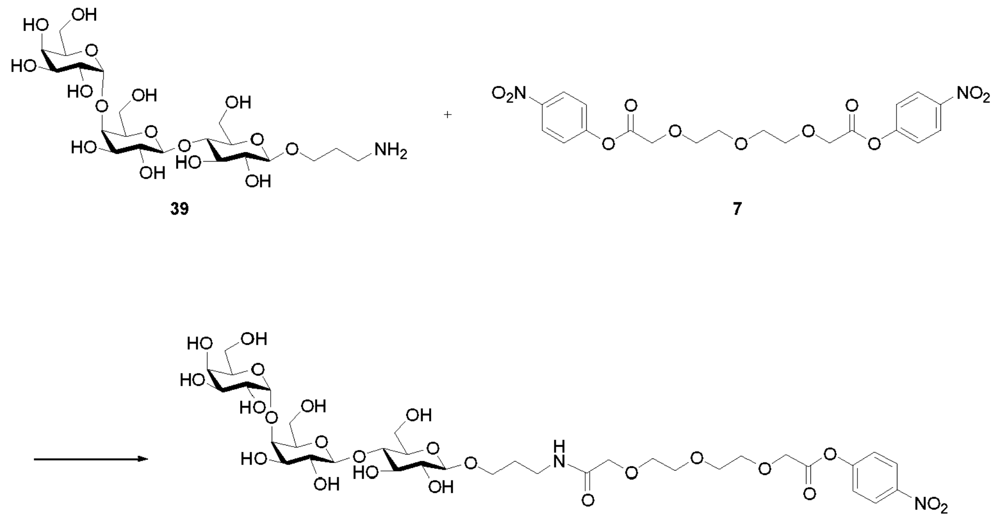

39 7

40

To a solution of deprotected aminopropyl Gb3 **39** (10.8 mg, 19.2 umol) in dry DMF (2.6 mL) and DIPEA (8.3 μL, 2.5 equiv), a solution of bis-activated DEG linker **7** (50 mg, 0.10 mmol, 5.6 equiv) in dry DMF (0.48 mL) was added at r.t., at which point the solution turned from clear to yellow. After stirring for 1 h, HPLC-MS monitoring confirmed consumption of aminopropyl Gb3. The reaction mixture was neutralized with 1.5 μL of TFA, turning from yellow to a clear solution. After removing DMF in vacuo while stirring at 30 °C, the resulting crude was 100issolved in $H_2O$/MeCN 1:1. Purification by RP-HPLC and lyophilization of the fraction containing the desired product afforded the Gb3-linker

conjugate **40** (7.88 mg, 46% yield).

**HPLC:** $t_R$ = 18.37 min, $\lambda_{max}$ = 193.52 nm and 271.52 nm. **$^1$H NMR** (600 MHz, $D_2O$) $\delta$ 8.40 - 8.33 (m, 1H), 7.48 - 7.41 (m, 2H), 4.95 (d, J = 4.0 Hz, 1H), 4.60 (d, J = 2.3 Hz, 2H), 4.50 (d, J = 7.7 Hz, 1H), 4.45 (d, J = 8.0 Hz, 2H), 4.36 (t, J = 6.3 Hz, 1H), 4.24 (s, 1H), 4.10-4.05 (m, 2H), 4.07 - 4.01 (m, 2H), 4.02 - 3.87 (m, 6H), 3.89 - 3.65 (m, 13H), 3.68 - 3.51 (m, 4H), 3.40-3.26 (m, 3H), 1.91 - 1.80 (m, 2H). **$^{13}$C NMR** (151 MHz, $D_2O$) $\delta$ 173.0, 171.1, 155.1, 146.2, 126.2, 123.2, 103.9, 102.6, 100.9, 79.3, 79.3, 78.0, 76.0, 75.4, 75.0, 73.5, 72.7, 71.5, 71.4, 71.1, 70.9, 70.6, 70.2, 70.2, 70.1, 70.1, 70.1, 70.1, 69.7, 69.5, 69.1, 68.4, 68.3, 68.2, 61.1, 60.9, 60.6, 36.5, 29.1. **MS (ESI)** *m/z*: Calcd for $[C_{35}H_{55}N_2O_{24}]^+$ $[M+H]^+$ 887.31, found 887.68.

**7.4. Preparation of Gb3-containing vaccines**

**Aminoacyl quillaic acid QA-Gb3 saponin (28)** [RF-III-116].

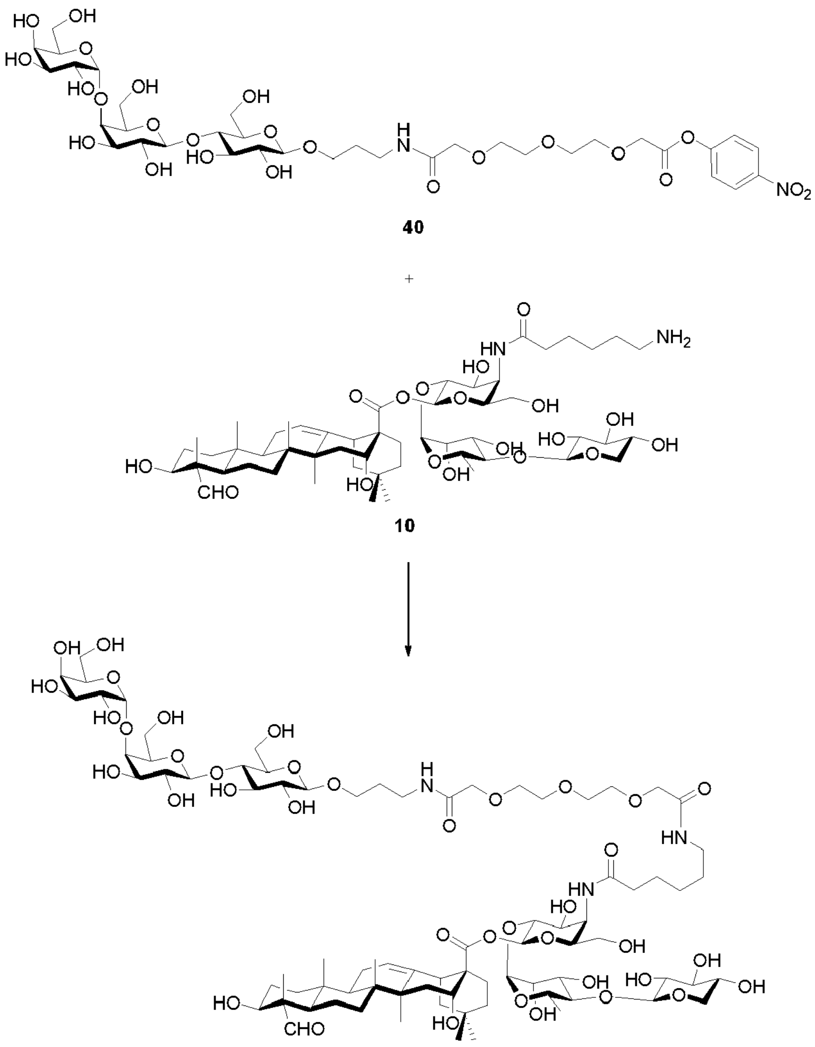

40

+

10

28

Saponin amine **10** (8.2 mg, 7.9 µmol, 1.0 equiv) was acylated with the activated ester functionalized Gb3 linker **40** (11.3 mg, 12.7 µmol, 1.6 equiv) in DMF (2.6 mL) for 1 h via preactivation with DIPEA (2.7 µL, 15.8 µmol, 2.0 equiv). After RP-HPLC purification [10-100% acetonitrile/water (0.05% TFA)] and lyophilization of the desired fraction containing the major peak, quillaic acid saponin QA-Gb3 conjugate **28** (11.4 mg, 80% yield) was obtained as a white solid.

**HPLC:** $t_R$ = 23.72 min, $\lambda_{max}$ =194.52 nm. **$^1$H NMR** (800 MHz, Methanol-$d_4$) $\delta$ 9.31 (s, 1H), 5.38 (d, *J* = 1.9 Hz, 1H), 5.34 (d, *J* = 7.9 Hz, 1H), 5.31 (t, *J* = 3.8 Hz, 1H), 4.95 (d, *J* = 3.8 Hz, 1H), 4.50 - 4.47 (m, 2H), 4.42 (d, *J* = 7.2 Hz, 1H), 4.34 (dd, *J* = 4.7, 1.8 Hz, 1H), 4.30 (d, *J* = 7.8 Hz, 1H), 4.26 (t, *J* = 6.2 Hz, 1H), 4.01 (s, 4H), 3.99 (d, *J* = 2.9 Hz, 1H), 3.97 - 3.76 (m, 16H), 3.75 - 3.67 (m, 13H), 3.65 - 3.60 (m, 1H), 3.59 - 3.50 (m, 7H), 3.48 - 3.44 (m, 1H), 3.44 - 3.37 (m, 5H), 3.34 - 3.31 (m, 1H), 3.28 - 3.24 (m, 3H), 3.23 - 3.17 (m, 3H), 2.94 (dd, *J* = 14.3, 4.7 Hz, 1H), 2.36 - 2.28 (m,

3H), 1.98 - 1.87 (m, 4H), 1.86 - 1.63 (m, 11H), 1.60 - 1.43 (m, 5H), 1.41 (s, 3H), 1.40 - 1.35 (m, 2H), 1.34 - 1.31 (m, 4H), 1.29 (d, *J* = 8.3 Hz, 1H), 1.20 - 1.04 (m, 3H), 1.02 (s, 3H), 1.00 (d, *J* = 2.2 Hz, 3H), 0.95 (s, 3H), 0.94 - 0.89 (m, 1H), 0.88 (s, 3H), 0.77 (s, 3H). **$^{13}$C NMR** (201 MHz, Methanol-$d_4$) $\delta$ 208.8, 178.2, 177.0, 172.6, 162.7, 162.5, 162.3, 144.8, 123.2, 107.0, 105.4, 104.2, 102.7, 101.3, 95.6, 84.1, 81.0, 79.8, 78.2, 76.5, 76.4, 76.3, 76.1, 74.9, 74.7, 74.4, 72.9, 72.8, 72.7, 72.2, 71.8, 71.3, 71.2, 71.1, 71.0, 70.6, 68.9, 68.6, 67.3, 62.7, 61.9, 61.7, 61.5, 56.8, 52.5, 50.0, 49.5, 48.0, 48.0, 42.8, 42.4, 41.1, 40.8, 39.9, 39.5, 37.5, 37.0, 36.5, 33.6, 33.4, 32.0, 31.3, 30.5, 30.3, 27.5, 27.2, 27.0, 26.8, 24.9, 24.5, 21.9, 18.4, 17.8, 16.3, 9.5. **MS (ESI)** *m*/*z*: Calcd for $[C_{81}H_{134}N_3O_{39}]^+$ $[M+H]^+$ 1786.87, found 1787.17.

**PV-aminoacyl quillaic acid-Gb3 saponin [QA(PV)-Gb3] (29)** [RF-III-118]

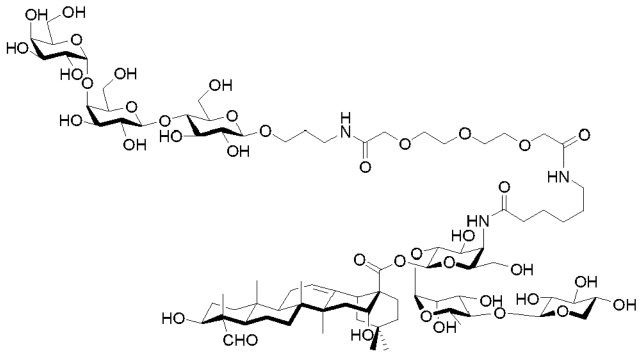

**28**

+

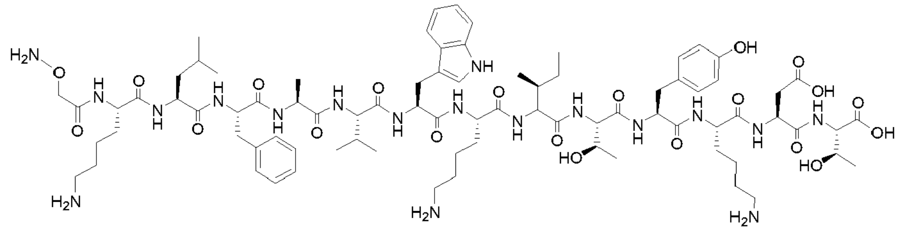

**11**

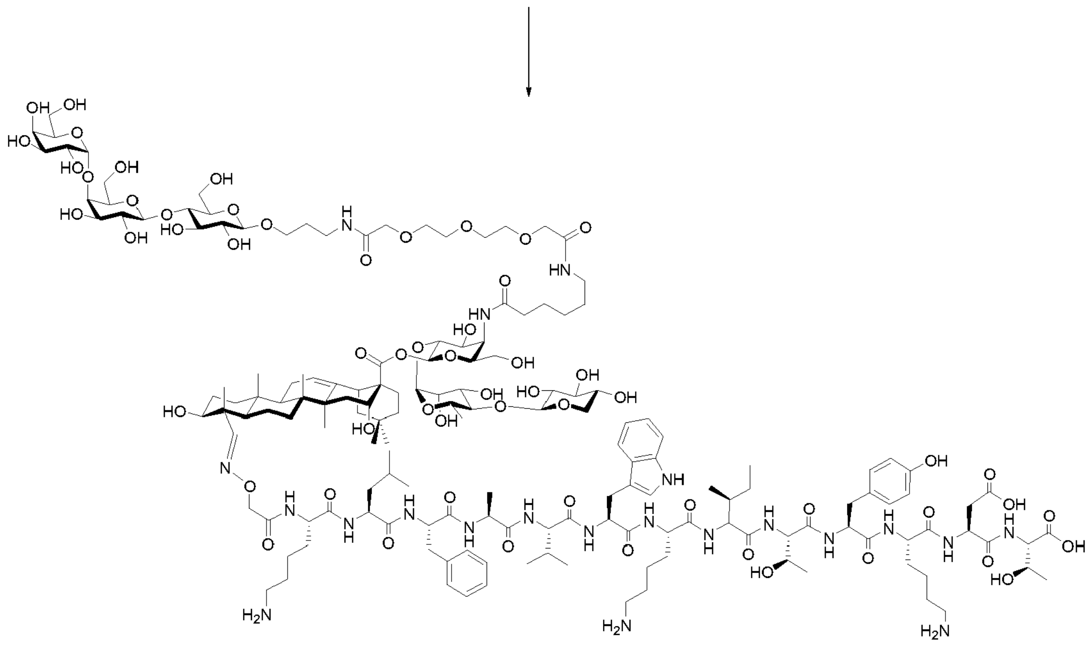

**29**

QA saponin-Gb3 conjugate **28** (5.0 mg, 2.8 μmol, 1.0 equiv) was coupled with aminooxy peptide PV **11** (9.4 mg, 5.6 μmol, 2.0 equiv) in acetonitrile/water (2:3, 0.05% TFA, 1.5 mL). After stirring for 16 h at 40 °C, the reaction mixture was HPLC purified [10-100% acetonitrile/water (0.05% TFA)]. The fraction containing the major peak was collected and lyophilized to dryness, affording the QA(PV) -Gb3 conjugate **29** (6.6 mg, 68% yield) as a white solid.

**HPLC:** $t_R$ = 24.88 min, $\lambda_{max}$ = 193.52 nm. **MS (ESI)** *m/z*: Calcd for $[C_{163}H_{259}N_{21}O_{59}]^{+2}$ $[M+2H]/2^{+2}$ 1727.39, found 1727.35, $[C_{163}H_{260}N_{21}O_{59}]^{+3}$ $[M+3H]/3^{+3}$ 1151.93, found 1152.20.

**IN VIVO IMMUNOLOGICAL EVALUATION**

**Animals.** Animals were cared for and handled in compliance with the Guidelines for Accommodation and Care of Animals (European Convention for the Protection of Vertebrate Animals Used for Experimental and Other Scientific Purposes) and internal guidelines. Mice were housed in standard cages with an automatic water system and fed on a standard diet ad libitum. All the experimental procedures were approved by the appropriate local authorities. The CIC bioGUNE animal facility is fully accredited by AAALAC Intl.

**Quantification of antibody production.** Antibody titers against OVA protein, or against MUC1 and TnMUC1 peptides in the case of the self-adjuvanting vaccine constructs. were measured by an indirect enzyme-linked immunosorbent assay (ELISA). Briefly, ELISA plates (Thermo Scientific) were coated with BSA-MUC1 or BSA-TnMUC1 conjugates at 0.05 μg/well in carbonate buffer (pH 9.5) and plates were incubated overnight at 4 °C. After washing the wells (PBS, 10 mM, containing 0.05% Tween 20), plates were blocked with 10% of fetal calf serum (FCS, Biowest) in PBS buffer for 1 h. Serial dilutions of mouse sera in blocking buffer (10% FCS in PBS buffer) were added to wells with appropriate controls and incubated for 1h at room temperature. After washing, goat anti-mouse total IgG (Jackson Immuno Research) antibodies conjugated to horseradish peroxidase (HRP) were added diluted 1/1000 in blocking buffer and incubated for 1 h at room temperature. KPL SureBlue reserve™ commercial solution (100μL/well, SeraCare) containing 3,3',5,5'-tetramethylbenzidine(TMB) was added as peroxidase substrate and after incubation for 10 min, the reaction was stopped with 2N $H_2SO_4$ (100 μL/well). Absorbance (OD, 450 nm) was immediately measured using a BioTek® Synergy HT multi-detection microplate reader.

***In vivo* experiment 1: Adjuvant activity of compound 25 vs prior art compound 13**

A straightforward proof-of-principle *in vivo* experiment was carried out to directly compare the adjuvant activities of prior art compound **13** and the compound of the invention **25** in terms of IgG antibody production, using chicken ovalbumin (OVA) as a model antigen. Groups of two C75BL6 mice each were administered three bi-weekly subcutaneous injections containing toxin-free OVA antigen (20 μg) in combination with QA-based adjuvant **13** (50 μg) or with the new QA(oxime) adjuvant **25** (50 μg). Moreover, one group was injected with toxin-free OVA (20 μg) alone as no-adjuvant control group and another group with PBS only as negative control. Endpoint sera after three immunizations were probed via ELISA assays by coating 96-well plates with toxin-free OVA (0.5 μg/ml) and total IgG optical density (OD) values (day 42) were calculated **(Figure 4).** The data show that that the compound of the invention **25** [QA(oxime)] induced a notable anti-OVA IgG antibody response, significantly higher than that observed when using OVA alone as well as OVA in the presence of the prior art compound **13.** Remarkably, these results reveal that the installation of the C4-oxime group results in saponin variants with improved adjuvant activity that are able to induce increased levels of antigen-specific IgG antibodies.

***In vivo* experiment 2: saponin conjugate 3 [QA(PV)-MUC1] as self-adjuvanting vaccine**

A first round of immunization in groups of five mice (C57BL/6) was performed to assess the ability of the saponin conjugates to produce anti-MUC1 IgG antibodies. This *in vivo* study involved a set of MUC1 peptide related compounds and formulations administered following a subcutaneous immunization schedule with a prime injection (day 0) and two boosts (day 14 and day 28), for a total of three bi-weekly immunizations **(Figure 5).** Middle point sera were obtained at day 21 (one week after the second immunization), while end point sera were collected at day 42 (two weeks after the third immunization). Four groups of mice (groups A-D) were immunized in this first experiment with the following synthetic molecules: (A) the MUC1 peptide antigen alone (compound **5**) [Group A], (B) a mixture of the MUC1 peptide (5) and the PV Th epitope (compound **22**) [Group B], (C) a mixture of the di-component saponin-MUC1 conjugate **1** and the TH epitope (**22**) [Group C], and (D) the tri-component saponin construct QA(PV)-MUC1 (**3**) incorporating both the MUC1 and the PV peptides [Group D]. Each mouse received a dose of antigen equimolar to 50 μg of MUC1 per injection in a 100 μL volume.

ELISA assays were performed by coating 96-well plates with a MUC1-functionalized BSA protein conjugate (**23**) **(Figure 6)**. Serial dilution versus optical density (OD, 450 nm) graphs demonstrated that mice immunized with a self-adjuvant vaccine of the invention such as tri-component QA(PV)-MUC1 construct **3** were the only ones capable of

producing high titers of total anti-MUC1 IgG antibodies. The intensity of the antibody response induced by the tri-component vaccine **3** was already pronounced at day 21.

Also, anti-MUC1 IgG subtypes were consistently higher for mice immunized with tri-component QA(PV)-MUC1 construct **3 (Figure 7).**

***In vivo* experiment 3: Saponin conjugate 4 [QA(PV)-TnMUC1] as self-adjuvanting vaccine**

A further *in vivo* study was performed with the TnMUC1 glycopeptide antigen **(Figure 8).** Three immunization groups received the TnMUC1 glycopeptide **(6),** which was co-administered with either: (i) the known QA-based saponin adjuvant **13** [Group B], (ii) a mixture of QA saponin **13** and PV epitope **22** [Group C], or (iii) a newly synthesized QA saponin-(PV) conjugate, that is molecule **14** [Group F]. Two immunization groups were administered with the di-component QA saponin-TnMUC1 construct **2:** either alone [Group D] or mixed with the PV epitope [Group E], and another group was injected with the tri-component QA(PV)-TnMUC1 construct **4** incorporating both PV and TnMUC1 peptides [Group G]. Finally, a negative control group was included receiving PBS only [Group A]. Each mouse was injected a dose of antigen equimolar to 50 μg of TnMUC1 per injection in a 100 μL volume, while QA saponin adjuvant **13** was administered at a fixed dose of 50 μg, according to our previous protocol.[68] Because in the previous experiment the difference in total IgG titers between day 21 and day 42 was not particularly pronounced, we decided to reduce the immunization schedule to two biweekly injections (one prime injection at day 0 followed by one boost at day 14). Middle point sera were obtained at day 10, while endpoint sera were collected at day 24, ten days after the second immunization.

In this setting, the total anti-TnMUC1 IgG antibody titers (at day 24) induced by the tri-component construct **4** of the present invention were significantly higher than those observed for the other groups **(Figure 9),** in line with the good results obtained in the previous immunization experiment with the corresponding tricomponent vaccine **3.** Analogously, induced IgG isotypes were also consistently higher for mice immunized with the tri-component construct **4** (Figure **10**).

Collectively, all these data prove that the incorporation of an oxime group (-C=N-O-) at position C4 in the new triterpene glycoside saponin adjuvants developed in the present invention leads to a much more pronounced adjuvant capacity as compared with known C-4 aldeyde variants. Morevover, potent self-adjuvanting vaccines have been constructed by conjugating to the saponin scaffold moieties capable of stimulating an immune response, e.g. by derivatization at the C4-aldehyde position through an oxime linkage as well as by functionalization of the acyl chain appended to the carbohydrate domain.

**List of references**

Ahmad, T. A., et al. Trials in Vaccinology 2016, Volume 5, Pages 71-83. https://doi.org)/10.1016/i.trivac.2016.04.003.

Bergmann-Leitner, E.; Leitner, W. Adjuvants in the Driver's Seat: How Magnitude, Type, Fine Specificity and Longevity of Immune Responses Are Driven by Distinct Classes of Immune Potentiators. Vaccines 2014, 2 (2), 252-296. https://doi.org/10.3390/vaccines2020252.

Buskas, T.; Ingale, S.; Boons, G.-J. Towards a Fully Synthetic Carbohydrate-Based Anticancer Vaccine: Synthesis and Immunological Evaluation of a Lipidated Glycopeptide Containing the Tumor-Associated Tn Antigen. Angew. Chemie Int. Ed. 2005, 44 (37), 5985-5988. hftps://doi.org/10.1002/anie.200501818.

Doe, B.; Steimer, K.S.; Walker, C.M.; Induction of HIV-1 envelope (gp120)-specific cytotoxic T lymphocyte responses in mice by recombinant CHO cell-derived gp120 is enhanced by enzymatic removal of N-linked glycans. Eur J Immunol. 1994 Oct;24(10):2369-76. https://doi.org/10.1002/eji.1830241017.

Dönnes, P.; Kohlbacher, O. Integrated modeling of the major events in the MHC class I antigen processing pathway. Protein Sci. 2005 Aug;14(8):2132-40. https://doi.org/10.1110/ps.051352405.

Doytchinova, I.A.; Guan, P.; Flower, D.R.. EpiJen: a server for multistep T cell epitope prediction. BMC Bioinformatics 2006 Mar 13;7:131. https://doi.org/10.1186/1471-2105-7-131.

Erickson, A.L.; Houghton, M.; Choo, Q.L.; Weiner, A.J., Ralston, R.; Muchmore, E.; Walker, C.M.; Hepatitis C virus-specific CTL responses in the liver of chimpanzees with acute and chronic hepatitis C. J Immunol. 1993 Oct 15;151(8):4189-99.

Fernández-Tejada, A.; Chea, E. K.; George, C.; Pillarsetty, N.; Gardner, J. R.; Livingston, P. O.; Ragupathi, G.; Lewis, J. S.; Tan, D. S.; Gin, D. Y. Development of a Minimal Saponin Vaccine Adjuvant Based on QS-21. Nat. Chem. 2014, 6 (7), 635-643. https://doi.org/10.1038/nchem.1963.

Ghirardello, M.; Ruiz-de-Angulo, A.; Sacristan, N.; Barriales, D.; Jiménez-Barbero, J.; Poveda, A.; Corzana, F.; Anguita, J.; Fernández-Tejada, A. Exploiting Structure-activity Relationships of QS-21 in the Design and Synthesis of Streamlined Saponin Vaccine Adjuvants. Chem. Commun. 2020, 56 (5), 719-722. https://doi.org/10.1039/C9CC07781B.

Payne, R. J.; Ficht, S.; Tang, S.; Brik, A.; Yang, Y. Y.; Case, D. A.; Wong, C. H. Extended Sugar-Assisted Glycopeptide Ligations: Development, Scope, and Applications. J. Am. Chem. Soc. 2007, 129 (44), 13527-13536. https://doi.org/10.1021/ja073653p.

Pifferi, C.; Fuentes, R.; Fernández-Tejada, A. Natural and Synthetic Carbohydrate-Based Vaccine Adjuvants and Their Mechanisms of Action. Nat. Rev. Chem. 2021, 25 (4), 3-7. https://doi.org/10.1038/s41570-020-00244-3.

Reche, P. A.; Glutting, J. P.; Zhang, H.; Reinherz, E.L. Enhancement to the RANKPEP resource for the prediction of peptide binding to MHC molecules using profiles. Immunogenetics 2004 Sep; 56(6):405-19. https://doi.org/10.1007/s00251-004-0709-7.

Reed, S. G.; Orr, M. T.; Fox, C. B. Key Roles of Adjuvants in Modern Vaccines. Nat. Med. 2013, 19 (12), 1597-1608. https://doi.org/10.1038/nm.3409.

Singh R.; Gupta P.; Sharma P. K.; Ades E. W.; Hollingshead S. K.; Singh S.; Lillard J. W. Prediction and characterization of helper T-cell epitopes from pneumococcal surface adhesin A. Immunology. 2014 Apr;141(4):514-30. https://doi.org/10.1111/imm.12194.

Wang R. F. Identification of MHC class II-restricted tumor antigens recognized by CD4+ T cells. Methods. 2003 Mar;29(3):227-35. https://doi.org/10.1016/s1046-2023(02)00345-6.

Wittmann, V.; Takayama, S.; Gong, K. W.; Weitz-Schmidt, G.; Wong, C. H. Ligand Recognition by E- and P-Selectin: Chemoenzymatic Synthesis and Inhibitory Activity of Bivalent Sialyl Lewis x Derivatives and Sialyl Lewis x Carboxylic Acids. J. Org. Chem. 1998, 63 (15), 5137-5143. https://doi.org/10.1021/jo980350s.

Wu, Z.; Guo, X.; Guo, Z. Chemoenzymatic Synthesis of Glycosylphosphatidylinositol-Anchored Glycopeptides. Chem. Commun. 2010, 46 (31), 5773-5774. https://doi.org/10.1039/C0CC00828A.

WO2009/126737, WO2015/184451, WO2017/079582, WO2017/106836, WO2018/191598, WO2018/200645 WO2018/200656 and WO2019/079160.

## Claims

**1.** A compound selected from:

- a compound of general formula (I) or a pharmaceutically acceptable salt thereof

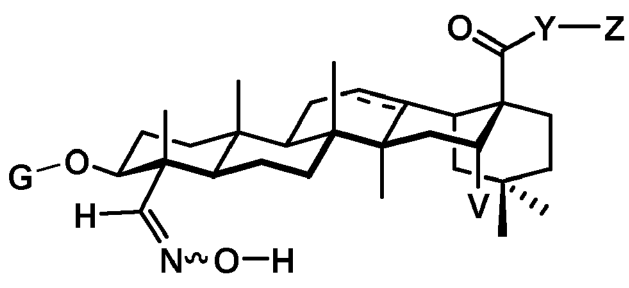

(I)

wherein G is hydrogen, a branched trisacchride of formula (VI) or a stereoisomer of formula (VI)

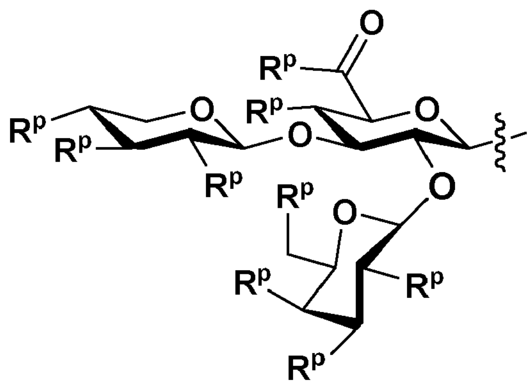

(VI)

wherein each occurrence of $R^p$ is independently hydrogen or $OR^q$;

wherein each occurrence of $R^q$ is independently hydrogen or an optionally substituted group selected from 6-10-membered aryl, benzyl, $C_{1-6}$ aliphatic, or $C_{1-6}$ heteroaliphatic having 1-2 heteroatoms independently selected from the group consisting of nitrogen, oxygen, and sulfur; or two $R^q$ are taken together to form a 5-7-membered heterocyclic ring having 1-2 heteroatoms independently selected from the group consisting of nitrogen, oxygen, and sulfur;

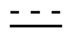

is a single or double bond;
V is H or $OR^x$;
Y is $CH_2$, -O-, -S-, -NR, or-NH-;
Z is a carbohydrate domain having the structure:

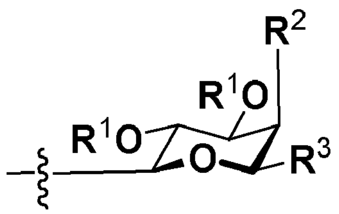

or

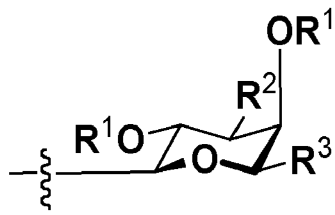

wherein each occurrence of $R^1$ is $R^x$ or a carbohydrate domain having the structure:

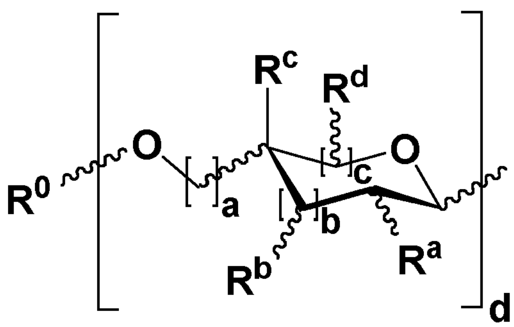

wherein:

each occurrence of a, b, and c is independently 0, 1, or 2;

d is an integer from 1-5, wherein each d bracketed structure may be the same or different; with the proviso that the d bracketed structure represents a furanose or a pyranose moiety, and the sum of b and c is 1 or 2;

$R^0$ is hydrogen; an oxygen protecting group selected from the group consisting of alkyl ethers, benzyl ethers, silyl ethers, acetals, ketals, esters, carbamates, and carbonates; or an optionally substituted moiety selected from the group consisting of acyl, $C_{1-10}$ aliphatic, $C_{1-6}$ heteroaliphatic, 6-10-membered aryl, arylalkyl, 5-10-membered heteroaryl having 1-4 heteroatoms independently selected from nitrogen, oxygen, or sulfur, 4-7-membered heterocyclyl having 1-2 heteroatoms independently selected from the group consisting of nitrogen, oxygen, and sulfur;

each occurrence of $R^a$, $R^b$, $R^c$, and $R^d$ is independently hydrogen, halogen, OH, OR, $OR^x$, $NR_2$, NHCOR, or an optionally substituted group selected from acyl, $C_{1-10}$ aliphatic, $C_{1-6}$ heteroaliphatic, 6-10-membered aryl, arylalkyl, 5-10-membered heteroaryl having 1-4 heteroatoms independently selected from nitrogen, oxygen, and sulfur; 4-7-membered heterocyclyl having 1-2 heteroatoms independently selected from the group consisting of nitrogen, oxygen, and sulfur;

$R^2$ is hydrogen, halogen, OH, OR, $OC(O)R^4$, $OC(O)OR^4$, $OC(O)NHR^4$, $OC(O)NRR^4$, $OC(O)SR^4$, $NHC(O)R^4$, $NRC(O)R^4$, $NHC(O)OR^4$, $NHC(O)NHR^4$, $NHC(O)NRR^4$, $NHR^4$, $N(R^4)_2$, $NHR^4$, $NRR^4$, $N_3$, or an optionally substituted group selected from $C_{1-10}$ aliphatic, $C_{1-6}$ heteroaliphatic, 6-10-membered aryl, arylalkyl, 5-10-membered heteroaryl having 1-4 heteroatoms independently selected from the group consisting of nitrogen, oxygen, and sulfur, 4-7-membered heterocyclyl having 1-2 heteroatoms independently selected from the group consisting of nitrogen, oxygen, and sulfur;

$R^3$ is hydrogen, halogen, $CH_2OR^1$, or an optionally substituted group selected from the group consisting of acyl, $C_{1-10}$ aliphatic, $C_{1-6}$ heteroaliphatic, 6-10-membered aryl, arylalkyl, 5-10-membered heteroaryl having 1-4 heteroatoms independently selected from the group consisting of nitrogen, oxygen, and sulfur, 4-7-membered heterocyclyl having 1-2 heteroatoms independently selected from the group consisting of nitrogen, oxygen, and sulfur;

$R^4$ is $-T-R^z$, $-C(O)-T-R^z$, $-NH-T-R^z$, $-O-T-R^z$, $-S-T-R^z$, $-C(O)NH-T-R^z$ , $C(O)O-T-R^z$, $C(O)S-T-R^z$, $C(O)NH-T-O-T-R^z$ - $O-T-R^z$, $-T-O-T-R^z$, $-T-S-T-R^z$, or

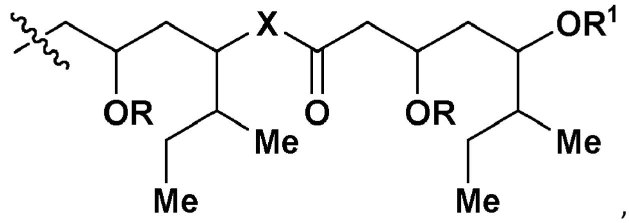

,

wherein

X is -O-, -NR-, or $T-R^z$;

T is a covalent bond or a bivalent $C_{1-26}$ saturated or unsaturated, straight or branched, aliphatic or heteroaliphatic chain; and

$R^z$ is hydrogen, halogen, -OR, $-OR^x$, $-OR^1$, -SR, $NR_2$, -C(O)OR, -C(O)R, -NHC(O)R,-NHC(O)OR, -NC(O)OR, or an optionally substituted group selected from acyl, arylalkyl, heteroarylalkyl, $C_{1-6}$ aliphatic, 6-10-membered aryl, 5-10-membered heteroaryl having 1-4 heteroatoms independently selected from nitrogen, oxygen, or sulfur, 4-7-membered heterocyclyl having 1-2 heteroatoms independently selected from the group consisting of nitrogen, oxygen, and sulfur;

each occurrence of $R^x$ is independently hydrogen or an oxygen protecting group selected from the group consisting of alkyl ethers, benzyl ethers, silyl ethers, acetals, ketals, esters, carbamates, and carbonates;

each occurrence of R is independently hydrogen, an optionally substituted group selected from acyl, arylalkyl, 6-10-membered aryl, $C_{1-6}$ aliphatic, or $C_{1-6}$ heteroaliphatic having 1-2 heteroatoms independently selected from the group consisting of nitrogen, oxygen, and sulfur, or,

two R on the same nitrogen atom are taken with the nitrogen atom to form a 4-7-membered heterocyclic ring having 1-2 heteroatoms independently selected from the group consisting of nitrogen, oxygen, and sulfur;

and

- a compound of general formula (II) or a pharmaceutically acceptable salt thereof

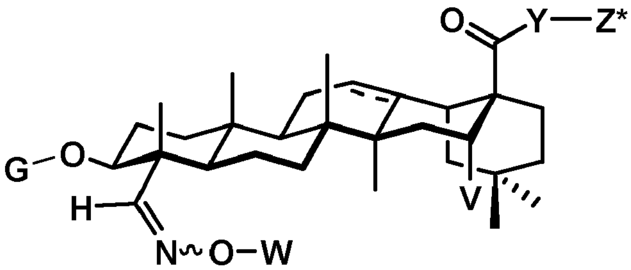

(II)

wherein - - -, G, V and Y take the meanings as in formula (I);
W is H or a moiety comprising either i) at least one T cell epitope, ii) at least one B cell epitope or iii) at least one T cell epitope and at least one B cell epitope;
Z* represents that Z, as defined in formula (I), is optionally conjugated with a moiety comprising either i) at least one T cell epitope, ii) at least one B cell epitope or iii) at least one T cell epitope and at least one B cell epitope;
with the proviso that at least one T cell epitope and at least one B cell epitope are present in the compound of formula (II);
wherein the at least one T cell epitope is independently selected in every instance from a helper T cell epitope or a CD8 epitope.

**2.** A compound selected from:

- a compound of general formula (III) or a pharmaceutically acceptable salt thereof

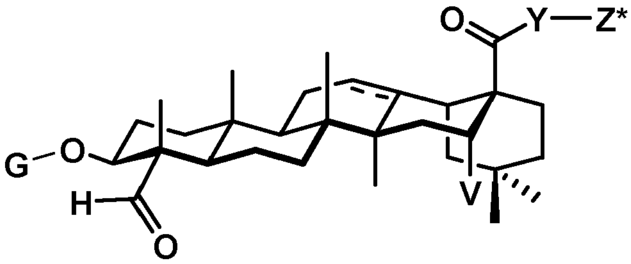

(III)

wherein - - -, G, V and Y take the meanings as in claim 1 and Z* represents that Z, as defined in formula (I) of claim 1, is conjugated with a moiety comprising either i) at least one T cell epitope selected from a helper T cell epitope or a CD8 epitope, ii) at least one B cell epitope or iii) at least one T cell epitope selected from a helper T cell epitope or a CD8 epitope and at least one B cell epitope;
and
- a compound of general formula (IV) or a pharmaceutically acceptable salt thereof

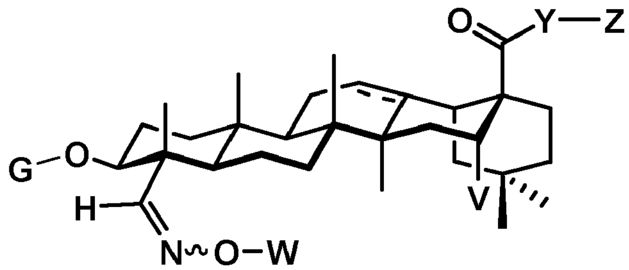

(IV)

wherein - - -, G, V, Y and Z take the meanings as in claim 1 and W is a moiety comprising either i) at least one T cell epitope selected from a helper T cell epitope or a CD8 epitope, ii) at least one B cell epitope or iii) at least one T cell epitope selected from a helper T cell epitope or a CD8 epitope and at least one B cell epitope.

**3.** The compound according to any one of claims 1 or 2,
wherein G is hydrogen or

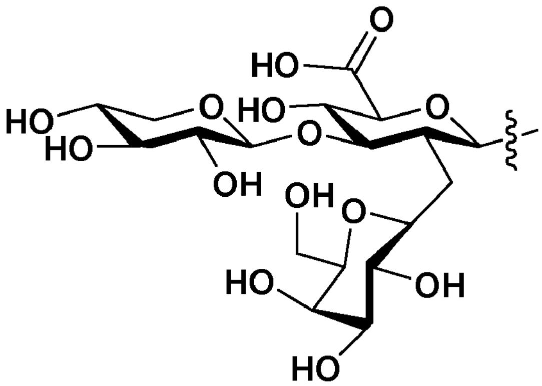

;

- - - is a single or double bond;
V is H or OH;
Y is -O-;
Z is a carbohydrate domain having the structure:

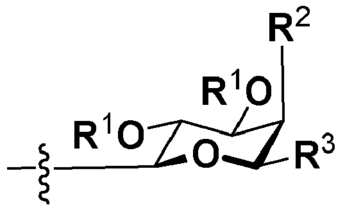

wherein
$R^1$ is independently H or

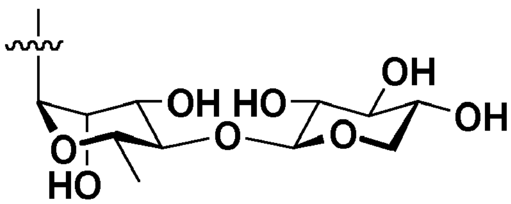

$R^2$ is $NHR^4$;

$R^3$ is $CH_2OH$; and
$R^4$ is -T-$R^z$, -C(O)-T-$R^z$, -NH-T-$R^z$, -O-T-$R^z$, -S-T-$R^z$, -C(O)NH-T-$R^z$, C(O)O-T-$R^z$, C(O)S-T-$R^z$, C(O)NH-T-O-T-$R^z$ -O-T-$R^z$, -T-O-T-$R^Z$, -T-S-T-$R^z$, or

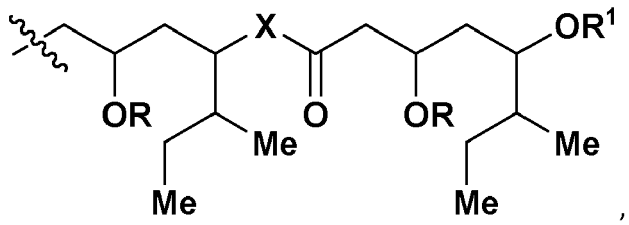

,

wherein:
X is -O-, -NR-, or T-$R^z$;
T is a covalent bond or a bivalent $C_{1-26}$ saturated or unsaturated, straight or branched, aliphatic or heteroaliphatic chain; and
$R^z$ is hydrogen, halogen, -OR, - $OR^x$, -$OR^1$, -SR, -$NR_2$, -C(O)OR, -C(O)R, -NHC(O)R,-NHC(O)OR, -NC(O)OR, or an optionally substituted group selected from acyl, arylalkyl, heteroarylalkyl, $C_{1-6}$ aliphatic, 6-10-membered aryl, 5-10-membered heteroaryl having 1-4 heteroatoms independently selected from nitrogen, oxygen, or sulfur, 4-7-membered heterocyclyl having 1-2 heteroatoms independently selected from the group consisting of nitrogen, oxygen, and sulfur;
$R^x$ is independently hydrogen or an oxygen protecting group selected from the group consisting of alkyl ethers, benzyl ethers, silyl ethers, acetals, ketals, esters, carbamates, and carbonates; and
R is independently hydrogen, an optionally substituted group selected from acyl, arylalkyl, 6-10-membered aryl, $C_{1-6}$ aliphatic, or $C_{1-6}$ heteroaliphatic having 1-2 heteroatoms independently selected from the group consisting of nitrogen, oxygen, and sulfur, or;
two R on the same nitrogen atom are taken with the nitrogen atom to form a 4-7-membered heterocyclic ring having 1-2 heteroatoms independently selected from the group consisting of nitrogen, oxygen, and sulfur.

**4.** The compound according to any one of claims 1 to 3, wherein in the compounds of formula (I) and (IV), $R^4$ comprises a functional group of a specific binding pair, wherein said functional group is selected from one member of the following binding pairs:

i) aldehyde, ketone, isothiocyanate, carboxylic acid or derivative thereof such as ester, anhydride, acyl halide, tosyl and N-hydrosuccinimide (NHS) ---- amine;
ii) alkyne or phosphine ---- azide;
iii) cycloalkene, cycloalkyne, cyclopropane, isonitrile (isocyanide) or vinyl boronic acid ---- tetrazine;
iv) alkyne or maleimide ---- thiol;
v) conjugated diene ---- substituted alkene;
vi) alkene, alkyne or copper acetylide ---- nitrone;
vii) aldehyde or ketone ---- alkoxyamine, hydroxylamine, hydrazine or hydrazide;
and in the compounds of formula (II) and (III), the conjugation of Z with a moiety comprising either i) at least one T cell epitope, ii) at least one B cell epitope or iii) at least one T cell epitope and at least one B cell epitope is carried out by a bond resulting from one of the above binding pairs.

**5.** The compound according to claim 1, wherein in the compounds of formula (II) $R^4$ is selected from:

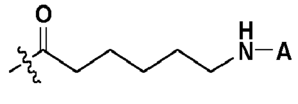 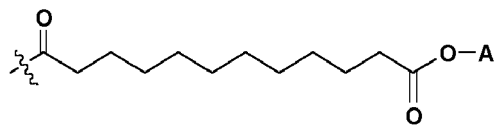

wherein A is H or a moiety comprising either i) at least one T cell epitope selected from a helper T cell epitope or a CD8 epitope, ii) at least one B cell epitope or iii) at least one T cell epitope selected from a helper T cell epitope or a CD8 epitope, and at least one B cell epitope; preferably, A is a moiety comprising at least one B cell epitope.

**6.** The compound according to claim 1, wherein the compouind of formula (II) is selected from the group consisting of:

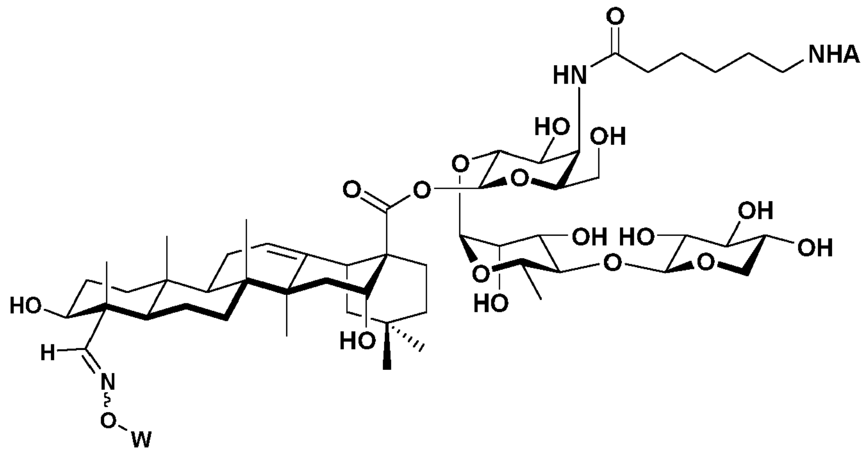

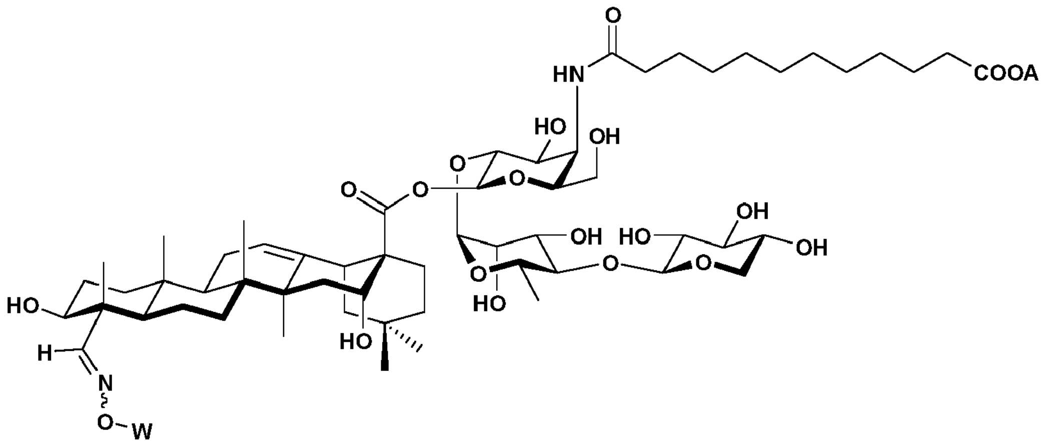

wherein W and A are independently H or a moiety comprising either i) at least one T cell epitope selected from a helper T cell epitope or a CD8 epitope, ii) at least one B cell epitope or iii) at least one T cell epitope selected from a helper T cell epitope or a CD8 epitope, and at least one B cell epitope;
with the proviso that the compound of formula (II) comprises at least one T cell epitope and at least one B cell epitope.

**7.** The compound according to any one of claims 1 to 6, wherein the B cell epitope or the CD8 T cell epitope is selected from the group consisting of peptides, glycopeptides and carbohydrates capable of inducing an immune response against a neurodegenerative disease, an infectious disease or a cancer cell.

**8.** The compound according to claim 7, wherein the B cell epitope or the CD8 T cell epitope is or is found within the immunogenic region of a cancer-associated antigen selected from the group consisting of:

- MUC1 peptide, TnMUC1 glycopeptide, Gb3 carbohydrate and Tn carbohydrate antigens such as Tn(Thr) antigen;
- Glycoproteins such as PSA; Mucins such as MUC1, MUC2, MUC4, MUC5AC, MUC6, MUC16; Mucin-derived carbohydrate antigens such as Tn, TF, STn; gangliosides such as GM2, GM3, GD2, GD3; globosides such as Gb4, Gb5, Globo-H;
- 5T4, 8H9, av beta 6 integrin, alphafetoprotein (AFP), B7-H6, CA-125, carbonic anhydrase 9 (CA9), CD19, CD20, CD22, CD30, CD33, CD38, CD44, CD44v6, CD44v7/8, CD52, CD123, CD171, carcionoembryonic antigen (CEA), EGFRvIII, epithelial glycoprotein-2 (EGP-2), epithelial glycoprotein-40 (EGP-40), ErbB1/EGFR, ErbB2/HER2/neu/EGFR2, ErbB3, ErbB4, epithelial tumor antigen (ETA), FBP, fetal acetylcholine receptor (AchR), folate receptor-a, G250/CAIX, ganglioside 2 (GD2), ganglioside 3 (GD3), HLA-A1, HLA-A2, high molecular weight melanoma-associated antigen (HMW-MAA), IL-13 receptor a2, KDR, k-light chain, Lewis Y (LeY), L1 cell adhesion molecule, melanoma-associated antigen (MAGE-A1), mesothelin, Murine CMV infected cells,

mucin-1 (MUC1), mucin-16 (MUC16), natural killer group 2 member D (NKG2D) ligands, nerve cell adhesion molecule (NCAM), NY-ESO-1, Oncofetal antigen (h5T4), prostate stem cell antigen (PSCA), prostate-specific membrane antigen (PSMA), receptor-tyrosine kinase-like orphan receptor 1 (ROR1), TAA targeted by mAb IgE, tumor-associated glycoprotein-72 (TAG-72), tyrosinase, and vascular endothelial growth factor (VEGF) receptors;

- human Her2/neu, Her1/EGF receptor (EGFR), Her3, A33 antigen, B7H3, CD5, CD19, CD20, CD22, CD23 (IgE Receptor), C242 antigen, 5T4, IL-6, IL-13, vascular endothelial growth factor VEGF (e.g., VEGF-A) VEGFR-1, VEGFR-2, CD30, CD33, CD37, CD40, CD44, CD51, CD52, CD56, CD74, CD80, CD152, CD200, CD221, CCR4, HLA-DR, CTLA-4, NPC-1C, tenascin, vimentin, insulin-like growth factor 1 receptor (IGF-1R), alpha-fetoprotein, insulin-like growth factor 1 (IGF-1), carbonic anhydrase 9 (CA-IX), carcinoembryonic antigen (CEA), integrin av beta 3, integrin a5 beta 1, folate receptor 1, transmembrane glycoprotein NMB, fibroblast activation protein alpha (FAP), glycoprotein 75, TAG-72, MUC1, MUC16 (or CA-125), phosphatidylserine, prostate-specific membrane antigen (PMSA), NR-LU-13 antigen, TRAIL-R1, tumor necrosis factor receptor superfamily member 10b (TNFRSF10B or TRAIL-R2), SLAM family member 7 (SLAMF7), EGP40 pancarcinoma antigen, B-cell activating factor (BAFF), platelet-derived growth factor receptor, glycoprotein EpCAM (17-1A), Programmed Death-1, protein disulfide isomerase (PDI), Phosphatase of Regenerating Liver 3 (PRL-3), prostatic acid phosphatase, Lewis-Y antigen, GD2 (a disialoganglioside expressed on tumors of neuroectodermal origin), glypican-3 (GPC3), or mesothelin;
- associated antigens belonging to unique gene products of mutated or recombined cellular genes, in particular cyclin-dependent kinases, p15Ink4b, p53, AFP, B-catenin, caspase 8, p53, Bcr-abl fusion product, MUM-1 MUM-2, MUM-3, ELF2M, HSP70-2M, HST-2, KIAA0205, RAGE, myosin/m, 707-AP, CDC27/m, ETV6/AML, TEL/AmII, Dekcain, LDLR/FUT, Pm1-RARa, TEL/AMLI; Cancer-testis (CT) antigens, members of the MAGE-family, BAGE, DAM-6, DAM-10, members of the GAGE-family, NY-ESO-1, NA-88A, CAG-3, RCC-associated antigen G250, Tumor virus antigens, in particular human papilloma virus (HPV) -derived E6 E7 oncoproteins, Epstein Barr virus EBNA2-6, LMP-1, LMP-2; gp77, gp100, MART-1/Melan-A, p53, tyrosinase, tyrosinase-related protein 1 and 2, PSA, PSM, MC1R; ART4, CAMEL, CEA, CypB, epithelial cell adhesion molecule (EpCAM) HER2/neu, HER-3, hTERT, hTRT, ICE, Muc1, Muc2, PRAME RU1, RU2, SART-1, SART-2, SART-3, and WT1, and fragments and derivatives thereof;
- CD19; CD123; CD22; CD30; CD171; CS-1 (also referred to as CD2 subset 1, CRACC, SLAMF7, CD319, and 19A24); C-type lectin-like molecule-1 (CLL-1 or CLECLI); CD33; epidermal growth factor receptor variant III (EGFRvIII); ganglioside G2 (GD2); ganglioside GD3 ($\alpha$NeuSAc(2-8)$\alpha$NeuSAc(2-3)$\beta$DGaip(1-4)bDGlcp(1-1)Cer); ganglioside GM3 ($\alpha$NeuSAc(2-3)$\beta$DGalp(1-4)$\beta$DGlcp(1-1)Cer); GM-CSF receptor; TNF receptor superfamily member 17 (TNFRSF17, BCMA); B-lymphocyte cell adhesion molecule; Tn antigen ((Tn Ag) or (GalNAc-$\alpha$-Ser/Thr)); prostate-specific membrane antigen (PSMA); Receptor tyrosine kinase-like orphan receptor 1 (RORI); Tumor-associated glycoprotein 72 (TAG72); CD38; CD44v6; Carcinoembryonic antigen (CEA); Epithelial cell adhesion molecule (EPCAM); B7H3 (CD276); KIT (CD117); Interleukin-13 receptor subunit alpha-2 (IL-13Ra2 or CD213A2); Mesothelin; Interleukin 11 receptor alpha (IL-11Ra); prostate stem cell antigen (PSCA); Protease Serine 21 (Testisin or PRSS21); vascular endothelial growth factor receptor 2 (VEGFR2); HLA class I antigen A-2 alpha; HLA antigen; Lewis(Y)antigen; CD24; Platelet-derived growth factor receptor beta (PDGFR-beta); Stage-specific embryonic antigen-4 (SSEA-4); CD20; delta like 3 (DLL3); Folate receptor alpha; Folate receptor beta, GDNF alpha 4 receptor, Receptor tyrosine-protein kinase, ERBB2 (Her2/neu); Mucin 1, cell surface associated (MUC1); APRIL receptor; ADP ribosyl cyclase-1; Ephb4 tyrosine kinase receptor, DCAMKL1 serine threonine kinase, Aspartate beta-hydroxylase, epidermal growth factor receptor (EGFR); neural cell adhesion molecule (NCAM); Prostase; prostatic acid phosphatase (PAP); elongation factor 2 mutated (ELF2M); Ephrin B2; fibroblast activation protein alpha (FAP); insulin-like growth factor 1 receptor (IGF-I receptor), carbonic anhydrase IX (CAIX); Proteasome (Prosome, Macropain) Subunit, Beta Type, 9 (LMP2); glycoprotein 100 (gp100);oncogene fusion protein consisting of breakpoint cluster region (BCR) and Abelson murine leukemia viral oncogene homolog 1 (Abl) (bcr-abl); tyrosinase; ephrin type-A receptor 2 (EphA2); ephrin type-A receptor 3 (EphA3), Fucosyl GM1; sialyl Lewis adhesion molecule (sLe); transglutaminase 5 (TGS5); high molecular weight-melanomaassociatedantigen (HMWMAA); o-acetyl-GD2 ganglioside (OAcGD2); Folate receptor beta; tumor endothelial marker 1 (TEM1/CD248); tumor endothelial marker 7-related (TEM7R); six transmembrane epithelial antigen of the prostate I (STEAP1); claudin 6 (CLDN6); thyroid stimulating hormone receptor (TSHR); G protein-coupled receptor class C group 5, member D (GPRCSD); IL-15 receptor (IL-15); chromosome X open reading frame 61 (CXORF61); CD97; CD179a; anaplastic lymphoma kinase (ALK); Polysialic acid; placenta-specific 1 (PLAC1); hexasaccharide portion of globoH glycoceramide (GloboH); mammary gland differentiation antigen (NY-BR-1); uroplakin 2 (UPK2); Hepatitis A virus cellular receptor 1 (HAVCR1); adrenoceptor beta 3 (ADRB3); pannexin 3 (PANX3); G protein-coupled receptor 20 (GPR20); lymphocyte antigen 6 complex, locus K 9 (LY6K); Olfactory receptor 51E2 (ORS IE2); TCR Gamma Alternate Reading Frame

Protein (TARP); Wilms tumor protein (WT1); Cancer/testis antigen 1 (NY-ESO-1); Cancer/testis antigen 2 (LAGE-Ia); Melanoma associated antigen 1 (MAGE-A1); Melanoma associated antigen 3 (MAGE-A3); Melanoma associated antigen 4 (MAGE-A4); T cell receptor beta 2 chain C; ETS translocation-variant gene 6, located on chromosome 12p (ETV6-AML); sperm protein 17 (SPA17); X Antigen Family, Member 1A (XAGE1); angiopoietin-binding cell surface receptor 2 (Tie 2); melanoma cancer testis antigen-1 (MADCT-1); melanoma cancer testis antigen-2 (MAD-CT-2); Fos-related antigen 1; tumor protein p53, (p53); p53 mutant; prostein; survivin; telomerase; prostate carcinoma tumor antigen-1 (PCTA-1 or Galectin 8), melanoma antigen recognized by T cells 1 (MelanA or MARTI); Rat sarcoma (Ras) mutant; human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N-Acetyl glucosaminyl-transferase V (NA17); paired box protein Pax-3 (PAX3); Androgen receptor; Cyclin-AI; Cyclin B1;v-myc avian myelocytomatosis viral oncogene neuroblastoma derived homolog (MYCN); Ras Homolog Family Member C (RhoC); Tyrosinase-related protein 2 (TRP-2); Cytochrome P450 1B1(CYP IBI); CCCTC-Binding Factor (Zinc Finger Protein)-Like (BORIS or Brother of the Regulator of Imprinted Sites), Squamous Cell Carcinoma Antigen Recognized By T Cells 3 (SART3); Paired box protein Pax-5 (PAXS); proacrosin binding protein sp32 (OY-TES I); lymphocyte-specific protein tyrosine kinase (LCK); A kinase anchor protein 4 (AKAP-4); Peptidoglycan recognition protein, synovial sarcoma, X breakpoint 2 (SSX2); Receptor for Advanced Glycation End products (RAGE-I); renal ubiquitous 1 (RUI); renal ubiquitous 2 (RU2); legumain; human papilloma virus E6 (HPV E6); human papilloma virus E7 (HPV E7); intestinal carboxyl esterase; heat shock protein 70-2 mutated (mut hsp70-2); CD79a; CD79b; CD72; Leukocyte-associated immunoglobulin-like receptor 1 (LAIRI); Fc fragment of IgA receptor (FCAR or CD89); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like module containing mucin-like hormone receptor-like 2 (EMR2); lymphocyte antigen 75 (LY75); Glypican-2 (GPC2); Glypican-3 (GPC3); Fc receptor-like 5 (FCRL5); and immunoglobulin lambda-like polypeptide 1 (IGLL1); and

- CD150, 5T4, ActRIIA, B7, TNF receptor superfamily member 17 (TNFRSF17, BCMA), CA-125, CCNA1, CD123, CD126, CD138, CD14, CD148, CD15, CD19, CD20, CD200, CD21, CD22, CD23, CD24, CD25, CD26, CD261, CD262, CD30, CD33, CD362, CD37, CD38, CD4, CD40, CD4OL, CD44, CD46, CD5, CD52, CD53, CD54, CD56, CD66a-d, CD74, CD8, CD80, CD92, CE7, CS-1, CSPG4, ED-B fibronectin, EGFR, EGFRvIII, EGP-2, EGP-4, EPHa2, ErbB2, ErbB3, ErbB4, FBP, HER1-HER2 in combination, HER2-HER3 in combination, HERV-K, HIV-1 envelope glycoprotein gp120, HIV-1 envelope glycoprotein gp41, HLA-DR, HLA class I antigen alpha G, HM1.24, K-Ras GTPase, HMW-MAA, Her2, Her2/neu, IGF-1R, IL-11Ralpha, IL-13R-alpha2, IL-2, IL-22R-alpha, IL-6, IL-6R, la, li, L1-CAM, L1-cell adhesion molecule, Lewis Y, LI-CAM, MAGE A3, MAGE-A1, MART-1, MUC1, NKG2C ligands, NKG2D Ligands, NYESO-1, OEPHa2, PIGF, PSCA, PSMA, ROR1, T101, TAC, TAG72, TIM-3, TRAIL-R1, TRAIL-R1 (DR4), TRAIL-R2 (DR5), VEGF, VEGFR2, WT-I, a G-protein coupled receptor, alphafetoprotein (AFP), an angiogenesis factor, an exogenous cognate binding molecule (ExoCBM), oncogene product, anti-folate receptor, c-Met, carcinoembryonic antigen (CEA), cyclin (D 1), ephrinB2, epithelial tumor antigen, estrogen receptor, fetal acetylcholine e receptor, folate binding protein, gp100, hepatitis B surface antigen, Epstein-Barr nuclear antigen 1, Latent membrane protein 1, Secreted protein BARF1, P2X7 purinoceptor, Syndecan-1, kappa chain, kappa light chain, kdr, lambda chain, livin, melanoma-associated antigen, mesothelin, mouse double minute 2 homolog (MDM2), mucin 16 (MUC16), mutated p53, mutated ras, necrosis antigens, oncofetal antigen, ROR2, progesterone receptor, prostate specific antigen, tEGFR, tenascin, β2-microglobulin, Fc Receptor-like 5 (FcRL5).

**9.** The compound according to any one of claims 1 to 8, wherein the helper T cell epitope is a peptide containing less than or about 20 amino acids and/or amino acid analogs.

**10.** The compound according to any one of claims 1 to 8, wherein the helper T cell epitope is selected from the group consisting of:

- Peptides derived from polio virus, such as (PV 103-115) KLFAVWKITYKDT;
- pan-DR binding (PADRE) peptides, such as DAla-Lys-Cha-Val-Ala-Ala-Trp-Thr-Leu-Lys-Ala-Ala-DAla;
- Peptides derived from tetanus toxin, such as (TT593-599) YSYFPSV, (TT830-843) QYIKANSKFIGITE, (TT830-844) QYIKANSKFIGITEL, (TT1084-1099) VSIDKFRIFCKANPK, (TT1174-1189) LKFIIKRYTPNNEIDS, (TT1064-1079) IREDNNITLKLDRCNN, and (TT947-967) FNNFTVSFWLRVPKVSASHLE;
- Peptides derived from *Neisseria meningitidis,* such as YAFKYARHANVGRNAFELFL ("YAF"); and
- Peptides derived from *P. falciparum* CSP such as, EKKIAKMEKASSVFNVNN.

**11.** A method of synthesizing the compound according to claim 1, which is selected from:

i) a method comprising:

a) providing a compound of formula (V) or a salt thereof

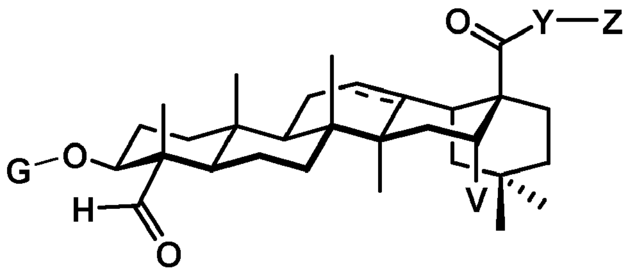

(V)

wherein - - -, G, V, Y and Z take the meanings as in claim 1; and
b) reacting the compound of formula (V) with $NH_2OH$ or a salt thereof, to form a compound of formula (I) or a salt thereof

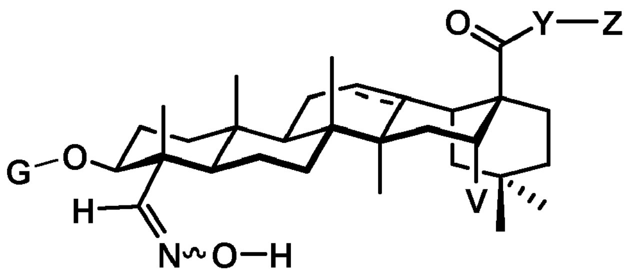

(I)

wherein - - -, G, V, Y and Z take the meanings as in claim 1;

ii) a method comprising:

a) providing a compound of formula (V) or a salt thereof

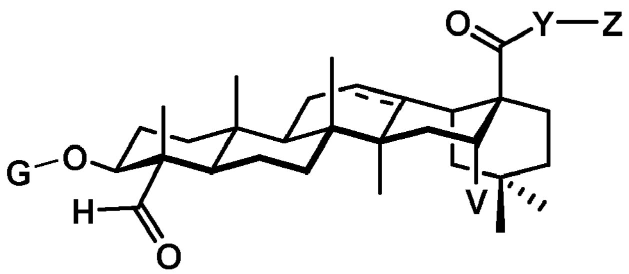

(V)

wherein - - -, G, V, Y and Z take the meanings as in claim 1;
b) conjugating the compound of formula (V) or a salt thereof with a moiety comprising either i) at least one T cell epitope selected from a helper T cell epitope or a CD8 epitope, ii) at least one B cell epitope or iii) at least one T cell epitope selected from a helper T cell epitope or a CD8 epitope and at least one B cell epitope, to form a compound of formula (III) or a salt thereof

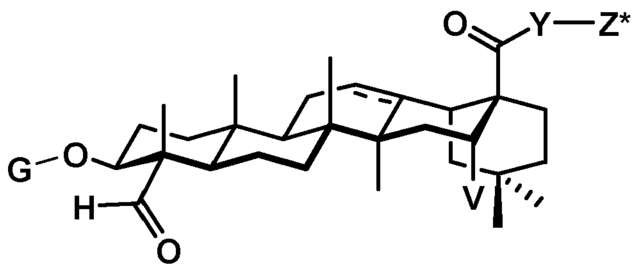

(III);

c) reacting the compound of formula (III) or a salt thereof with a compound of formula

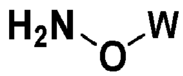

or a salt thereof, wherein W is H or a moiety comprising either i) at least one T cell epitope selected from a helper T cell epitope or a CD8 epitope, ii) at least one B cell epitope or iii) at least one T cell epitope selected from a helper T cell epitope or a CD8 epitope and at least one B cell epitope, to form a compound of formula (II) or a salt thereof

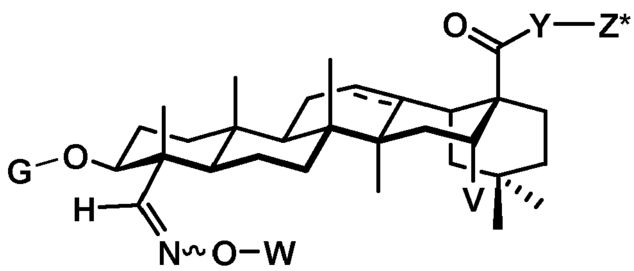

(II);

iii. a method comprising:

a) providing a compound of formula (V) or a salt thereof

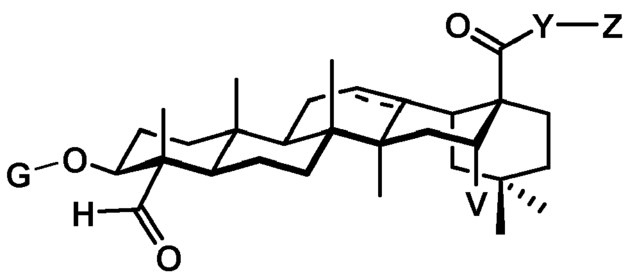

(V)

wherein - - -, G, V, Y and Z take the meanings as in claim 1;
b) reacting the compound of formula (V) or a salt thereof with a compound of formula

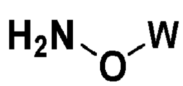

or a salt thereof, wherein W is H or a moiety comprising either i) at least one T cell epitope selected from a helper T cell epitope or a CD8 epitope, ii) at least one B cell epitope or iii) at least one T cell epitope selected from a helper T cell epitope or a CD8 epitope and at least one B cell epitope, to form a compound of formula (IV) or a salt thereof

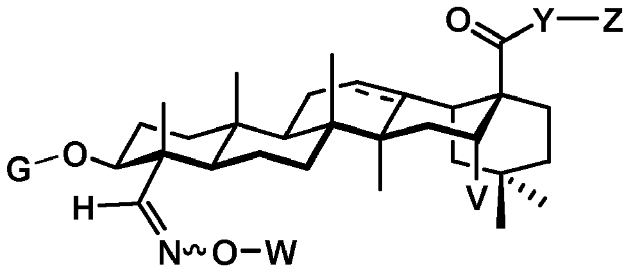

(IV)

wherein the compound of formula (IV) when W is a moiety comprising at least one T cell epitope selected from a helper T cell epitope or a CD8 epitope and at least one B cell epitope falls under formula (II);
c) optionally, conjugating the compound of formula (IV) or a salt thereof with a moiety comprising either i) at least one T cell epitope selected from a helper T cell epitope or a CD8 epitope, ii) at least one B cell epitope or iii) at least one T cell epitope selected from a helper T cell epitope or a CD8 epitope and at least one B cell epitope, to form a compound of formula (II) or a salt thereof

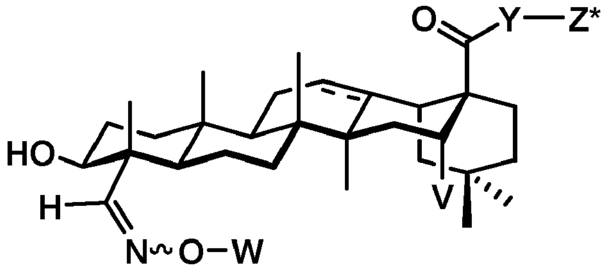

(II).

**12.** A pharmaceutical composition comprising:

- a compound of formula (I) or a pharmaceutically acceptable salt thereof according to claim 1, a pharmaceutically acceptable carrier and an antigen comprising at least one B cell epitope, or
- a compound of formula (II) or a pharmaceutically acceptable salt thereof according to claim 1 and a pharmaceutically acceptable carrier.

**13.** A compound according to claim 1 for use in medicine.

**14.** A compound according to claim 1 or a pharmaceutical composition according to claim 12 for use in the treatment and/or prevention of cancer, an infectious disease or a neurodegenerative disease.

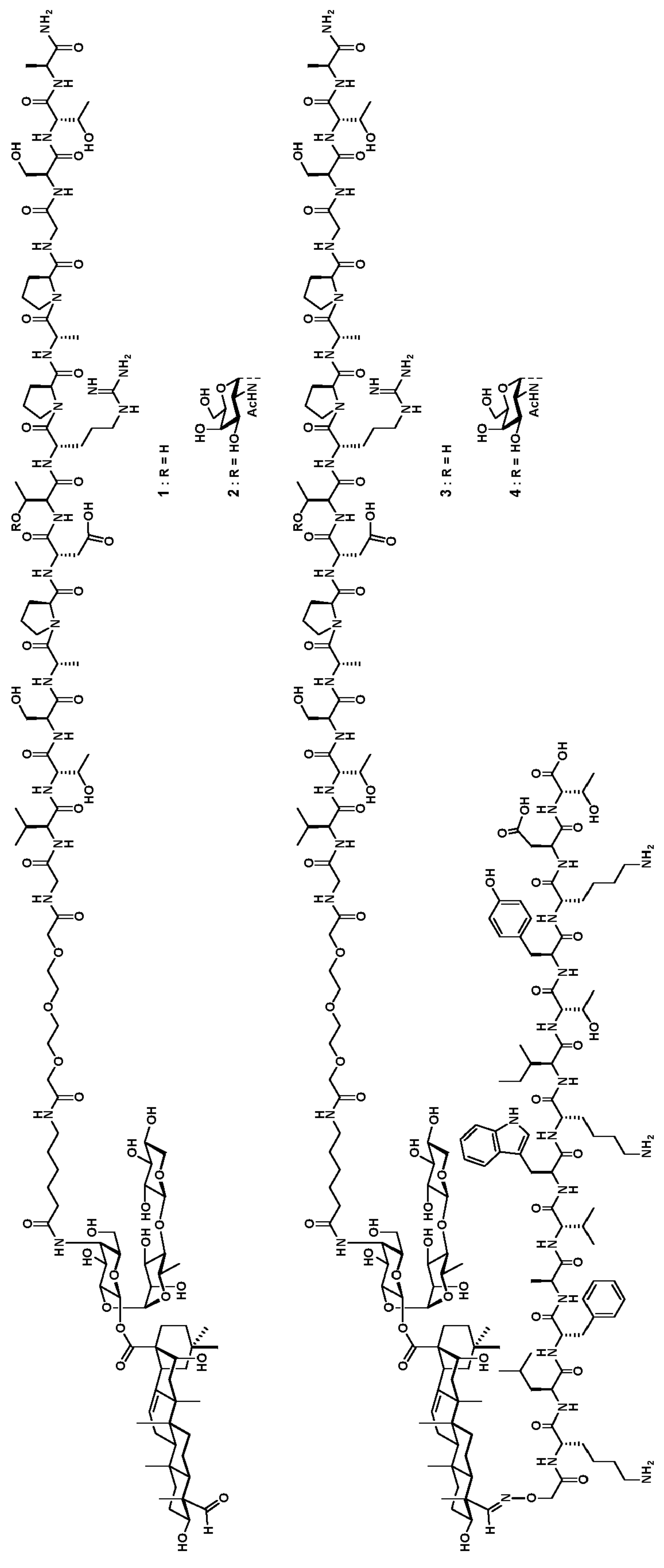

1 : R = H

2 : R =

3 : R = H

4 : R =

Fig. 1

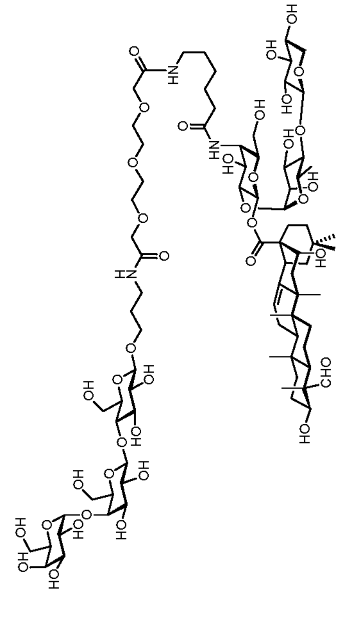

QA-Gb3 (28)

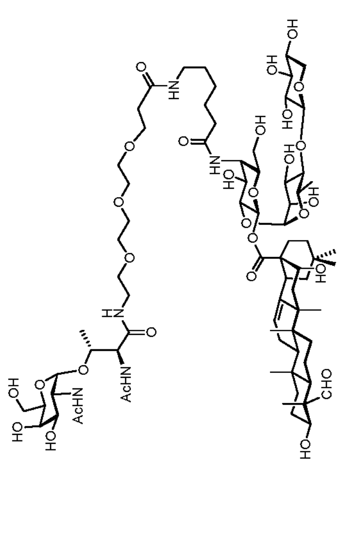

QA-Tn(Thr) (26)

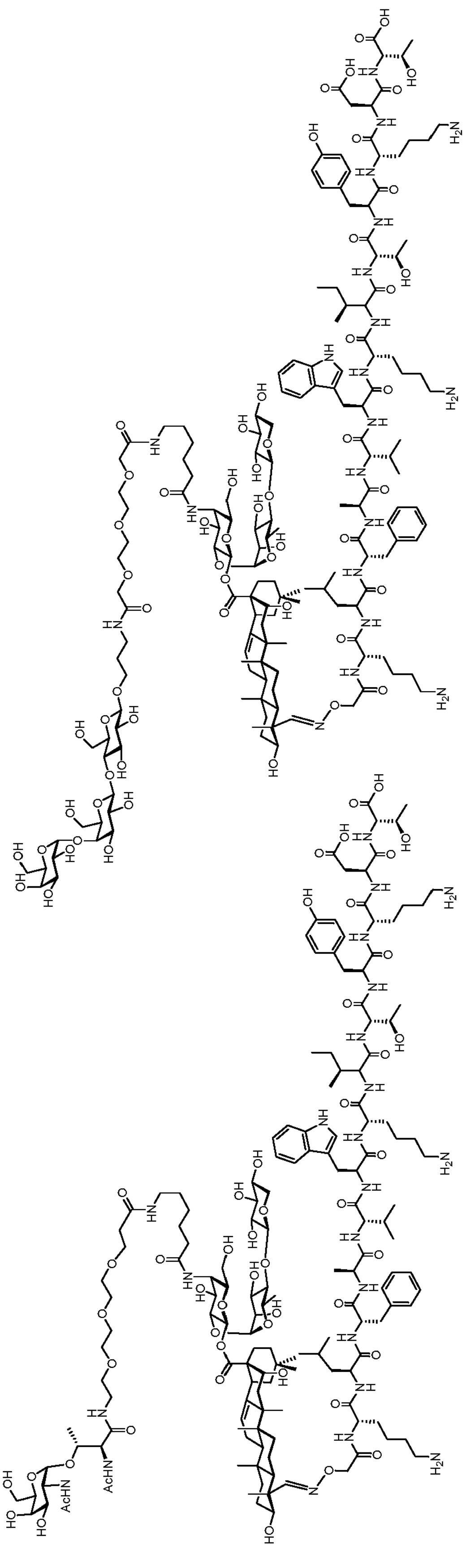

PV-QA-Gb3 (29)

PV-QA-Tn(Thr) (27)

Fig. 2

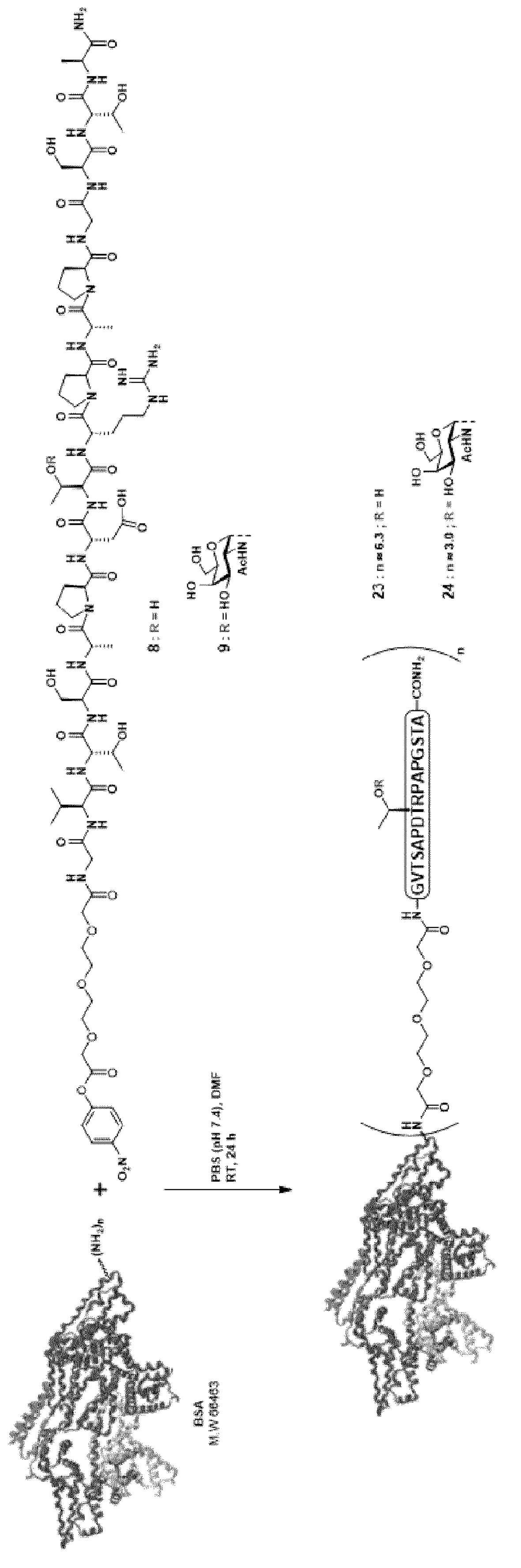


8 : R = H
9 : R = HO

23 : n ≈ 6.3 ; R = H
24 : n ≈ 3.0 ; R = HO

Fig. 3

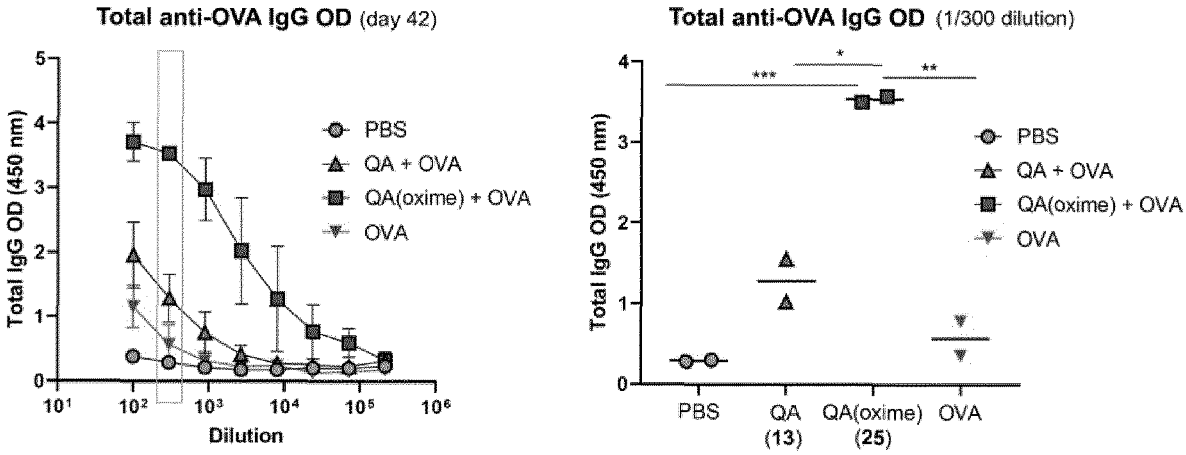


Fig. 4

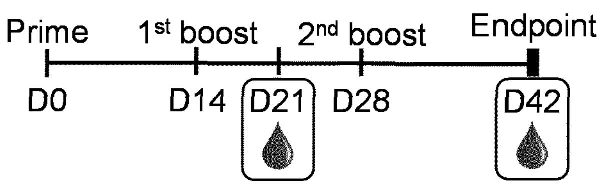


| Group | Formulation |
|---|---|
| A | MUC1 (5) |
| B | MUC1 (5) + PV (22) |
| C | QA-MUC1 (1) + PV (22) |
| D | QA(PV)-MUC1 (3) |

Fig. 5

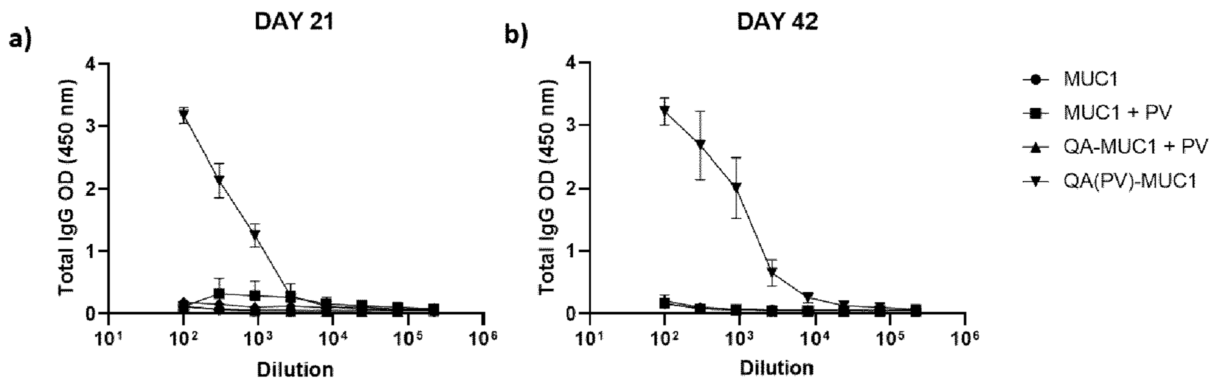
a)
DAY 21
b)
DAY 42
Total IgG OD (450 nm)
Dilution
MUC1
MUC1 + PV
QA-MUC1 + PV
QA(PV)-MUC1

Fig. 6

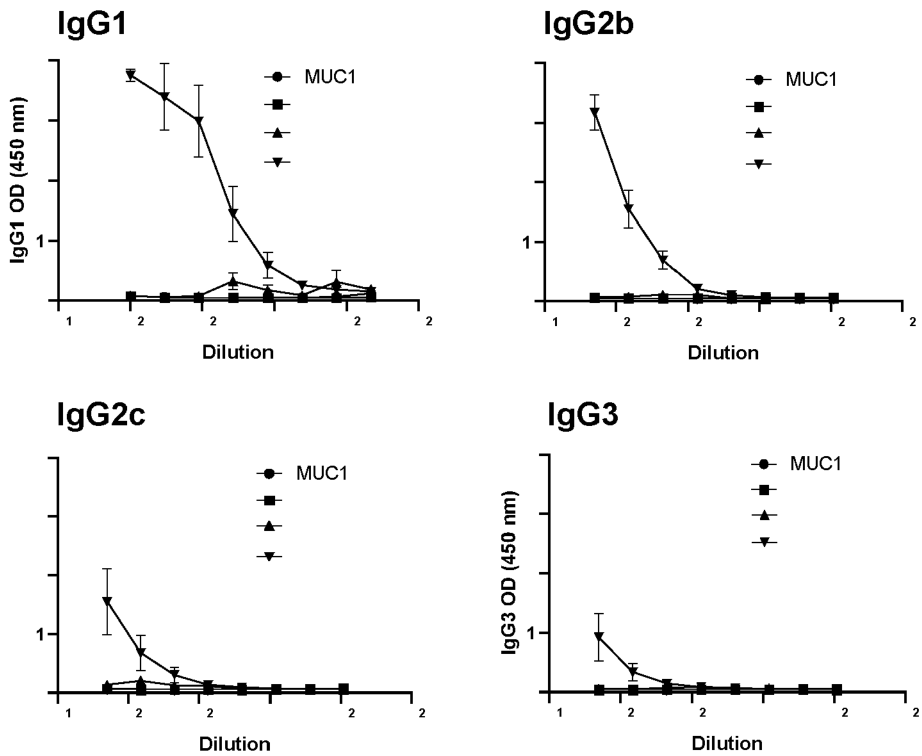
IgG1
IgG2b
IgG2c
IgG3
MUC1
IgG1 OD (450 nm)
IgG3 OD (450 nm)
Dilution

Fig. 7

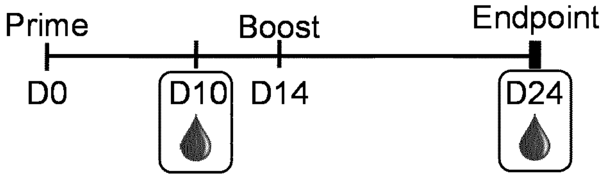


| Group | Formulation |
|---|---|
| A | PBS |
| B | QA (13) + TnMUC1 (6) |
| C | QA (13) + TnMUC1 (6) + PV (22) |
| D | QA-TnMUC1 (2) |
| E | QA-TnMUC1 (2) + PV (22) |
| F | QA(PV) (14) + TnMUC1 (6) |
| G | QA(PV)-TnMUC1 (4) |

Fig. 8

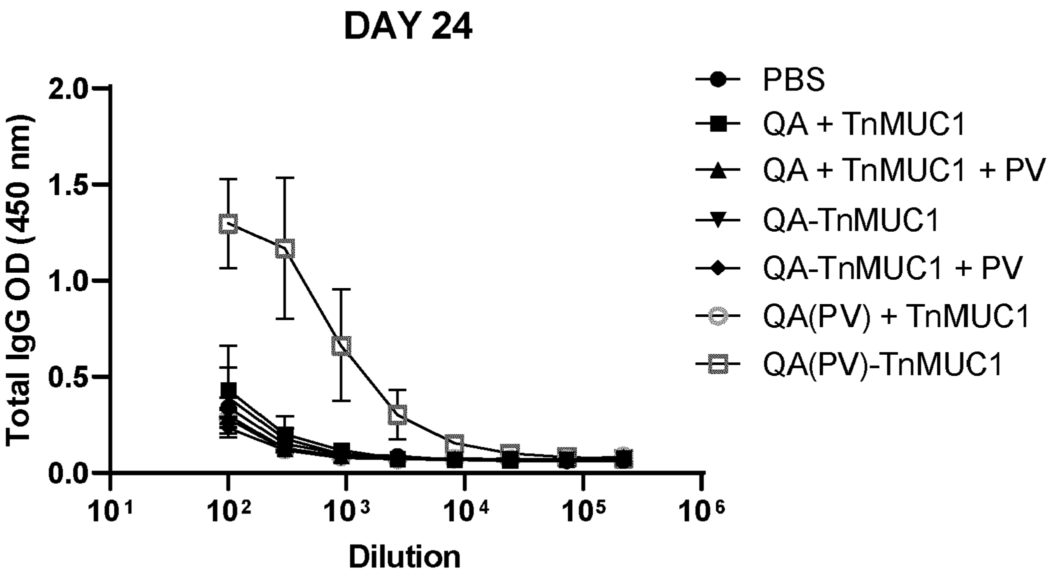


Fig. 9

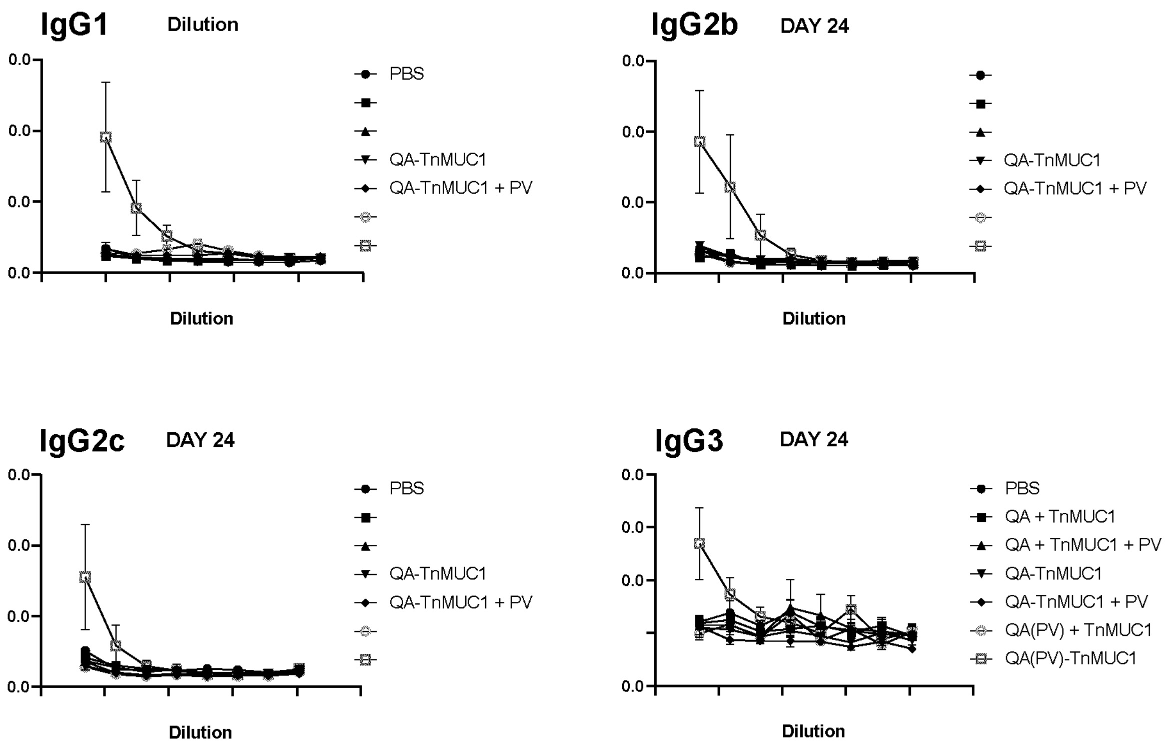
IgG1
Dilution
0.0
0.0
0.0
0.0
PBS
QA-TnMUC1
QA-TnMUC1 + PV
Dilution
IgG2b
DAY 24
0.0
0.0
0.0
0.0
QA-TnMUC1
QA-TnMUC1 + PV
Dilution
IgG2c
DAY 24
0.0
0.0
0.0
0.0
PBS
QA-TnMUC1
QA-TnMUC1 + PV
Dilution
IgG3
DAY 24
0.0
0.0
0.0
0.0
PBS
QA + TnMUC1
QA + TnMUC1 + PV
QA-TnMUC1
QA-TnMUC1 + PV
QA(PV) + TnMUC1
QA(PV)-TnMUC1
Dilution

Fig. 10

Europäisches Patentamt
European Patent Office
Office européen des brevets

# EUROPEAN SEARCH REPORT

Application Number
EP 21 38 3098

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ALBERTO FERNÁNDEZ-TEJADA ET AL: "Design, synthesis, and immunologic evaluation of vaccine adjuvant conjugates based on QS-21 and tucaresol", BIOORGANIC, vol. 22, no. 21, 17 September 2014 (2014-09-17), pages 5917-5923, XP055423243, AMSTERDAM, NL ISSN: 0968-0896, DOI: 10.1016/j.bmc.2014.09.016 * abstract * * figures 1-4 * * page 5921, column 1, paragraph 3 - column 2, paragraph 1 * ----- | 1-14 | INV. A61K47/55 A61K47/64 A61K39/39 A61K39/385 A61K39/00 C07H15/256 C07J51/00 A61P25/28 A61P31/00 A61P35/00 |
| A | US 5 583 112 A (KENSIL CHARLOTTE A [US] ET AL) 10 December 1996 (1996-12-10) * column 9, line 4 - column 11, line 5 * * examples 18-21, 23, 24 * ----- | 1-14 | |
| | | | TECHNICAL FIELDS SEARCHED (IPC) |
| | | | A61K A61P C07H C07J |

The present search report has been drawn up for all claims

1

| Place of search | Date of completion of the search | Examiner |
|---|---|---|
| The Hague | 10 June 2022 | Monami, Amélie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 21 38 3098

This annex lists the patent family members relating to the patent documents cited in the above-mentioned European search report. The members are as contained in the European Patent Office EDP file on
The European Patent Office is in no way liable for these particulars which are merely given for the purpose of information.

10-06-2022

| Patent document cited in search report | Publication date | Patent family member(s) | Publication date |
|---|---|---|---|
| US 5583112 A | 10-12-1996 | AT 217795 T | 15-06-2002 |
| | | AU 662562 B2 | 07-09-1995 |
| | | CA 2118928 A1 | 01-04-1993 |
| | | DE 69232616 T2 | 05-09-2002 |
| | | DK 0606317 T3 | 16-09-2002 |
| | | EP 0606317 A1 | 20-07-1994 |
| | | ES 2174837 T3 | 16-11-2002 |
| | | JP 3717511 B2 | 16-11-2005 |
| | | JP H07504156 A | 11-05-1995 |
| | | NZ 244410 A | 28-03-1995 |
| | | US 5583112 A | 10-12-1996 |
| | | WO 9305789 A1 | 01-04-1993 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009126737 A
- WO 2015184451 A
- WO 2017079582 A
- WO 2017106836 A
- WO 2018191598 A
- WO 2018200645 A
- WO 2018200656 A
- WO 2019079160 A

### Non-patent literature cited in the description


- **PIFFERI, C. et al.** *Nat. Rev. Chem.,* 2021, vol. 25 (4), 3-7
- **BERGMANN-LEITNER, E. et al.** *Vaccines,* 2014, vol. 2 (2), 252-296
- **REED, S. G. et al.** *Nat. Med.,* 2013, vol. 19 (12), 1597-1608
- *J. Pharmaceutical Sciences,* 1977, vol. 66, 1-19
- **T. W. GREENE ; P. G. M. WUTS.** Protecting Groups in Organic Synthesis. John Wiley & Sons, 1999
- **SMALL.** HANDBOOK OF LIPID RESEARCH. 1986, vol. 4, 49-50
- **AHMAD, T. A. et al.** *Trials in Vaccinology,* 2016, vol. 5, 71-83
- **WANG R. F.** *Methods.,* 2003, vol. 29 (3), 227-35
- **SINGH R. et al.** *Immunology,* 2014, vol. 141 (4), 514-30
- **RECHE et al.** *Immunogenetics,* 2004, vol. 56 (6), 405-419
- **DÖNNES ; KOHLBACHER.** *Protein Sci.,* 2005, vol. 14 (8), 2132-2140
- **DOYTCHINOVA et al.** *BMC Bioinformatics,* 2006, vol. 7 (1), 131
- **ERICKSON et al.** *J. Immunol.,* 1993, vol. 151, 4189-4199
- **DOE et al.** *Eur. J. Immunol.,* 1994, vol. 24, 2369-2376
- The Merck Manual. 1999
- **GHIRARDELLO et al.** *Chem. Commun.,* 2020, vol. 56 (5), 719-722
- **PAYNE et al.** *J. Am. Chem. Soc.,* 2007, vol. 129 (44), 13527-13536
- **Z. WU et al.** *Chem. Commun.,* 2010, vol. 46, 5773-5774
- **WITTMANN et al.** *J. Org. Chem.,* 1998, vol. 63 (15), 5137-5143
- **FERNÁNDEZ-TEJADA et al.** *Nat. Chem.,* 2014, vol. 6 (7), 635-643
- *CHEMICAL ABSTRACTS,* 9048-46-8
- **T. BUSKAS et al.** *Angew. Chem. Int. Ed.,* 2005, vol. 44, 5985-5988
- **AHMAD, T. A. et al.** *Trials in Vaccinology,* 2016, vol. 5, 71-83, https://doi.org)/10.1016/i.trivac.2016.04.003
- **BERGMANN-LEITNER, E. ; LEITNER, W.** Adjuvants in the Driver's Seat: How Magnitude, Type, Fine Specificity and Longevity of Immune Responses Are Driven by Distinct Classes of Immune Potentiators. *Vaccines,* 2014, vol. 2 (2), 252-296, https://doi.org/10.3390/vaccines2020252
- **BUSKAS, T. ; INGALE, S. ; BOONS, G.-J.** Towards a Fully Synthetic Carbohydrate-Based Anticancer Vaccine: Synthesis and Immunological Evaluation of a Lipidated Glycopeptide Containing the Tumor-Associated Tn Antigen. *Angew. Chemie Int. Ed.,* 2005, vol. 44 (37), 5985-5988, hftps://doi.org/10.1002/anie.200501818
- **DOE, B. ; STEIMER, K.S. ; WALKER, C.M.** Induction of HIV-1 envelope (gp120)-specific cytotoxic T lymphocyte responses in mice by recombinant CHO cell-derived gp120 is enhanced by enzymatic removal of N-linked glycans. *Eur J Immunol.,* October 1994, vol. 24 (10), 2369-76, https://doi.org/10.1002/eji.1830241017
- **DÖNNES, P. ; KOHLBACHER, O.** Integrated modeling of the major events in the MHC class I antigen processing pathway. *Protein Sci.,* August 2005, vol. 14 (8), 2132-40, https://doi.org/10.1110/ps.051352405
- **DOYTCHINOVA, I.A. ; GUAN, P. ; FLOWER, D.R.** EpiJen: a server for multistep T cell epitope prediction. *BMC Bioinformatics,* 13 March 2006, vol. 7, 131, https://doi.org/10.1186/1471-2105-7-131
- **ERICKSON, A.L. ; HOUGHTON, M. ; CHOO, Q.L. ; WEINER, A.J. ; RALSTON, R. ; MUCHMORE, E. ; WALKER, C.M.** Hepatitis C virus-specific CTL responses in the liver of chimpanzees with acute and chronic hepatitis C. *J Immunol.,* 15 October 1993, vol. 151 (8), 4189-99

- **FERNÁNDEZ-TEJADA, A. ; CHEA, E. K. ; GEORGE, C. ; PILLARSETTY, N. ; GARDNER, J. R. ; LIVINGSTON, P. O. ; RAGUPATHI, G. ; LEWIS, J. S. ; TAN, D. S. ; GIN, D. Y.** Development of a Minimal Saponin Vaccine Adjuvant Based on QS-21. *Nat. Chem.,* 2014, vol. 6 (7), 635-643, https://doi.org/10.1038/nchem.1963
- **GHIRARDELLO, M. ; RUIZ-DE-ANGULO, A. ; SACRISTAN, N. ; BARRIALES, D. ; JIMÉNEZ-BARBERO, J. ; POVEDA, A. ; CORZANA, F. ; ANGUITA, J. ; FERNÁNDEZ-TEJADA, A.** Exploiting Structure-activity Relationships of QS-21 in the Design and Synthesis of Streamlined Saponin Vaccine Adjuvants. *Chem. Commun.,* 2020, vol. 56 (5), 719-722, https://doi.org/10.1039/C9CC07781B
- **PAYNE, R. J. ; FICHT, S ; TANG, S. ; BRIK, A. ; YANG, Y. Y. ; CASE, D. A. ; WONG, C. H.** Extended Sugar-Assisted Glycopeptide Ligations: Development, Scope, and Applications. *J. Am. Chem. Soc.,* 2007, vol. 129 (44), 13527-13536, https://doi.org/10.1021/ja073653p
- **PIFFERI, C. ; FUENTES, R. ; FERNÁNDEZ-TEJADA, A.** Natural and Synthetic Carbohydrate-Based Vaccine Adjuvants and Their Mechanisms of Action. *Nat. Rev. Chem.,* 2021, vol. 25 (4), 3-7, https://doi.org/10.1038/s41570-020-00244-3
- **RECHE, P. A. ; GLUTTING, J. P ; ZHANG, H. ; REINHERZ, E.L.** Enhancement to the RANKPEP resource for the prediction of peptide binding to MHC molecules using profiles. *Immunogenetics,* September 2004, vol. 56 (6), 405-19, https://doi.org/10.1007/s00251-004-0709-7.
- **REED, S. G. ; ORR, M. T. ; FOX, C. B.** Key Roles of Adjuvants in Modern Vaccines. *Nat. Med.,* 2013, vol. 19 (12), 1597-1608, https://doi.org/10.1038/nm.3409
- **SINGH R. ; GUPTA P. ; SHARMA P. K. ; ADES E. W. ; HOLLINGSHEAD S. K. ; SINGH S.; LILLARD J. W.** Prediction and characterization of helper T-cell epitopes from pneumococcal surface adhesin A. *Immunology.,* April 2014, vol. 141 (4), 514-30, https://doi.org/10.1111/imm.12194
- **WANG R. F.** Identification of MHC class II-restricted tumor antigens recognized by CD4+ T cells. *Methods,* March 2003, vol. 29 (3), 227-35, https://doi.org/10.1016/s1046-2023(02)00345-6
- **WITTMANN, V. ; TAKAYAMA, S. ; GONG, K. W. ; WEITZ-SCHMIDT, G. ; WONG, C. H. LIGAND.** Recognition by E- and P-Selectin: Chemoenzymatic Synthesis and Inhibitory Activity of Bivalent Sialyl Lewis x Derivatives and Sialyl Lewis x Carboxylic Acids. *J. Org. Chem.,* 1998, vol. 63 (15), 5137-5143, https://doi.org/10.1021/jo980350s
- **WU, Z. ; GUO, X. ; GUO, Z.** Chemoenzymatic Synthesis of Glycosylphosphatidylinositol-Anchored Glycopeptides. *Chem. Commun.,* 2010, vol. 46 (31), 5773-5774, https://doi.org/10.1039/C0CC00828A